# EUROPEAN PATENT SPECIFICATION

(11) **EP 1 204 665 B1**
(45) Date of publication and mention of the grant of the patent: **07.01.2009**
(21) Application number: 00950880.5
(22) Date of filing: 28.07.2000
(51) Int. Cl.: C07D 491/044, A61K 31/4353, A61K 31/4365, A61K 31/55, A61K 31/451, A61P 29/00, C07D 409/06, C07D 401/06, C07D 211/52, C07D 405/06, C07D 471/04, C07D 519/00, C07D 495/04

(54) **CHEMOKINE RECEPTOR ANTAGONISTS AND METHODS OF USE THEREFOR**
CHEMOKINE-REZEPTOR-ANTAGONISTEN UND METHODEN ZU DEREN ANWENDUNG
ANTAGONISTES DU RECEPTEUR DE CHIMIOKINE ET PROCEDES D'UTILISATION ASSOCIES

(30) Priority: 28.07.1999 US 362837
(43) Date of publication of application: 15.05.2002
(73) Proprietor: MILLENNIUM PHARMACEUTICALS, INC., Cambridge, MA 02139 (US); KYOWA HAKKO KOGYO CO., LTD., Chiyoda-ku, Tokyo 100-8185 (JP)
(72) Inventor: LULY, Jay, R., Wellesley, MA 02481 (US); NAKASATO, Yoshisuke, Sunto-gun, Shizuoka 411 (JP); OHSHIMA, Etsuo, Nagareyama-shi, Chiba 270-0115 (JP); SONE, Hiroki, Sunto-gun, Shizuoka 411 (JP); KOTERA, Osamu, Sunto-gun, Shizuoka 411 (JP); HARRIMAN, Geraldine, C., B., Charlestown, RI 02813 (US); CARSON, Kenneth, G., Needham, MA 02194 (US)
(74) Representative: Keen, Celia Mary
(86) International application number: PCT/US2000/020732
(87) International publication number: WO 2001/009138

(56) References cited:
- EP-A- 0 235 796
- EP-A- 0 325 755
- WO-A-00/32193
- WO-A-99/37651
- GB-A- 1 003 950
- GB-A- 1 013 574
- GB-A- 1 085 406
- GB-A- 1 206 216
- GB-A- 1 330 966
- GB-A- 1 347 935
- DATABASE CAPLUS [Online] CHEMICAL ABSTRACTS SERVICE, COLUMBUS, OHIO, US; PROTIVA: "4-(3-Dibenzo[b,e]thiepin-11-ylpropyl)-1-p iperazineethanols" retrieved from STN Database accession no. 107:134327 XP002257703 & CS 236 549 A (PROTIVA) 2 February 1984 (1984-02-02)
- DATABASE CAPLUS [Online] CHEMICAL ABSTRACTS SERVICE, COLUMBUS, OHIO, US; PROTIVA: "Preparation of 4-(dibenzo[b,e]thiepin-11-yllidenepropyl)- 1-piperazineethanols esters" retrieved from STN Database accession no. 107:134326 XP002257704 & CS 236 550 A (PROTIVA) 2 February 1984 (1984-02-02)
- DATABASE CAPLUS [Online] CHEMICAL ABSTRACTS SERVICE, COLUMBUS, OHIO, US; OSHIMA: "Preparation of 11-(3-carboxypropenylidene)dibenz[b,e]oxep in N-heterocyclyamide derivatives as antagonists for platelet-activating factor (PAF) receptors" retrieved from STN Database accession no. 115:256025 XP002257711 & JP 03 176487 A (KYOWA) 31 July 1991 (1991-07-31)
- DATABASE CAPLUS [Online] CHEMICAL ABSTRACTS SERVICE, COLUMBUS, OHIO, US; PROTIVA: "Tricyclic 3-(4-phenylpiperidino)propylidene derivatives" retrieved from STN Database accession no. 72:3387 XP002257706 & CS 128 004 A (PROTIVA) 15 June 1968 (1968-06-15)

## Description

### BACKGROUND OF THE INVENTION

Chemoattractant cytokines or chemokines are a family of proinflammatory mediators that promote recruitment and activation of multiple lineages of leukocytes and lymphocytes. They can be released by many kinds of tissue cells after activation. Continuous release of chemokines at sites of inflammation mediates the ongoing migration of effector cells in chronic inflammation. The chemokines characterized to date are related in primary structure. They share four conserved cysteines, which form disulfide bonds. Based upon this conserved cysteine motif, the family is divided into two main branches, designated as the C-X-C chemokines (α-chemokines), and the C-C chemokines (β-chemokines), in which the first two conserved cysteines are separated by an intervening residue, or adjacent respectively (Baggiolini, M. and Dahinden, C. A., Immunology Today, 15:127-133 (1999)).

The C-X-C chemokines include a number of portent chemoattractants and activators of neutrophils, such as interleukin 8 (IL-8), PF4 and neutrophil-activating peptide-2 (NAP-2). The C-C chemokines include RANTES (Regulated on Activation, Normal T Expressed and Secreted), the macrophage inflammatory proteins 1α and 1β(MIP-1α and MIP-1β), eotaxin and human monocyte chemotactic proteins 1-3 (MCP-1, MCP-2, MCP-3), which have been characterized as chemoattractants and activators of monocytes or lymphocytes but do not appear as RANTES and MIP-1α, have been implicated in a wide range of human acute and chronic inflammatory diseases including respiratory diseases, such as asthma and allergic disorders.

The chemokine receptors are members of a superfamily of G protein-coupled receptors (GPCR) which share structural features that reflect a common mechanism of action of signal transduction (Gerard, C. and Gerard, N.P., Annu Rev. Immunol., 12:775-808 (1994); Gerard, C. and Gerard, N. P., Curr. Opin. Immunol., 6:140-145 (1994)). Conserved features include seven hydrophobic domains spanning the plasma membrane, which are connected by hydrophilic extracellular and intracellular loops. The majority of the primary sequence homology occurs in the hydrophobic transmembrane regions with the hydrophilic regions being more diverse. The first receptor for the C-C chemokines that was cloned and expressed binds the chemokines MIP-1α and RANTES. Accordingly, this MIP-1α/RANTES receptor was designated C-C chemokine receptor 1 (also referred to as CCR-1; Neote, K., et al., Cell, 72: 415-425 (1993); Horuk, R. et al., WO 94/11504, May 26, 1994; Gao, J. -I. et al., J. Exp. Med., 177:1421-1427 (1993)). Three receptors have been characterized which bind and/or signal in response to RANTES: CCR3 mediates binding and signaling of chemokines including eotaxin, RANTES, and MCP-3 (Ponath et al., J. Exp. Med., 183:2437 (1996)), CCR4 binds chemokines including RANTES, MIP-1α, and MCP-1 (Power, et al., J. Biol. Chem., 270:19495 (1995)), and CCR5 binds chemokines including MIP-1α, RANTES, and MIP-1β (Samson, et al., Biochem. 35: 3362-3367 (1996)). RANTES is a chemotactic chemokine for a variety of cell types, including monocytes, eosinophils, and a subset of T-cells. The responses of these different cells may not all be mediated by the same receptor, and it is possible that the receptors CCR1, CCR4 and CCR5 will show some selectivity in receptor distribution and function between leukocyte types, as has already been shown for CCR3 (Ponath *et al.*). In particular, the ability of RANTES to induce the directed migration of monocytes and a memory population of circulating T-cells (Schall, T. et al., Nature, 347:669-71 (1990)) suggests this chemokine and its receptor (s) may play a critical role in chronic inflammatory diseases, since these diseases are characterized by destructive infiltrates of T cells and monocytes.

Many existing drugs have been developed as antagonists of the receptors for biogenic amines, for example, as antagonists of the dopamine and histamine receptors. No successful antagonists have yet been developed to the receptors for the larger proteins such as chemokines and C5a. Small molecule antagonists of the interaction between C-C chemokine receptors and their ligands, including RANTES and MIP-1α, would provide compounds useful for inhibiting harmful inflammatory processes "triggered" by receptor ligand interaction, as well as valuable tools for the investigation of receptor-ligand interactions.

### SUMMARY OF THE INVENTION

It has now been found that a class of small organic molecule are antagonists of chemokine receptor function and can inhibit leukocyte activation and/or recruitment. An antagonist of chemokine receptor function is a molecule which can inhibit the binding and/or activation of one or more chemokines, including C-C chemokines such as RANTES, MIP-1α, MCP-2., MCP-3 and MCP-4 to one or more chemokine receptors on leukocytes and/or other cell types. As a consequence, processes and cellular responses mediated by chemokines receptors can be inhibited with these small organic molecules. Based on this discovery, the molecules can be used in the treatment of a disease associated with aberrant leukocyte recruitment and/or activation and also in the treatment of a disease mediated by chemokine receptor function. The compounds of the invention can be administered to a subject in need an effective amount of a compound or small organic molecule which is an antagonist of chemokine receptor function. Compounds or small organic molecules which have been identified as antagonists of chemokine receptor function are discussed in detail hereinbelow, and can be used for the manufacture of a medicament for treating or for preventing a disease associated with aberrant leukocyte recruitment and/or activation. The invention also relates to the disclosed compounds and small organic molecules for use in treating or preventing a disease associated with aberrant leukocyte recruitment and/or activation. The invention also includes pharmaceutical compositions comprising one or more of the compounds or small organic molecules which have been identified herein as antagonists of chemokine function and a suitable pharmaceutical carrier. The invention further relates to novel compounds which can be used to treat an individual with a disease associated with aberrant leukocyte recruitment and/or activation and methods for their preparation.

Accordingly, provided herewith is a compound of the invention for use in a method of treatment of the human or animal body by therapy.

In one embodiment, a compound of the invention for use in the treatment of a disease associated with aberrant leukocyte recruitment and/or activation is provided.

In another embodiment, a compound of the invention for use in the treatment of a chronic inflammatory disease is provided.

In a further embodiment, a compound of the invention for use in the treatment of rheumatoid arthritis is provided.

In an additional embodiment, a compound of the invention for use in the treatment of multiple sclerosis is provided.

The present invention also provides the use of a compound of the invention in the manufacture of a medicament for the treatment of a disease associated with aberrant leukocyte recruitment and/or activation.

The present invention further provides the use of a compound of the invention in the manufacture of a medicament for the treatment of a chronic inflammatory disease.

The present invention additionally provides the use of a compound of the invention in the manufacture of a medicament for the treatment of rheumatoid arthritis.

The present invention also provides the use of a compound of the invention in the manufacture of a medicament for the treatment of multiple sclerosis.

### BRIEF DESCRIPTION OF THE DRAWINGS

Figure 1 is a schematic showing the preparation of the compounds represented by Structural Formula (I).
Figure 2 is a schematic showing the preparation of the compounds represented by Compound (VI-b).
Figure 3 is a schematic showing the preparation of the compounds represented by Structural Formula (I)
Figure 4 is a schematic showing the preparation of the compounds represented by Structural Formula (I), wherein Z is represented by Structural Formula (III) and wherein Ring A and/or Ring B in Z is substituted with R⁴⁰.
Figure 5 is a schematic showing the preparation of the compounds represented by Structural Formula (I), wherein Z is represented by Structural Formula (III) and wherein Ring A and/or Ring B in Z is substituted with - (O)ᵤ(CH₂)ₜ-COOR²⁰, -(O)ᵤ-(CH₂)ₜ-OC(O)R²⁰, -(O)ᵤ-(CH₂)ₜ-C(O)-NR²¹R²² or -(O)ᵤ-(CH₂)ₜ-NHC(O)O-R²⁰.
Figure 6 shows the preparation of compounds represented by Structural Formula (I), where in Z is represented by Structural Formulas (III) and wherein Ring A or Ring B in Z is substituted with R⁴⁰.

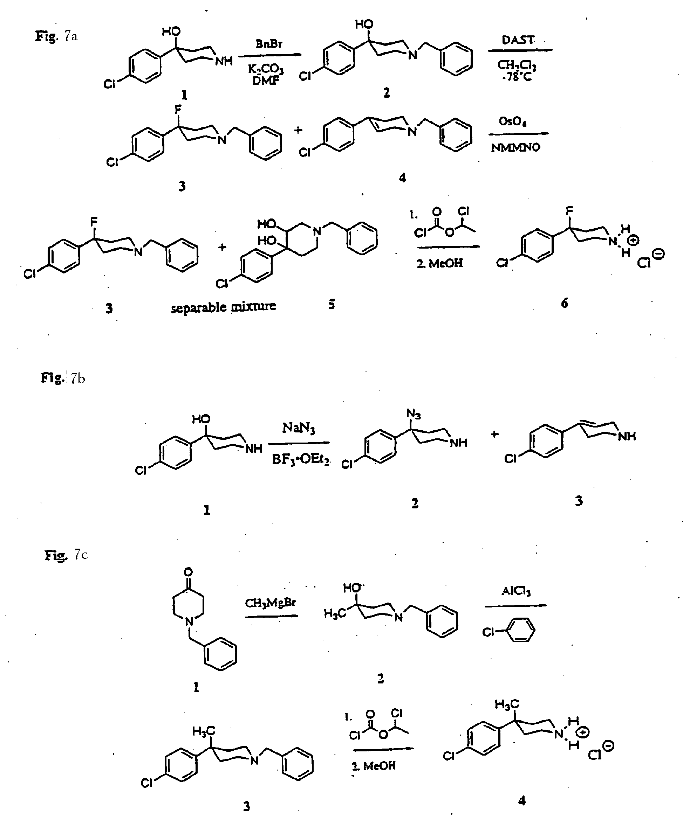

Figure 7a is a schematic showing the preparation of 9-(4-chlorophenyl)-4-fluoropiperidine.
Figure 7b is a schematic showing the preparation of 9-4-azido-4-(4-chlorophenyl)piperidine.
Figure 7c is a schematic showing the preparation of 4-(9-chlorophenyl)-9-methylpiperidine.
Figure 8a is a schematic showing the preparation of compounds represented by Structural Formulas (I), (VIII) and (VIII) wherein R¹ is an amine.
Figure 8b is a schematic showing the preparation of compounds represented by Structural Formulas (I), (VIII) and (VIII) wherein R¹ is an alkylamine.
Figure 8c is a schematic showing the preparation of 2-(4-chlorophenyl)-1-(*N*-methyl)ethylamine.
Figure 8d is a schematic showing the preparation of 3-(4-chlorophenyl)-3-chloro-1-hydroxypropane.

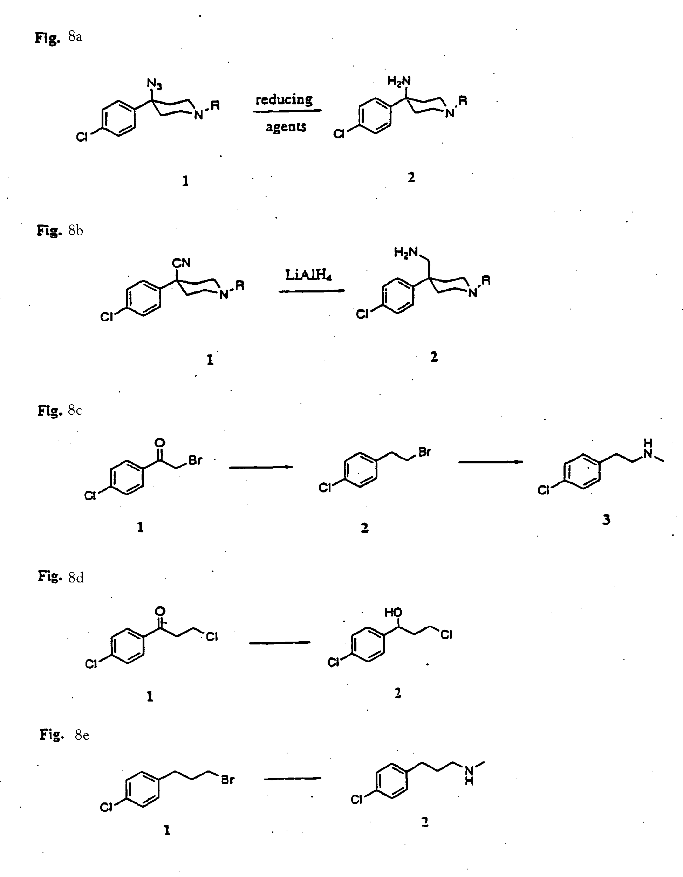

Figure 8c is a schematic showing the preparation of 3-(4-chlorophenyl)-1-*N*-methylaminopropane.

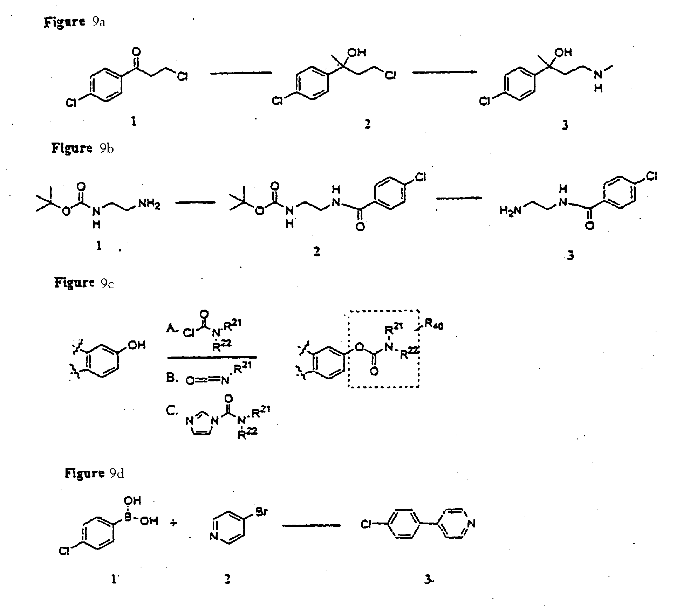

Figure 9a is a schematic showing the preparation of 3-(4-chlorophenyl)-3-hydroxyl-3-methyl-1-*N-*methylaminopropane.
Figure 9b is a schematic showing the preparation of 1-(4-chlorobenzol)-1,3-propylenediamine.
Figure 9c is a schematic showing three procedures for the preparation of compounds represented by Structural Formulas (I), (VII), (VIII), (IX) and (XI) wherein Z is represented by Structural Formula (III) and wherein Ring A or Ring B in Z is substituted with R⁴⁰. In Figure 9c, R⁴⁰ is represented by -(O)ᵤ-(CH₂)ₜ-C(O)-NR²¹R²², u is one, t is zero.
Figure 9d is a schematic showing the preparation of 4-(4-chlorophenyl)-4-pyridine.

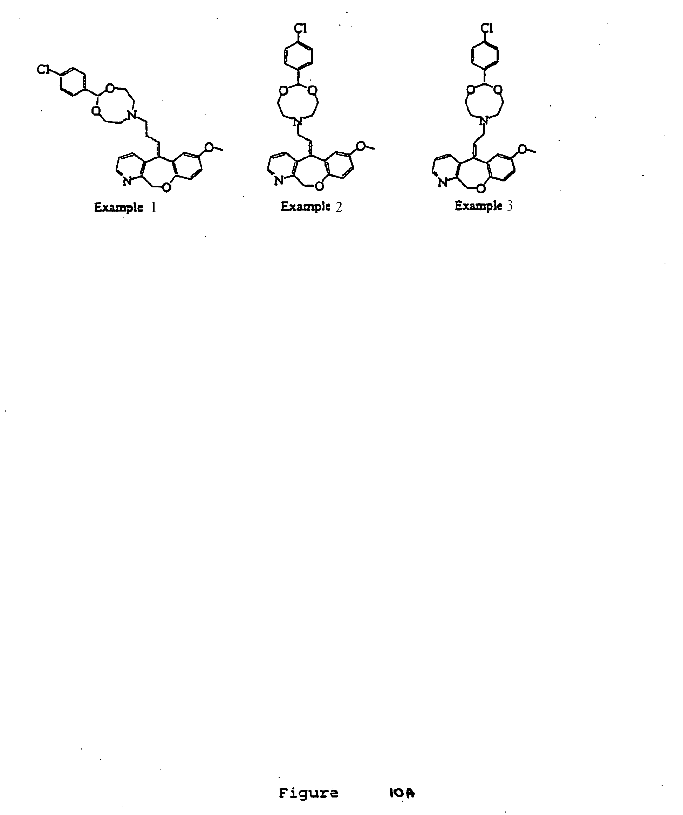

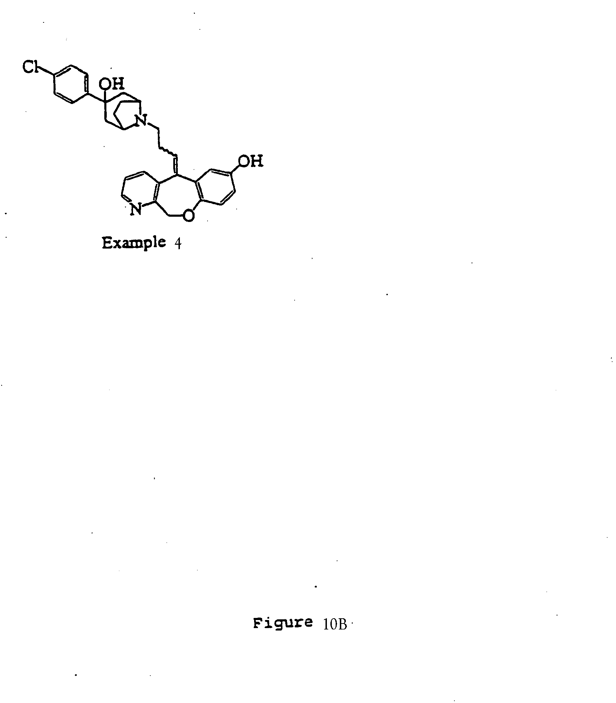

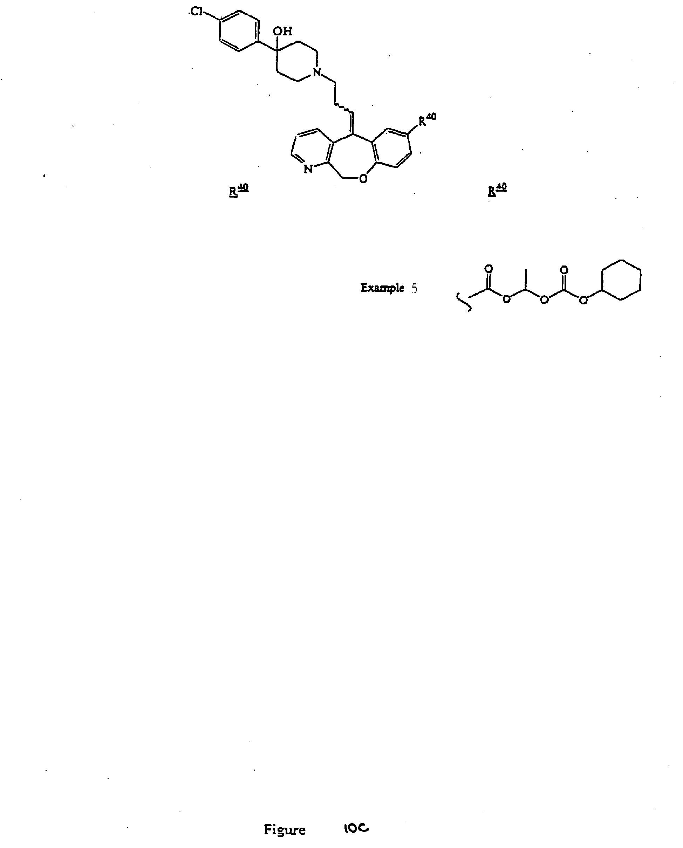

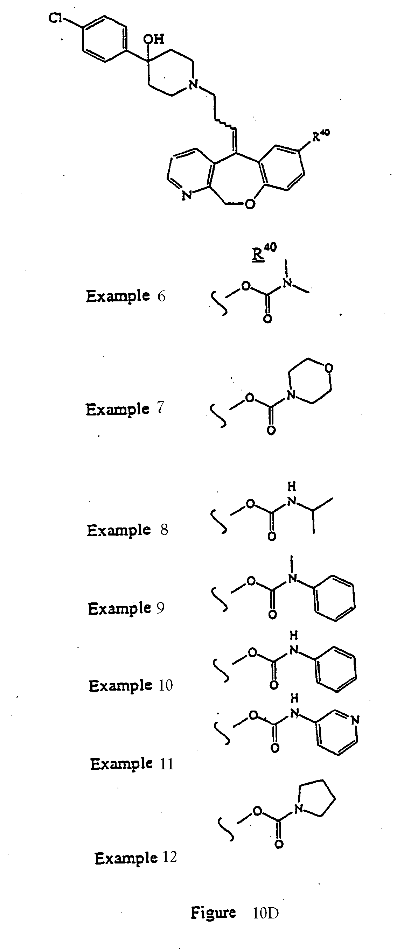

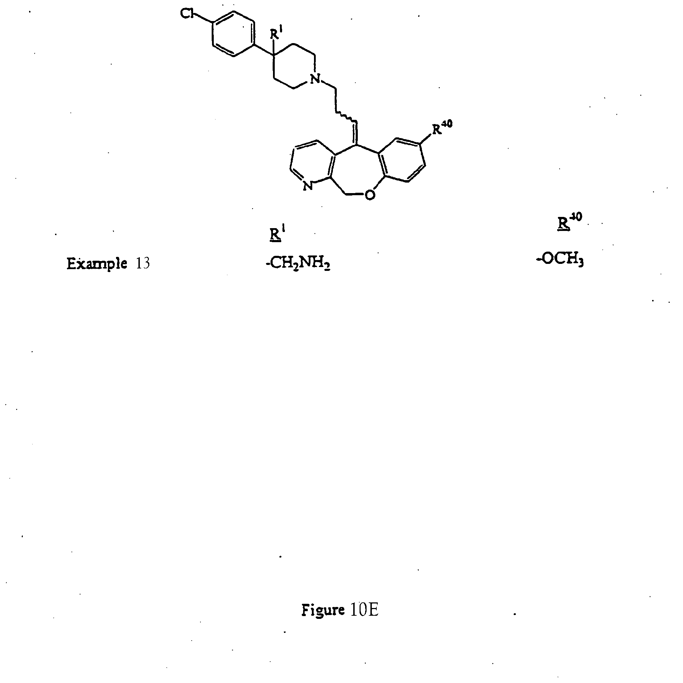

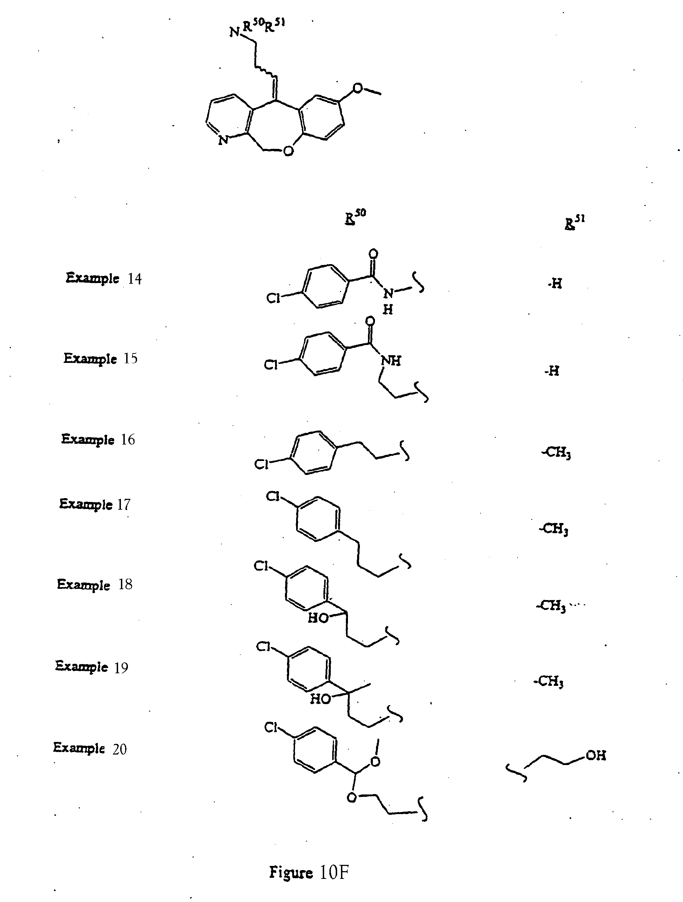

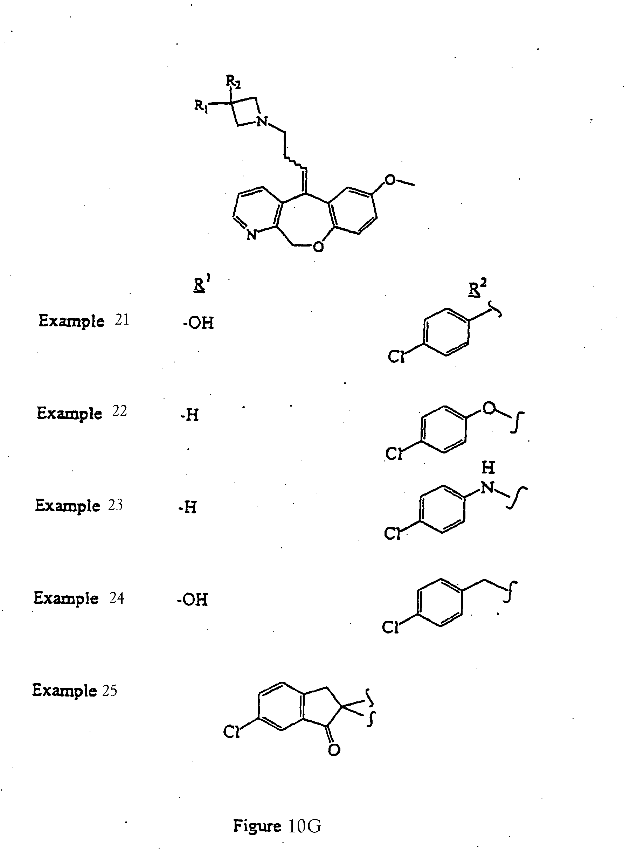

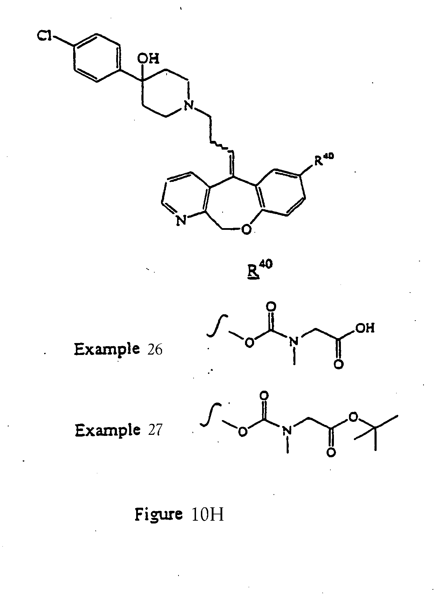

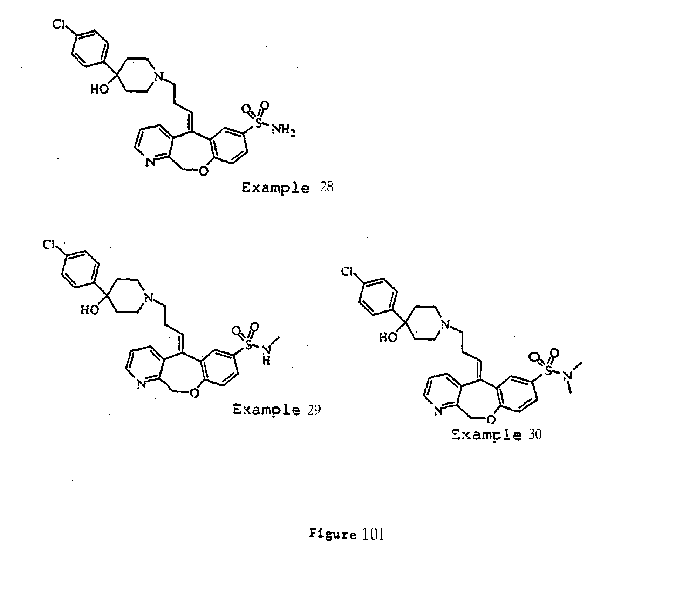

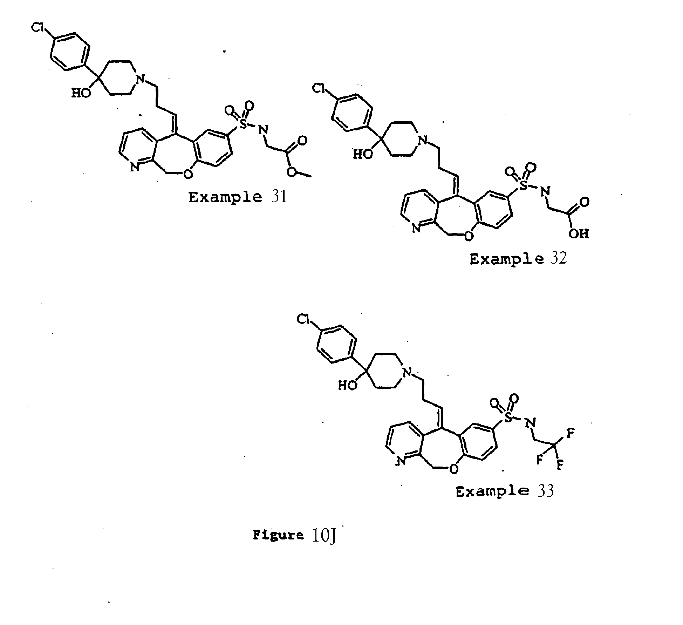

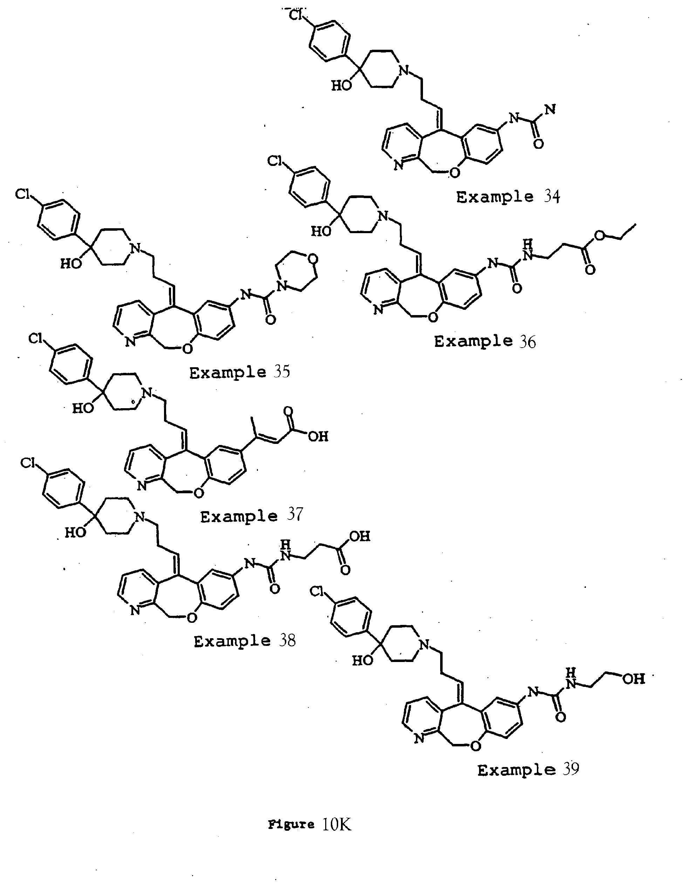

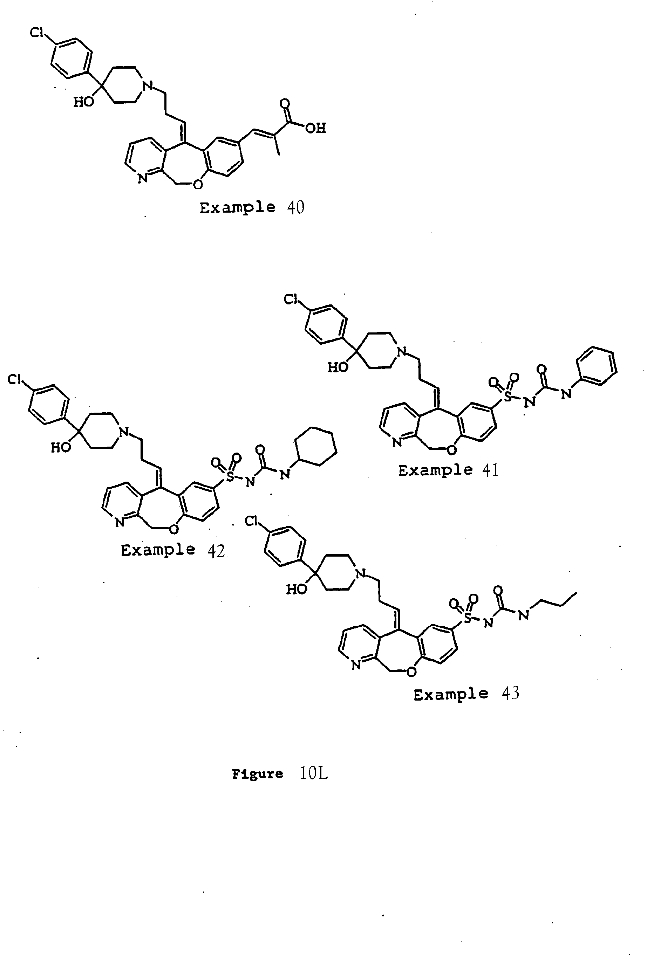

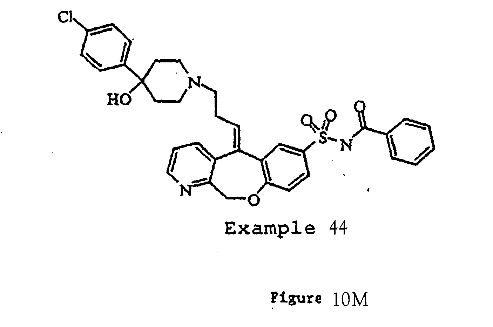

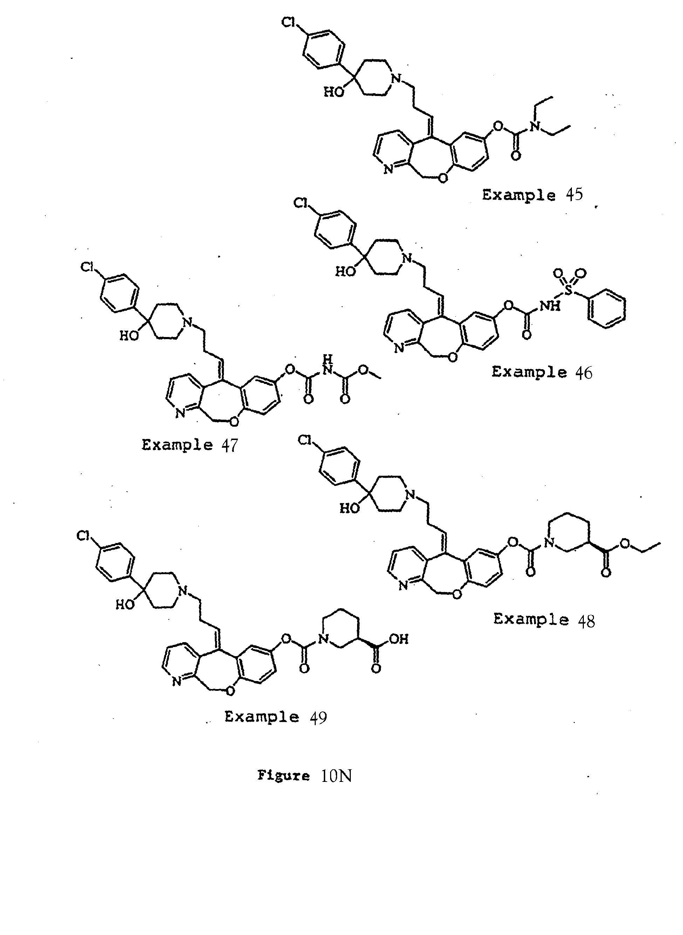

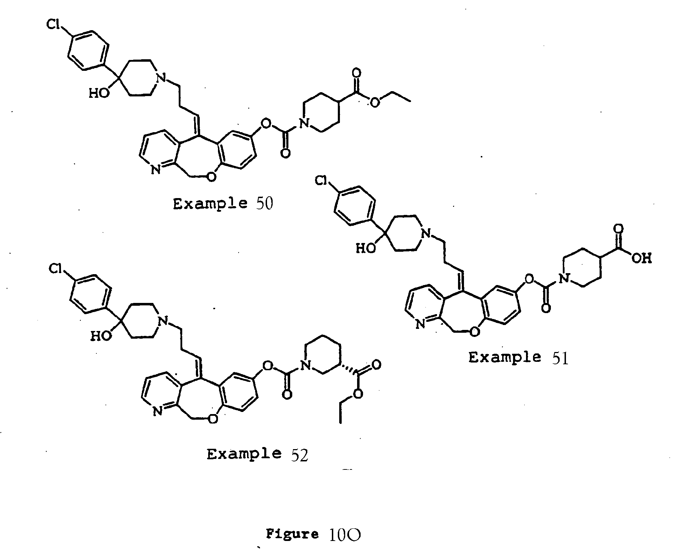

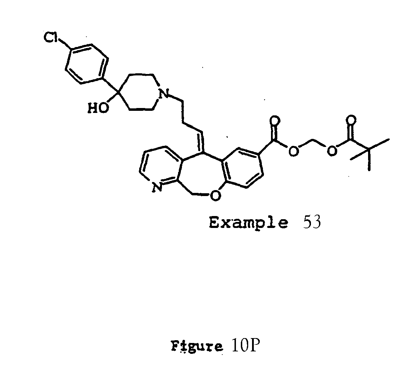

Figures 10A-10P show the structures of exemplary compounds of the present invention.

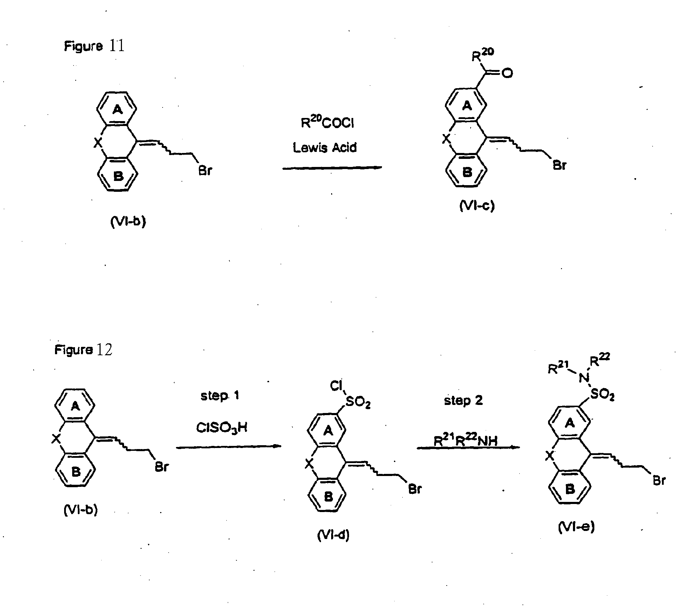

Figure 11 is a schematic showing the preparation of compounds of formula (VI-c).
Figure 12 is a schematic showing the preparation of compounds of formula (VI-e).

### DETAILED DESCRIPTION OF THE INVENTION

The present invention relates to small molecule compounds which are modulators of chemokine receptor function. In a preferred embodiment, the small molecule compounds are antagonists of chemokine receptor function. Accordingly, processes or cellular responses mediated by the binding of a chemokine to a receptor can be inhibited (reduced or prevented, in whole or in part), including leukocyte migration, integrin activation, transient increases in the concentration of intracellular free calcium [Ca⁺⁺]ᵢ, and/or granule release of proinflammatory mediators.

The invention further relates to the use of the compounds of the invention in the manufacture of a medicament for treatment, including prophylactic and therapeutic treatments, of a disease associated with aberrant leukocyte recruitment and/or activation or mediated by chemokines or chemokine receptor function, including chronic inflammatory disorders characterized by the presence of RANTES, MIP-1α, MCP-2, MCP-3 and/or MCP-4 responsive T cells, monocytes and/or eosinophils, including but not limited to diseases such as arthritis (e.g., rheumatoid arthritis), atherosclerosis, arteriosclerosis, restenosis, ischemia/reperfusion injury, diabetes mellitus (e.g., type 1 diabetes mellitus), psoriasis, multiple sclerosis, inflammatory bowel diseases such as ulcerative colitis and Crohn's disease, rejection of transplanted organs and tissues (i.e., acute allograft rejection, chronic allograft rejection), graft versus host disease, as well as allergies and asthma. Other diseases associated with aberrant leukocyte recruitment and/or activation which can be treated (including prophylactic treatments) with the methods disclosed herein are inflammatory diseases associated with Human Immunodeficiency Virus (HIV) infection, e.g., AIDS associated encephalitis, AIDS related maculopapular skin eruption, AIDS related interstitial pneumonia, AIDS related enteropathy, AIDS related periportal hepatic inflammation and AIDS related glomerulo nephritis. The method comprises administering to the subject in need of treatment an effective amount of a compound (i.e., one or more compounds) which inhibits chemokine receptor function, inhibits the binding of a chemokine to leukocytes and/or other cell types, and/or which inhibits leukocyte migration to, and/or activation at, sites of inflammation.

The invention further relates use of a compound of the invention in the manufacture of a medicament for antagonizing a chemokine receptor, such as CCR1. mammal such a medicament can be administered to a as described herein.

By administering such a medicament, chemokine-mediated chemotaxis and/or activation of pro-inflammatory cells bearing receptors for chemokines can be inhibited. As used herein, "pro-inflammatory cells" includes but is not limited to leukocytes, since chemokine receptors can be expressed on other cell types, such as neurons and epithelial cells.

While not wishing to be bound by any particular theory or mechanism, it is believed that compounds of the invention are antagonists of the chemokine receptor CCR1, and that therapeutic benefits derived from the method of the invention are the result of antagonism of CCR1 function. Thus, the compounds of the invention can be used to treat a medical condition involving cells which express CCR1 on their surface and which respond to signals transduced through CCR1, as well as the specific conditions recited above.

In one embodiment, the present invention provides a tertiary amine of the following formula: 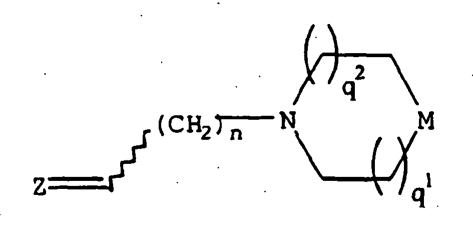 wherein:
n is one to four,
M is >CR¹R², -O-CR¹R²-O- or -CH₂-CR¹R²-O-;
q¹ is zero to three;
q² is zero or one;
R¹ is a substituted aliphatic group or an aminoalkyl group;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R⁵ and R⁶ taken together with the atom to which they are bonded, form a substituted or unsubstituted non-aromatic carbocyclic or heterocyclic ring,
Z is: 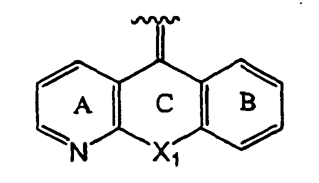
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond,
ring A and ring B are independently substituted or unsubstituted,
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group and rings A and B when substituted comprise one or more substitutents selected from the group consisting of an electron withdrawing group, halo, azido, -CN, -COOH, -OH, -CONR²⁰R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤCH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂-NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a **C₆** aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings;
or a physiologically acceptable salt thereof, other than:

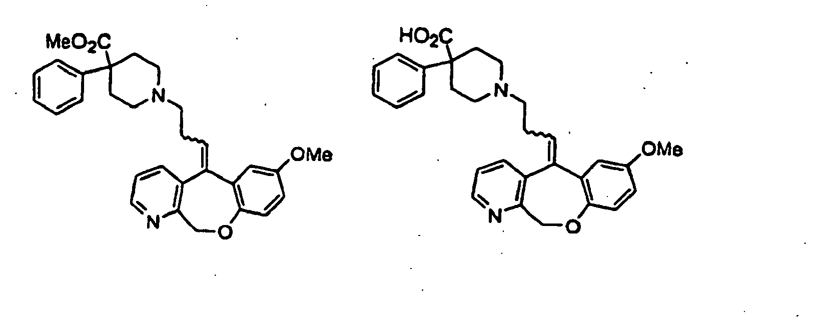

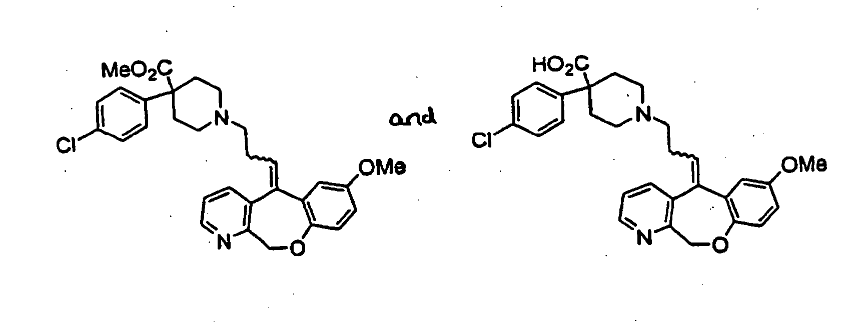

In this embodiment, it is preferred that q¹ and q² are each one.

It is further preferred that M is >CR¹R²

It is additionally preferred that R² is a substituted aromatic group, such as

R² is 4-chlorophenyl.

In accordance with this embodiment, it is preferred that R¹ is an alkylamino group selected from the group consisting of aminomethyl, 2-aminoethyl, 3-aminopropyl, 4-aminobutyl, dimethylaminoethyl, diethylaminomethyl, methylaminohexyl and aminoethylenyl.

In accordance with this embodiment, it is also preferred that R' is a substituted linear, branched or cyclic C₁-C₂₀ alkyl, alkenyl or alkynyl group.

In one embodiment, the present invention also provides a tertiary amine of the following formula: 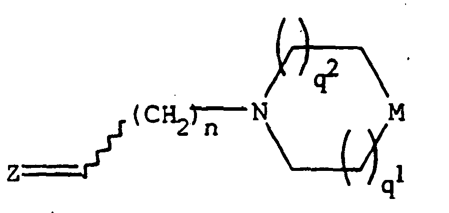 wherein:
n is one to four;
M is >NR², >CR¹R², -O-CR¹R²-O-or -CH₂-CR¹R²-O-;
q¹ is zero to three;
q² is zero or one;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -O-(substituted or unsubstituted aromatic group);
R³ and R⁴ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R³ and R⁴ taken together with the atom to which they are bonded, form a substituted or unsubstituted non-aromatic carbocyclic or heterocyclic ring;
Z is: 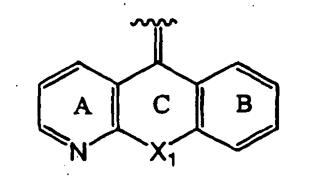
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond;
ring A and ring B are independently substituted or unsubstituted,
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜC(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜNHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group and rings A and B when substituted comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜC(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring; or
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³), -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ C₆ aromatic heterocycle that is fused to one or more other rings;
or a physiologically acceptable salt thereof.

In this embodiment, it is preferred that q¹ and q² are each one.

It is further preferred that M is >CR¹R²

It is additionally preferred that R¹ is -OH.

In one embodiment, the present invention provides a tertiary amine of the following formula: 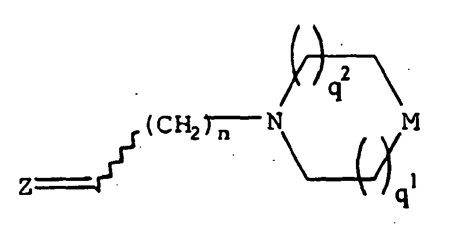 wherein:
n is one to four,
M is -O-CR¹R²-O- or -CH₂-CR¹R²-O-;
q¹ is zero to three;
q² is zero or one;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is: 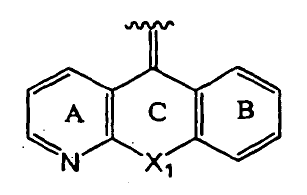
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond;
ring A and ring B are independently substituted or unsubstituted;
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O,N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings;
or a physiologically acceptable salt thereof.

In this embodiment, it is preferred that q¹ and q² are each one.

It is further preferred that R¹ is -OH and R² is a substituted aromatic group, such as

4-chlorophenyl.

In one embodiment, the present invention provides a tertiary amine of the following formula: 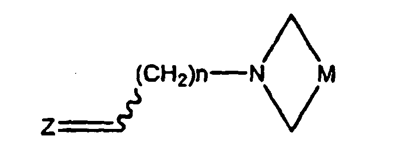 wherein:
n is one to four;
M is >NR² or >CR¹R²;
R¹ is -H, -OH,-N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is: 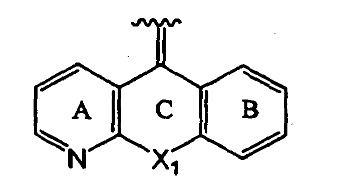
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond;
ring A and ring B are independently substituted or unsubstituted;
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)₁-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group),-Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², ₋(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²² , -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings; or a physiologically acceptable salt thereof.

In this embodiment, it is preferred that M is >CR¹R².

It is further preferred that R¹ is -OH and R² is a substituted aromatic group, such as

4-chlorophenyl.

In one embodiment, the present invention provides a tertiary amino of the following formula: 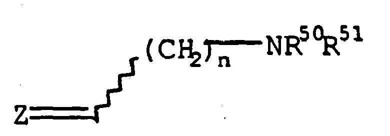 wherein:
n is one to four;
R⁵⁰ and R⁵¹ are each, independently, -H, an aliphatic group, a substituted aliphatic group, an aminoalkyl group, -NR³R⁴, an aromatic group, a substituted aromatic group, a benzyl group, a substituted benzyl group, a non-aromatic heterocyclic group, a substituted non-aromatic heterocyclic group, or a covalent bond between the nitrogen atom an adjacent carbon atom;
R³ and R⁴ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group;
Z is: 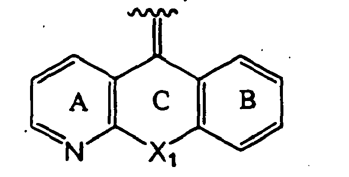
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond;
ring A and ring B are independently substituted or unsubstituted,
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ₋NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group and rings A and B when substituted comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)-(CH₂)ₜ-OC(O)R²⁰, -(O)ᵤ-(CH₂)ₜC(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, NHC(O)-O-(aliphatic group), -NHC(O)-O-(aromatic group) or -NHC(O)-O-(non-aromatic heterocyclic group); or
R²¹ and R²¹, taken together with the nitrogen atom to which they are bonded, form a non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings,
or a physiologically acceptable salt thereof.

In one embodiment, the present invention provides a tertiary amino of the following formula: 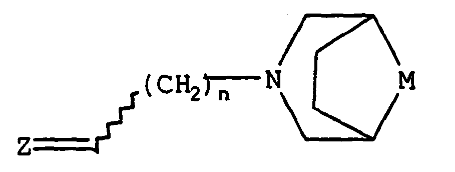 wherein:
n is one to four;
M is >NR², >CR¹R², -O-CR¹R²-O- or -CH₂-CR¹R²⁰-O-;
the ring containing M is substituted or unsubstituted;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is: 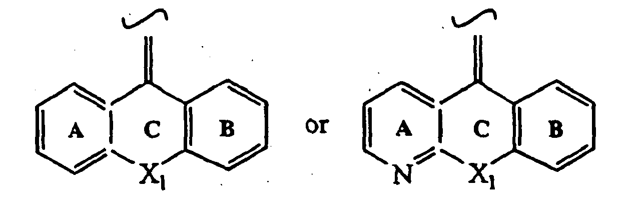
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond; and
ring A and ring B are independently substituted or unsubstituted,
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO_{3H}, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group and rings A and B when substituted comprise one or more substitutents selected from the group consisting of an electron withdrawing group, halo, azido, -CN, -COOH, -OH, -CONR²⁴R²⁵, -NR²⁴R²⁵, -OS(O)NR2⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁ -C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings,
or a physiologically acceptable salt thereof.

In this embodiment, it is preferred that Z is: 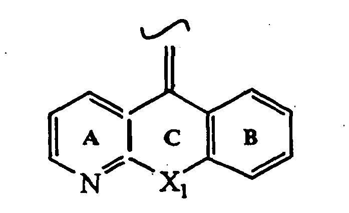 wherein:
X₁ is -CH₂-S-, -S-CH₂-, -O-CH₂-, CH₂-O-, -SO-CH₂-, -CH₂-SO-, -S(O)₂-CH₂-, -CH₂-S(O)₂- or a bond; and
ring A and ring B are independently substituted or unsubstituted,

In the above embodiments of the invention, it is preferred that ring B is substituted para to the carbon atom of ring B that is bonded to X₁ in ring C, such that Z is: 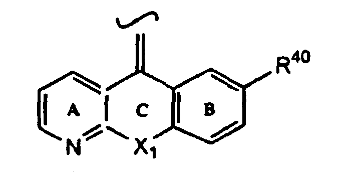 wherein:
R⁴⁰ is -OH, -COOH, -NO₂, halogen, aliphatic group, substituted aliphatic group, an aromatic group, a substituted aromatic group, -NR²⁴R²⁵, -CONR²⁴R²⁵, Q-(aliphatic group), Q-(substituted aliphatic group), -O-(aliphatic group), -O-(substituted aliphatic group), -O-(aromatic group), -O-(substituted aromatic group), an electron withdrawing group, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²² or -(O)ᵤ-(CH₂)ₜ-NHC(O)O-R²⁰;
R²⁰, R²¹ or R²² are independently -H, an aliphatic group, a substituted aliphatic group, an aromatic group, a substituted aromatic group or a non-aromatic heterocyclic group; or
R²¹ and R²², taken together with the nitrogen atom to which they are bonded form a non-aromatic heterocyclic ring;
Q is -NR²⁴C(O)-, -NR²⁴S(O)₂- or -C(O)O-;
R²⁴ and R²⁵ are independently -H, -OH, an aliphatic group or a substituted aliphatic group;
u is zero or one; and
t is an integer from zero to 3.

In the above embodiment, it is alternatively preferred that ring B is substituted para to the carbon atom of ring B that is bonded to X₁ in ring C, such that Z is: 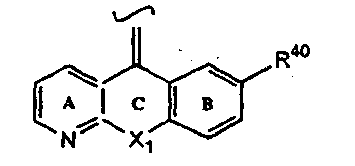 wherein:
R⁴⁰ is -C(=NR⁶⁰)NR²¹R²², -O-C(O)-NR²¹R²⁶, -S(O)₂-NR²¹R²² or -NH-C(O)NR²¹R²²; wherein
R²¹ and R²² are independently -H, an aliphatic group, a substituted aliphatic group, an aromatic group, a substituted aromatic group or a non-aromatic heterocyclic group; or
R²¹ and R²², taken together with the nitrogen atom to which they are bonded, form a substituted or unsubstituted non-aromatic heterocyclic ring;
R²⁶ is -H, an aliphatic group, a substituted aliphatic group, an aromatic group, a substituted aromatic group, a non-aromatic heterocyclic group, -C(O)-O-(substituted or unsubstituted aliphatic group), -C(O)-O-(substituted or unsubstituted aromatic group), -S(O)₂-(substituted or unsubstituted aliphatic group), -S(O)₂-(substituted or unsubstituted aromatic group); or
R²⁶ and R²¹, taken together with the nitrogen atom to which they are bonded, form a substituted or unsubstituted non-aromatic heterocyclic ring.
   In one embodiment, the present invention provides a compound which is a tertiary amine of the following formula: 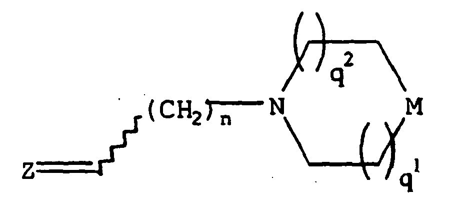
wherein:
n is one to four,
M is >NR², >CR¹R², -O-CR¹R²-O- or -CH₂-CR¹R²-O-;
q¹ is zero to three;
q² is zero or one;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R³, R⁴ R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is: 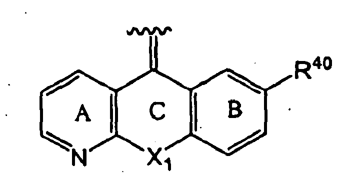
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂-, or a bond;
R⁴⁰ is -S(O₂)-NR²¹R²², -NH-C(O)-NR²¹R²², -O-C(O)-NR²¹R²⁶, 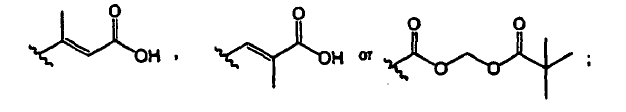
wherein:
said substituted aliphatic group comprises one or more substituents selected from the group consisting of an electron withdrawing group, halo, azido, -COOH, -OH, ₋CONR²⁴R²⁵, ₋NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵,-S(O)₂NR²⁴R²⁵, -SO₃H, -S(O)₂NH₂, guanidino, -(O)ᵤ-(CH₂)ₜ-C(O)OR²⁰, -(O)ᵤ-(CH2)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)p-(non-aromatic heterocyclic group), aliphatic group, substituted aliphatic group, =O, =S, NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-ammatic heterocyclic group);
R²⁶ is -H, aliphatic group, substituted aliphatic group, aromatic group, substituted aromatic group, non-aromatic heterocyclic group, -C(O)-O-(substituted or unsubstituted aliphatic group), -C(O)-O-(substituted or unsubstituted aromatic group), -S(O)₂-(substituted or unsubstituted aliphatic group), -S(O)₂-substituted or unsubstituted aromatic group; or
R²¹ and R²², taken together with the nitrogen atom to which they are bonded, form a non-aromatic heterocyclic ring, or
R²¹ taken together with R²⁶ and the nitrogen atom to which they are bonded form la substituted or unsubstituted non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)-or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings;
or a physiologically acceptable salt thereof.

In this embodiment, it is preferred that q¹ and q² are each one.

It is further preferred that M is >CR¹R².

It is additionally preferred that R¹ is -OH and R² is a substituted aromatic group, such as

4-chlorophenyl.

In the above embodiments, it is preferred that X₁ is -CH₂-O-

The present invention also provides a tertiary amine of the following formula: 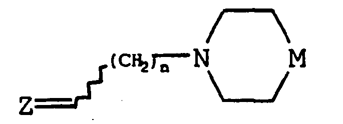 wherein:
M is CR¹R²;
R¹ is -OH;
R² is 4-chlorophenyl;
n is two;
Z is: 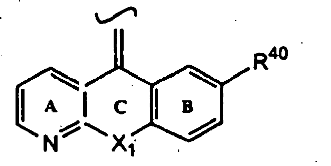
X₁ is -CH₂-O-; and
R⁴⁰ is 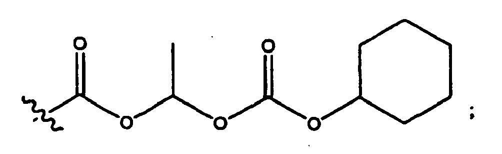 or a physiologically acceptable salt thereof.

We also disclose, the antagonist of chemokine receptor function represented by Structural Formula (I): 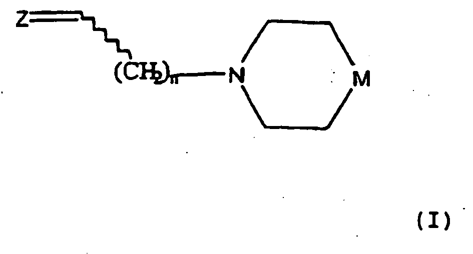 and physiologically acceptable salts thereof.

Z is a cycloalkyl or non-aromatic heterocyclic ring group fused to a phenyl and a pyridyl ring of two phenyl rings, wherein each ring in Z is independently substituted or unsubstituted.

n is an integer, such as an integer from one to four. Preferably, n is one, two or three. More preferably n is two. In alternative embodiments, other aliphatic or aromatic spacer groups (L) can be employed for (CH₂)ₙ.

M is >NR² or >CR¹R². M is preferably >C(OH)R².

R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴; or R¹ can be a covalent bond between the ring atom at M and an adjacent carbon atom in the ring which contains M. R¹ is preferably -H or -OH.

R² is , -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group). R² is preferably an aromatic group or a substituted aromatic group.

R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group.

R¹ and R², R³ and R⁴, or R⁵ and R⁶ taken together with the atom to which they are bonded, can alternatively form a substituted or unsubstituted non-aromatic carbocyclic or heterocyclic ring.

In embodiments where M is >CR¹R² and R¹ is a covalent bond between the carbon atom at M and an adjacent carbon atom in the ring which contains M, the antagonist of chemokine function can be represented by Structural Formula (Ia).

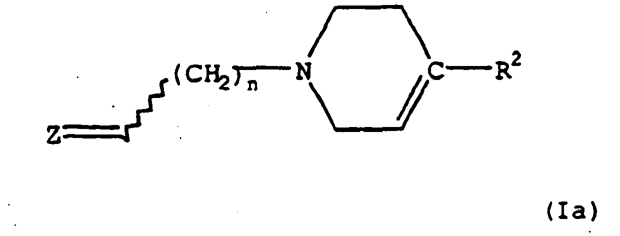

z, n and R² are as described in Structural Formula (I).

In one preferred embodiment, Z is a tricyclic ring system comprising two carbocyclic aromatic groups fused to a five, six, seven or eight membered cycloalkyl group or to a non-aromatic heterocyclic ring. In one example, Z is represented by Structural Formula (II):

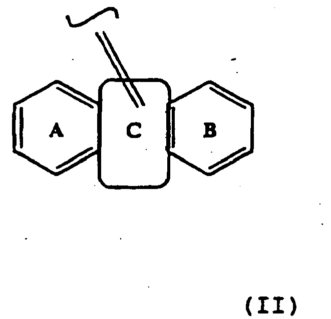

The phenyl rings in Structural Formula (II), labeled with an "A" and "B", are referred to herein as "Ring A" and "Ring B", respectively. The central ring, labeled with a "C", is referred to as "Ring C" and can be, for example, a five, six, seven or eight membered non-aromatic carbocyclic ring (e.g., a cycloheptane or cyclooctane ring) or a non-aromatic heterocyclic ring. When Ring C is a non-aromatic heterocyclic ring, it can contain one or two- heteroatoms such as nitrogen, sulfur or oxygen. In particular embodiments, Ring c is When Z is represented by Structural Formula (II), the tricyclic ring system can be connected to the remainder of the molecule by a covalent double bond between a carbon atom in Ring C and the carbon atom which, as depicted in Structural Formula (I), is bonded to Z.

Ring A and/or Ring B in Structural Formula (II) can be unsubstituted. Alternatively, Ring A and/or Ring B can have one or more substituents. Suitable substituents are as described hereinbelow. In one example, Ring A or Ring B is substituted with -(O)ᵤ-(CH₂)ₜ-C(O)OR²⁰,
-(O)ᵤ-(CH₂)ₜ-OC(O)R²⁰, -(O)ᵤ-(CH₂)ₜ-C(O)-NR²¹R²² or
- (O)ᵤ-(CH₂)ₜ-NHC(O)O-R²⁰.

u is zero or one.

t is an integer, such as an integer from zero to about three, and the methylene group -(CH₂)ₜ- can be substituted, as described herein for aliphatic groups, or unsubstituted.

R²⁰, R²¹ or R²² are independently -H, an aliphatic group, a substituted aliphatic group, an aromatic group, a substituted aromatic group or a non-aromatic heterocyclic group. Alternatively, R²¹ and R²², taken together with the nitrogen atom to which they are bonded, can form a non-aromatic heterocyclic ring.

Ring C optionally contains one or more substituents, as described hereinbelow.

Examples of suitable tricyclic ring systems, Z, are provided by Structural Formula (III):

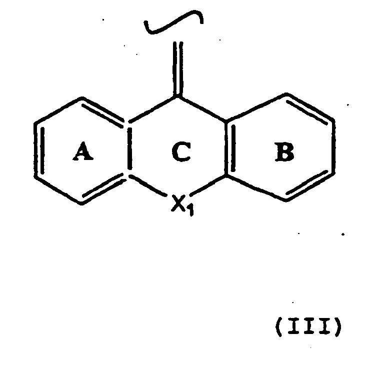

Ring A and Ring B in Structural Formula (III) are as described for Structural Formula (II).

X₁ is a bond, -CH₂-S-, -S-CH₂-, -O-CH₂-, -CH₂-O-,-SO-CH₂-, -CH₂-SO-, -S(O)₂-CH₂-or -CH₂-S(O)₂-, Preferably X₁, is -CH₂-O-, -CH₂-CH₂- or -CH₂-S-.

In another embodiment of the present invention Z is represented by Structural Formula (V):

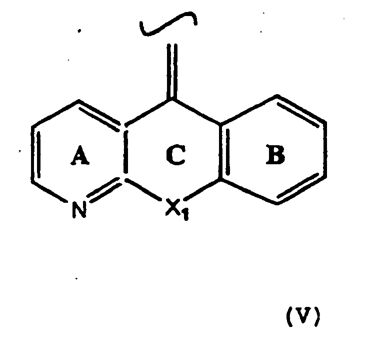

Ring A and Ring B can be independently substituted or unsubstituted as described above in Structural Formula (II), and X₁ can be as described above for Structural Formula (III).

In a preferred embodiment, Ring B in Structural Formula (V) is substituted para to the carbon atom of Ring B which is bonded to X₁ of Ring C, and Z is represented by Structural Formula (VI):

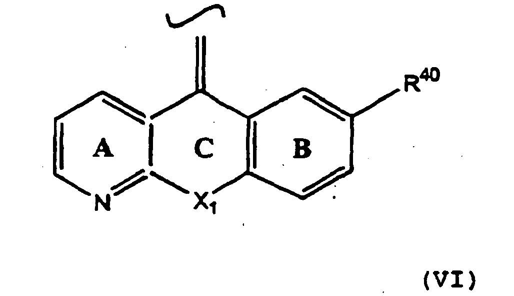

X₁ can be as described above in Structural Formula (II). Preferably X₁ is -CH₂-O-, -CH₂-CH₂- or -CH₂-S-.

R⁴⁰ is a substituent as described herein for aromatic groups. In one embodiment, R⁴⁰ is -OH, -COOH, a halogen, -NO₂, an aliphatic group, a substituted aliphatic group, an aromatic group, a substituted aromatic group, -NR²⁴R²⁵, -CONR²⁴R²⁵ , -C(=NR⁶⁰)NR²¹R²², -Q-(aliphatic group), -Q-(substituted aliphatic group),-O-(aliphatic group), -O-(substituted aliphatic group),-O-(aromatic group), -O-(substituted aromatic group), an electron withdrawing group, - (O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²² or -(O)ᵤ-(CH₂)ₜ-NHC(O)O-R²⁰. Q, R²⁰, R²¹, R²², R²⁴, R²⁵, R⁶⁰, u and t are as described herein.

Preferably R⁴⁰ is an aliphatic group, substituted aliphatic group, -O-(aliphatic group) or -O-(substituted aliphatic group). More preferably R⁴⁰ is an -O-alkyl, such as -O-CH₃, -O-C₂H₅, -O-C₃H₇ or -O-C₄H₉.

In another embodiment, R⁴⁰ can be represented by -(O)ᵤ-(CH₂)ₜ-C(O)-NR²¹R²², wherein u is one, t is zero, and R²¹ and R²² are as described herein. In this embodiment, R²¹ and R²² can each independently be -H, a substituted or unsubstituted aliphatic group, a substituted or unsubstituted aromatic group, or R²¹ and R²² taken together with the nitrogen atom to which they are bonded form a substituted or unsubstituted nonaromatic heterocyclic ring (e.g., pyrrolidine, piperidine, morpholine).

In another embodiment, R⁴⁰ can be represented by -(O)ᵤ-(CH₂)ₜ-C(O)-NR²¹R²², wherein u is zero, t is one to about three, and R²¹ and R²² are as described herein.

In another embodiment, R⁴⁰ can be represented by -(O)ᵤ-(CH₂)ₜ-C(O)-NR²¹R²², wherein both u and t are zero, and R²¹ and R²² are as described herein.

In another embodiment, R⁴⁰ is an aliphatic group (e.g., methyl, ethyl, propyl) that is substituted with **-**NR²⁴R²⁵ or -CONR²⁴R²⁵, wherein R²⁴ and R²⁵ are as described herein. For example, R⁴⁰ can be represented by

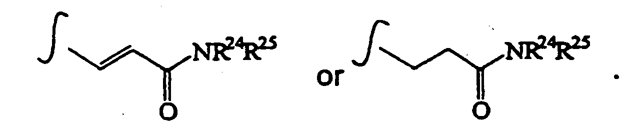

In another embodiment, R⁴⁰ is -O-C(O)-NR²¹R²⁶, wherein R²¹ is as described herein, R²⁶ can be -H, an aliphatic group, a substituted aliphatic group, an aromatic group, a substituted aromatic group, a non-aromatic heterocyclic group, -C(O)-O-(substituted or unsubstituted aliphatic group), -C(0)-O-(substituted or unsubstituted aromatic group), -S(O)₂-(substituted or unsubstituted aliphatic group), -S(O)₂-(substituted or unsubstituted aromatic group) or R²¹ and R²⁶, taken together with the nitrogen atom to which they are bonded, can form a substituted or unsubstituted non-aromatic heterocyclic ring.

In additional embodiments, R⁴⁰ can be -S(O)₂-NR²¹R²² or -N-C(O)-NR²¹R²², wherein R²¹ and R²² are as described herein.

In a preferred embodiment, the chemokine receptor antagonist can be represented by Structural Formula I wherein n is three, M is C(OH)R², R² is a phenyl group or a halophenyl group (e.g., 4-chlorophenyl) and Z is represented by Structural Formula (VI) wherein X₁ is -CH₂-O-. In one example of this embodiment, R⁴⁰ can be -O-(substituted aliphatic group), such as

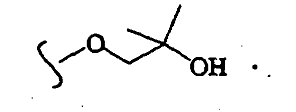

In particularly preferred embodiments, R⁴⁰ is 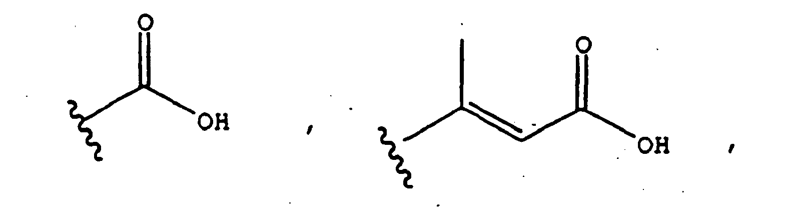 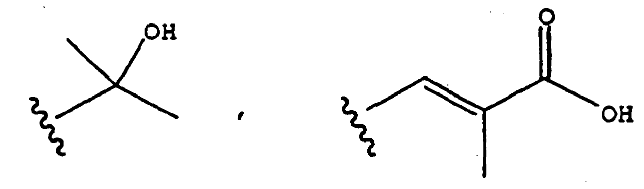 or

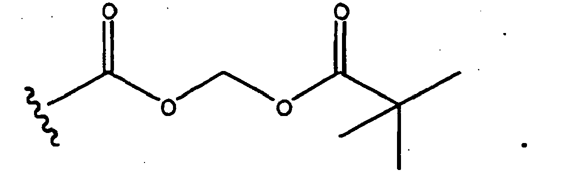

In another embodiment, the antagonist of chemokine activity can be represented by Structural Formula (VII): 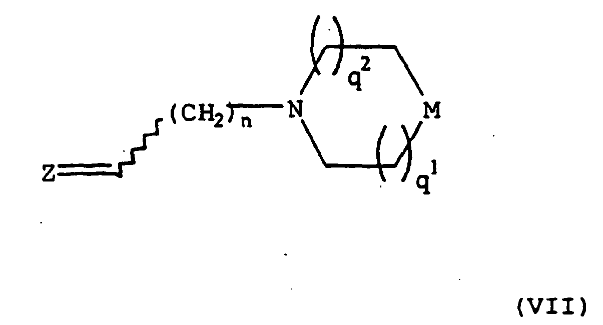 and physiologically acceptable salts thereof.

n is as described in Structural Formula (I). Z is as described herein, preferably as described in Structural Formula (V) or (VI).

M is >NR², >CR¹R², -O-CR¹R²-O- or -CH₂-CR¹R²-O-.

R¹ and R² are as described in Structural Formula (I).

q¹ is an integer, such as an integer from zero to about three, and q² is an integer from zero to about one. The ring containing M can be substituted or unsubstituted.

Thus, the antagonist of chemokine function can be represent by, for example, Structural Formulas (VIIa)-(VIIk): 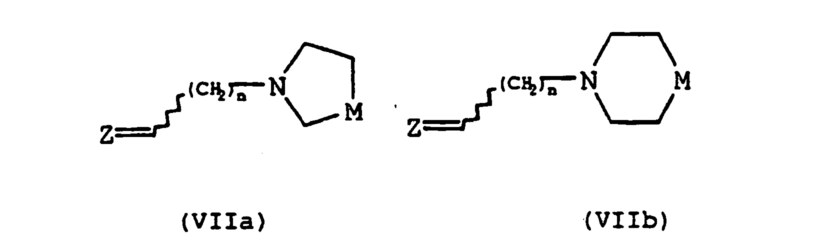 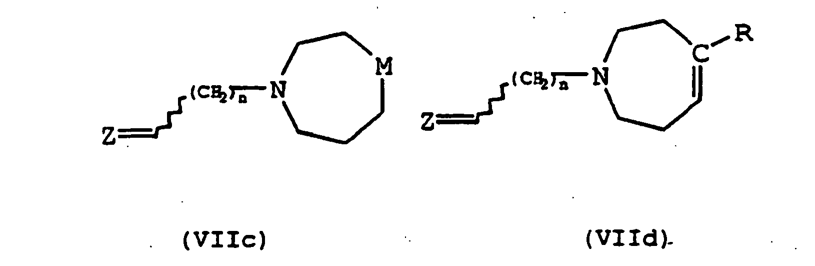 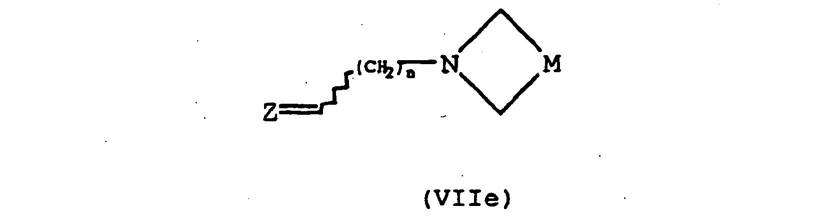 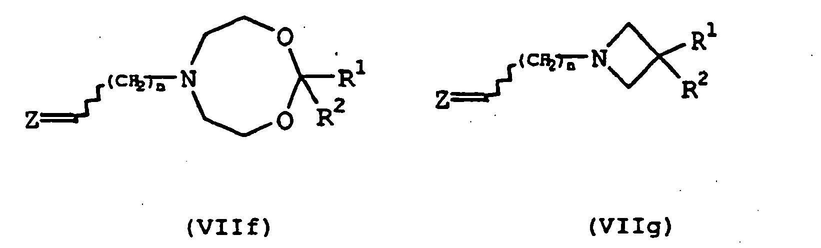 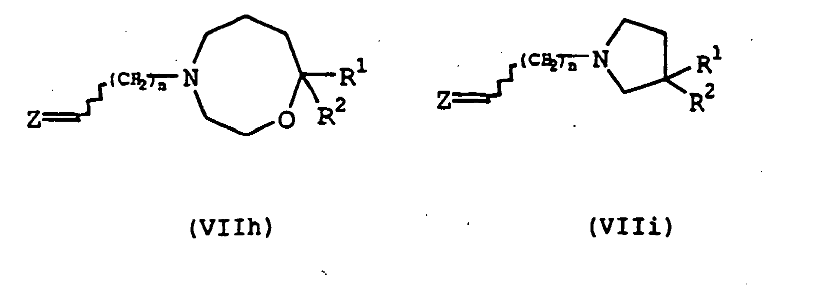 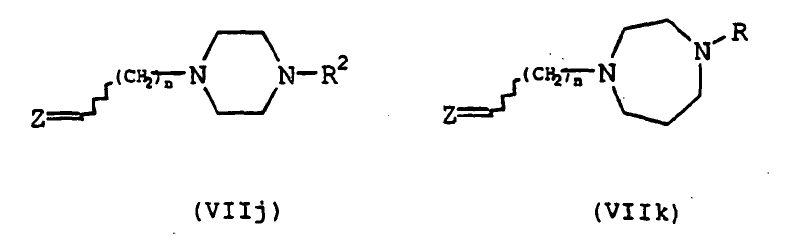 and physiologically acceptable salts thereof, wherein Z, n and M are as described in Structural Formula (VII), and the ring which contains M is substituted or unsubstituted. The ring containing M can have one or more suitable substituents which are the same or different. Suitable substituents for the ring which contains M and other nonaromatic heterocyclic rings are as described herein. For example, the ring containing M can be substituted with a methyl, ethyl, propyl, butyl or oxo group.

The nitrogen atom in the ring containing M can be a tertiary nitrogen as depicted in Structural Formula (IV), or the nitrogen atom can be quaternized with a suitable substituent, such as a C₁ to about C₆ or a C₁ to about C₃ substituted or unsubstituted aliphatic group. Compounds which comprise a quaternary nitrogen atom can also contain a counteranion such as chloride, bromide, iodide, acetate, perchlorate and the like.

The antagonist of chemokine function can be represented by Structural Formula (VII) wherein the heterocyclic ring containing M is substituted with a suitable bivalent group which is bonded to two atoms that are in the ring, thereby forming a bicyclic moiety. Suitable bivalent groups include, for example, substituted or unsubstituted bivalent aliphatic groups, such as a C₁-C₆ alkylene group.

The antagonist of chemokine receptor function can comprise a variety of bicyclic moieties. In one embodiment, the antagonist of chemokine receptor function can be represented by Structural Formula (VIII): 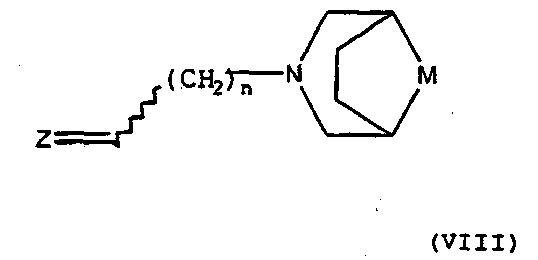 and physiologically acceptable salts thereof.

M is >NR², >CR¹R², -O-CR¹R²-O- or -CH₂-CR¹R²-O-. Preferably, M is >NR² or >CR¹R². R¹ and R² are as described in Structural Formula (I), and n and Z are as described in structural Formula (VII).

In another embodiment, the antagonist of chemokine receptor function is represented by Structural Formula (IX): 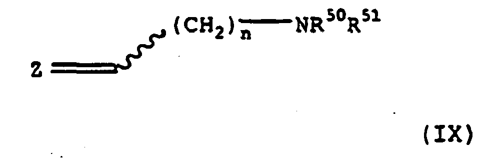 and physiologically acceptable salts thereof.

Z is as described herein, preferably as described in Structural Formula (V) or (VI).

n is an integer, such as an integer from one to about four. Preferably, n is one, two or three. More preferably n is two. In alternative embodiments, other aliphatic or aromatic spacer groups (L) can be employed for (CH₂)ₙ.

R⁵⁰ and R⁵¹ are each independently -H, an aliphatic group, a substituted aliphatic group, an aminoalkyl group, -NR³R⁴, an aromatic group, a substituted aromatic group, a benzyl group, a substituted benzyl group, a non-aromatic heterocyclic group, a substituted non-aromatic heterocyclic group or a covalent bond between the nitrogen atom an adjacent carbon atom.

R³ and R⁴ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group.

R³ and R⁴ taken together with the atom to which they are bonded, can alternatively form a substituted or unsubstituted non-aromatic carbocyclic or heterocyclic ring.

In a preferred embodiment R⁵⁰ is a substituted aliphatic group, such as a substituted C₁ to about C₁₂ alkyl group, and R⁵¹ is -H or a substituted or unsubstituted aliphatic group. More preferably, R⁵⁰ is a substituted linear or branched C₂ to about C₇ aliphatic group wherein one or more carbon atoms can be replaced by a heteroatom, such as nitrogen, oxygen or sulfur, and R⁵¹ is -H or a linear or branched C₁ to about C₆ or a C₁ to about C₃ aliphatic group wherein one or more carbon atoms can be replaced by a heteroatom. R⁵⁰ and R⁵¹ can be substituted with one or more suitable substituents, as described herein, Preferably an aromatic group(e.g., phenyl, 4-halophenyl). For example, R⁵⁰ can be selected from the group consisting of: 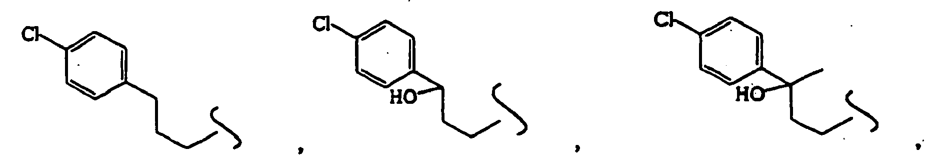 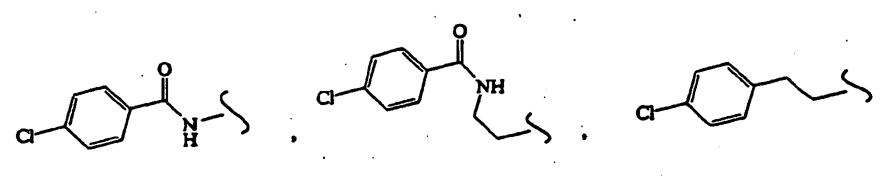 and

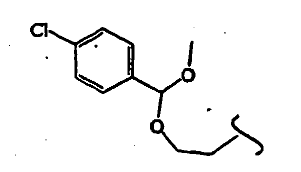

The activity of chemokine receptor antagonists represented by Structural Formula IX can be affected by the character of the nitrogen atom to which R⁵⁰ and R⁵¹ are bonded. It is believed that compounds in which said nitrogen atom is basic can have potent chemokine receptor antagonist activity. It is known that the basicity of a nitrogen atom can be decreased when the nitrogen atom is bonded to a carbonyl group, sulfonyl group or a sulfinyl group. Therefore, it is preferred that neither R⁵⁰ nor R⁵¹ comprise a carbonyl group, sulfonyl group or sulfinyl group that is directly bonded to the nitrogen atom.

The chemokine receptor antagonist described herein can be prepared and administered as active compounds or as prodrugs. Generally, prodrugs are analogues of pharmaceutical agents which can undergo chemical conversion by metabolic processes to become fully active. For example, A prodrug of the invention can be prepared by selecting appropriate groups for R⁴⁰. In one embodiment, a prodrug can be represented by Structural Formula (XI): 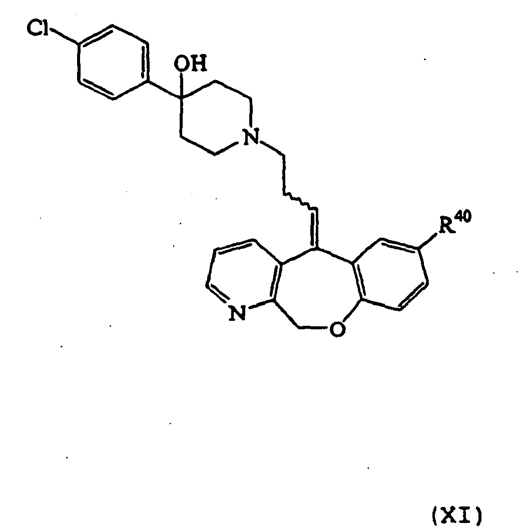 wherein, R⁴⁰ is Q-substituted aliphatic group, and the aliphatic group is substituted with -(O)ᵤ-(CH₂)ₜ-C(O)OR²⁰, wherein Q is -C (O) O-, u is one, t is zero and R²⁰ is a cyclic aliphatic group. For example, when the substituted aliphatic group is a substituted ethyl group, R⁴⁰ can be represented by: 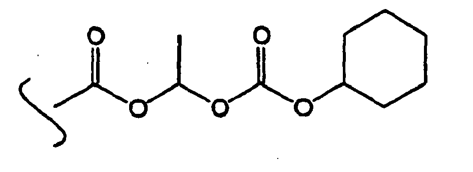 Such a prodrug can be converted to an active chemokine receptor antagonist represented by Structural Formula (XI, wherein R⁴⁰ is -COOH.

Another embodiment of the present invention includes the novel compounds disclosed herein.

The compounds disclosed herein can be obtained as E-and Z-configurational isomers. It is expressly pointed out that the invention includes compounds of the E-configuration and the Z-configuration around the double bond connecting Ring C of Z to the remainder of the molecule, and a method of treating a subject with compounds of the E-configuration, the Z-configuration, and mixtures thereof. Accordingly, in the structural formulas presented herein, the symbol: 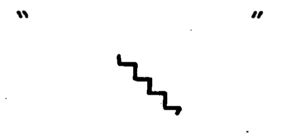 is used to represent both the E-configuration and the Z-configuration. Preferably Ring A and the alkylene chain bonded to Ring C are in the cis configuration. For example, the compounds can have the configuration of:

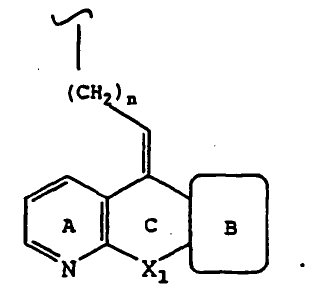

It is understood that one configuration can have greater activity than another. The desired configuration can be determined by screening for activity, employing the methods described herein.

Additionally, certain compounds of the invention may be obtained as different sterioisomers (e.g., diastereomers and enantiomers). It is pointed out that the invention includes all isomeric forms and racemic mixtures of the disclosed compounds and a method of treating a subject with both pure isomers and mixtures thereof, including racemic mixtures. Again, it is understood that one sterioisomer may be more active than another. The desired isomer can be determined by screening.

Also included in the present invention are physiologically acceptable salts of the compounds represented by Structural Formulas (I) through (XI). Salts of compounds containing an amine or other basic group can be obtained, for example, by reacting with a suitable organic or inorganic acid, such as hydrogen chloride, hydrogen bromide, acetic acid, citric acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium, ammonium, calcium and the like.

As used herein, aliphatic groups include straight chained, branched or cyclic C₂-C₂₀ hydrocarbons which are completely saturated or which contain one or more units of unsaturation. Preferred aliphatic groups are C₁ to about C₁₀ hydrocarbons. More preferred are C₁ to about C₆ or C₁ to about C₃ hydrocarbons. For example, suitable aliphatic groups include substituted or unsubstituted linear, branched or cyclic C₁-C₂₀ alkyl, alkenyl or alkynyl groups.

An aminoalkyl group is an alkyl group substituted with -NR²⁴R²³, R²⁴ and R²⁵ are as described herein. Preferably the alkyl moiety comprises one to about twelve, more preferably one to about six carbon atoms. The alkyl moiety of an aminoalkyl group can be unsubstituted or substituted as described herein for aliphatic groups. Examples of suitable aminoalkyl groups include aminomethyl, 2-aminoethyl, 3-aminopropyl, 4-aminobutyl, dimethylaminoethyl, diethylaminomethyl, methylaminohexyl, aminoethylenyl and the like.

Aromatic groups include carbocyclic aromatic groups such as phenyl, 1-naphthyl, 2-naphthyl, 1-anthracyl and 2-anthracyl, and heterocyclic aromatic or heteroaryl groups such as N-imidazolyl, 2-imidazolyl, 4-imidazolyl, 5-imidazolyl, 2-thienyl, 3-thienyl, 2-furanyl, 3-furanyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 4-pyridazinyl, 3-pyrazolyl, 4-pyrazolyl, 5-pyrazolyl, 2-pyrazinyl, 2-thiazolyl, 4-thiazolyl, 5-thiazolyl, 5-tetrazolyl, 2-oxazolyl, 4-oxazolyl and 5-oxazolyl. Where these rings are fused, for example, to Ring C, the stated point of attachment can be either of the two fused bonds.

Aromatic groups also include fused polycyclic aromatic ring systems in which a carbocyclic aromatic ring or heteroaryl ring is fused to one or more other rings. Examples include tetrahydronaphthyl, 2-benzothienyl, 3-benzothienyl, 2-benzofuranyl, 3-benzofuranyl, 2-indolyl, 3-indolyl, 2-quinolinyl, 3-quinolinyl, 2-benzothiazolyl, 2-benzooxazolyl, 2-benzimidazolyl, 2-quinolinyl, 3-quinolinyl, 1-isoquinolinyl, 3-quinolinyl, 1-isoindolyl, 3-isoindolyl, acridinyl, 3-benzisoxazolyl, and the like. Also included within the scope of the term "aromatic group", as it is used herein, is a group in which one or more carbocyclic aromatic rings and/or heteroaryl rings are fused to a cycloalkyl or non-aromatic heterocyclic ring, for example, benzocyclopentane, benzocyclohexane.

Non-aromatic heterocyclic rings are non-aromatic carbocyclic rings which include one or more heteroatoms such as nitrogen, oxygen or sulfur in the ring. The ring can be five, six, seven or eight-membered and/or fused to another ring, such as a cycloalkyl on aromatic ring. Examples include 1,3-dioxolan-2-yl, 3-1H-benzimidazol-2-one, 3-1-alkyl-benzimidazol-2-one, 3-1-methyl-benzimidazol-2-one, 2-tetrahydrofuranyl, 3-tetrahydrofuranyl, 2-tetrahyrothiophenyl, 3-tetrahyrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidyl, 1-3-alkyl-phthalimidyl, benzoxane, benzopyrolidine, benzopiperidine, benzoxolane, benzothiolane, benzothiane, tetrahydrofuran-2-one- 3-yl, 2,5-dihydro-5-oxo-4H-1,2,4-thiadiazol-3-yl, 2-oxo-3H-1, 2, 3, 5-oxathiadiazol-4-yl,

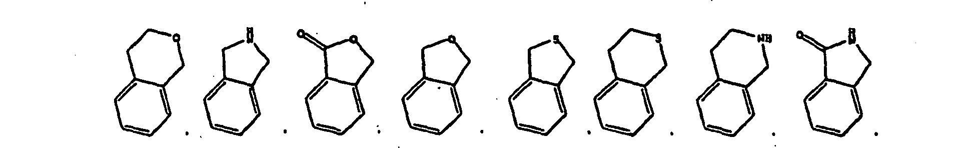

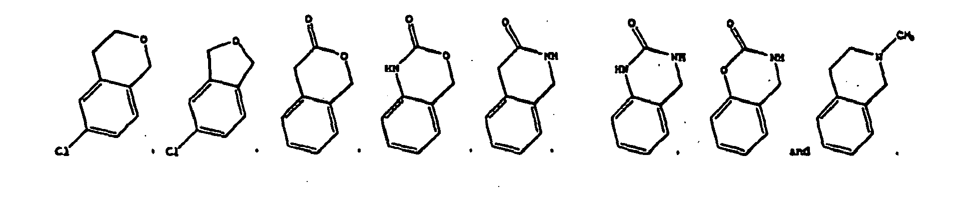

Suitable substituents on an aliphatic group, aromatic group (carbocyclic and heteroaryl), non-aromatic heterocyclic ring or benzyl group include, for example, an electron withdrawing group, a halogen, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, ureido, oxalo, amidino, -C(=NR⁶⁰)NR²¹R²², =NR⁶⁰, -(O)ᵤ-(CH₂)ₜ-C(O)OR²⁰, - (O)ᵤ-(CH₂)ₜ-OC(O)R²⁰, -(O)ᵤ-(CH₂)ₜ-C(O)-NR²¹R²², - (O)ᵤ-(CH₂)ₜ-NHC(O)O-R²⁰, -Q-H, -Q- (aliphatic group), -Q-(substituted aliphatic group), -Q-(aryl), -Q-(aromatic group), -Q-(substituted aromatic group), -Q-(CH₂)ₚ-(substituted or unsubstituted aromatic group) p is an integer from 1-5), -Q-(non-aromatic heterocyclic group) or -Q-(CH₂)ₚ-(non-aromatic heterocyclic group).

R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group) and wherein R²¹ and R²², taken together with the nitrogen atom to which they are bonded, can form a substituted or unsubstituted non-aromatic heterocyclic ring.

R⁶⁰ is a -H, -OH, -NH₂, an aromatic group or a substituted aromatic group.

t is an integer from zero to about three, and the methylene group, -(CH₂)ₜ-, can be substituted, as described herein for aliphatic groups, or unsubstituted.

u is zero or one.

Q is -O-**,** -S-, -S (O) -, -S(O)₂-, -OS(O)₂-, -C (O) -, -OC(O)-, -C(O)O-, -C(O)C(O)-O-, -O-C(O)C(O)-, -C(O)NH-, -NHC(O)-, -OC (O) NH-, -NHC(O)O-, -NH-C (O) -NH-, -S(O)₂NH-, -NHS(O)₂-, -N(R²³)-, -C(NR²³)NHNH-, -NHNHC(NR²³)-, -NR²⁴C(O)-or -NR²⁴S(O)₂-.

R²³ is -H, an aliphatic group, a benzyl group, an aryl group or non-aromatic heterocyclic group.

R²⁴ and R²⁵ are independently -H, -OH, an aliphatic-group, a substituted aliphatic group, a benzyl group, an aryl group, non-aromatic heterocyclic group or R²⁴ and R²⁵ taken together with the nitrogen atom to which they are bonded can form a substituted or unsubstituted non-aromatic heterocyclic ring.

A substituted non-aromatic heterocyclic ring, benzyl group or aromatic group can also have an aromatic group, an aliphatic or substituted aliphatic group, as a substituent. When a non-aromatic ring (carbocyclic or heterocyclic) or an aromatic ring (carbocyclic aromatic or heteroaryl) is substituted with another ring, the two rings can be fused. A substituted aliphatic group can also have an oxo group, epoxy group, non-aromatic heterocyclic ring, benzyl group, substituted benzyl group, aromatic group or substituted aromatic group as a substituent. A substituted non-aromatic heterocyclic ring can also have =O, =S, =NH or =N(aliphatic, aromatic or substituted aromatic group) as a substituent. A substituted aliphatic, substituted aromatic, substituted non-aromatic heterocyclic ring or substituted benzyl group can have more than one substituent, which can be the same or different.

Acyl groups include substituted and unsubstituted aliphatic carbonyl, aromatic carbonyl, aliphatic sulfonyl and aromatic sulfonyl.

Suitable electron withdrawing groups include, for example, alkylimines, alkylsulfonyl, carboxamido, carboxylic alkyl esters, -CH=NH, -CN, -NO₂ and halogens.

In the structural formulas depicted herein, the single or double bond by which a chemical group or moiety is connected to the remainder of the molecule or compound is indicated by the following symbol: 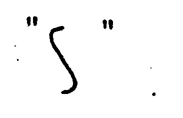 For example, the corresponding symbol in Structural Formulas (II), (III) and (IV) indicates the double bond by which the central ring of the tricyclic ring system is connected to the remainder of the molecule represented by Structural Formula (I).

A "subject" is preferably a bird or mammal, such as a human, but can also be an animal in need of veterinary treatment, e.g., domestic animals (e.g., dogs, cats, and the like), farm animals (e.g., cows, sheep, fowl, pigs, horses, and the like) and laboratory animals (e.g., rats, mice, guinea pigs, and the like).

An "effective amount" of a compound is an amount which results in the inhibition of one or more processes mediated by the binding of a chemokine to a receptor in a subject with a disease associated with aberrant leukocyte recruitment and/or activation. Examples of such processes include leukocyte migration, integrin activation, transient increases in the concentration of intracellular free calcium [Ca²⁺]₁ and granule release of proinflammatory mediators. Alternatively, an "effective amount" of a compound is a quantity sufficient to achieve a desired therapeutic and/or prophylactic effect, such as an amount which results in the prevention of or a decrease in the symptoms associated with a disease associated with aberrant leukocyte recruitment and/or activation.

The amount of compound administered to the individual will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. Typically, an effective amount of the compound can range from about 0.1 mg per day to about 100 mg per day for an adult. Preferably, the dosage ranges from about 1 mg per day to about 100 mg per day. An antagonist of chemokine receptor function can also be administered in combination with one or more additional therapeutic agents, e.g. theophylline, β-adrenergic bronchodilators, corticosteroids, antihistamines, antiallergic agents, immunosuppressive agents (e.g., cyclosporin A, FK-506, prednisone, methylprednisolone) and the like.

The compound can be administered by any suitable route, including, for example, orally in capsules, suspensions or tablets or by parenteral administration. Parenteral administration can include, for example, systemic administration, such as by intramuscular, intravenous, subcutaneous, or intraperitoneal injection. The compound can also be administered orally (e.g., dietary), transdermally, topically, by inhalation (e.g., intrabronchial, intranasal, oral inhalation or intranasal drops), or rectally, depending on the disease or condition to be treated. Oral or parenteral administration are preferred modes of administration.

The compound can be administered to the individual in conjunction with an acceptable pharmaceutical or physiological carrier as part of a pharmaceutical composition for treatment of HIV infection, inflammatory disease, or the other diseases discussed above. Formulation of a compound to be administered will vary according to the route of administration selected (e.g., solution, emulsion, capsule). Suitable carriers may contain inert ingredients which do not interact with the compound. Standard pharmaceutical formulation techniques can be employed, such as those described in Remington's Pharmaceutical Sciences, Mack Publishing Company, Easton, PA. Suitable carriers for parenteral administration include, for example, sterile water, physiological saline, bacteriostatic saline (saline containing about 0.9% mg/ml benzyl alcohol), phosphate-buffered saline, Hank's solution, Ringer's-lactate and the like. Methods for encapsulating compositions (such as in a coating of hard gelatin or cyclodextran) are known in the art (Baker, et al., "Controlled Release of Biological Active Agents", John Wiley and Sons, 1986).

The activity of compounds of the present invention can be assessed using suitable assays, such as receptor binding assays and chemotaxis assays. For example, as described in the Exemplification Section, small molecule antagonists of RANTES and MIP-1α binding have been identified utilizing THP-1 cells which bind RANTES and chemotax in response to RANTES and MIP-1α as a model for leukocyte chemotaxis. Specifically, a high through-put receptor binding assay, which monitors ¹²I-RANTES and ¹²⁵I-MIP-1α binding to THP-1 cell membranes, was used to identify small molecule antagonists which block binding of RANTES and MIP-1α. Compounds of the present invention can also be identified by virtue of their ability to inhibit the activation steps triggered by binding of a chemokine to its receptor, such as chemotaxis, integrin activation and granule mediator release. They can also be identified by virtue of their ability to block RANTES and MIP-1α mediated HL-60, T-cell, peripheral blood mononuclear cell, and eosinophil chemotactic response.

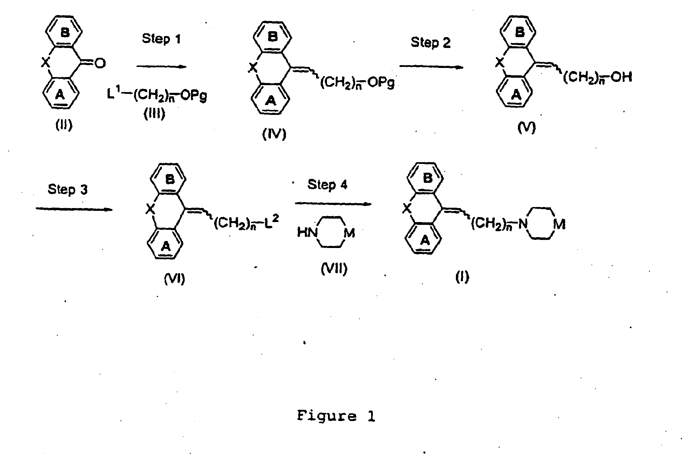

The compounds disclosed herein can be prepared accordingly to the schemes shown in Figures 1 -6 The schemes are described in greater detail below.

Figure 1 shows the preparation of compounds represented by Structural Formula (I) . L¹ is PPh₃Cl, PPh₃Br, PPh₃I or (EtO)₂P(O), L² is a suitable leaving group such as halogen, p-toluene sulfonate, mesylate, alkoxy, and phenoxy; Pg is a suitable protecting group such as tetrahydropyranyl; and the other symbols are as defined above.

In Step 1 of Figure 1, a Wittig reaction is carried out in a solvent such as ether, or tetrahydrofuran (THF) in the presence of a base such as sodium hydride, n-butyl lithium or lithium diisopropylamide (LDA) at 0°C up to the reflux temperature for the solvent used for 5 minutes to 72 h. Compounds represented by Formula II in Figure 1 can be prepared by methods disclosed in JP 61/152673, U.S. Patent 5089496, WO 89/10369, WO 92/20681 and WO 93/02081, the entire teachings of which are incorporated herein by reference.

In Step 2 of Figure 1, deprotection is carried out with an acid in a solvent such as methanol at room temperature up to the reflux temperature for the solvent used for 5 minutes to 72 h. Alternatively, a compound of represented by Formula V in Figure 1 can be prepared directly from step 1 without isolating an intermediate. The reaction mixture obtained after the work up of the reaction described in step 1 can be dissolved in the solvent and reacted with the acid.

In Step 3 of Figure 1, the hydroxy group can be converted to a leaving group by known methods. Compounds represented by Formula VI in Figure 1 can be prepared by methods disclosed in J. Med. Chem., 1992 (35) 2074-2084 and JP 61/152673.

In Step 4 of Figure 1, an alkylation reaction is carried out in a solvent such as acetone, methyl ethyl ketone, ethyl acetate, toluene, tetrahydrofuran (THF) or dimethylformamide (DMF) in the presence of a base such as potassium carbonate or sodium hydride and a catalyst such as an alkali metal iodide at room temperature up to the reflux temperature for the solvent used for 5 minutes to 72 h.

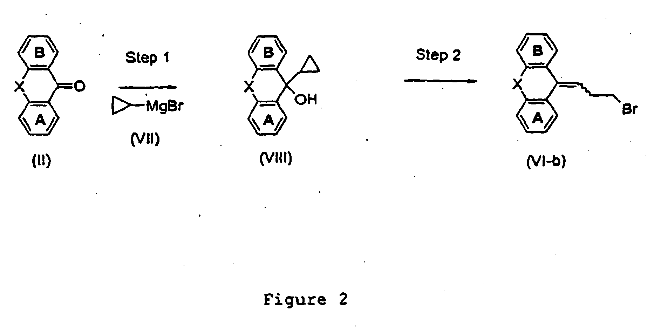

Figure 2 shows the preparation of compounds represented by Compound (VI-b). In Step 1 of Figure 2, a Grignard reaction may be carried out in a solvent such as ether, or tetrahydrofuran (THF) at 0°C up to the reflux temperature for the solvent used for 5 minuets to 72 h. Compound VII is available commercially.

In Step 2 of Figure 2, bromination may be carried out with brominate agents such as hydrobromic acid, bromotrimethylsilane or boron tribromide-methyl sulfide complex in a solvent such as acetic acid, dichloromethane or dichloroethane at room temperature up to the reflux temperature for the solvent used for 5 minutes to 72 h.

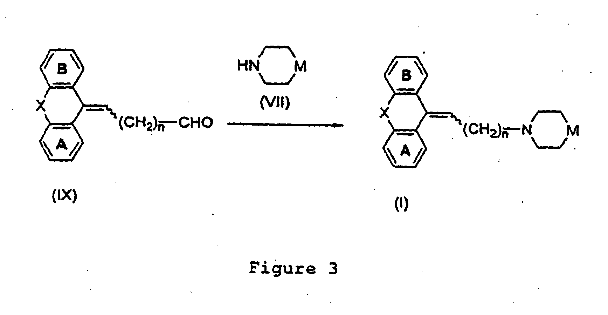

Figure 3 shows the preparation of compounds represented by Structural Formula (I). In Figure 3, a reductive amination may be carried out with reducing reagents such as sodium cyanoborohydride, sodium acetoxyborohydride or sodium borohydride in a solvent such as methanol, ethanol, tetrahydrofuran (THF), dichloromethane or dichloroethane at room temperature up to the reflux temperature for the solvent used for 5 minutes to. 7.2 h.

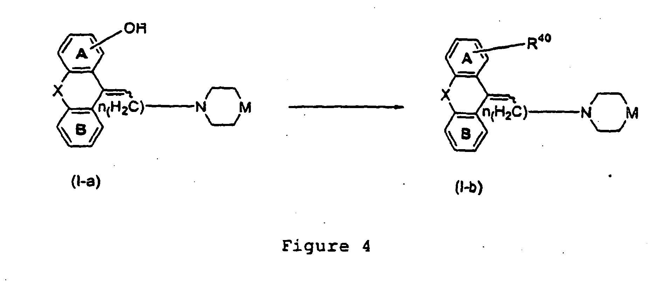

Figure 4 shows the preparation of compounds represented by Structural Formula (I), where in Z is represented by Structural Formulas (III) and wherein Ring A and/or Ring B in Z is substituted with R⁴⁰. In Figure 4, the alkylation reaction can be carried out in a solvent such as acetone, methyl ethyl ketone, ethyl acetate, toluene, tetrahydrofuran (THF) or dimethylformamide (DMF) in the presence of a base such as potassium carbonate or sodium hydride and a catalyst such as an alkali metal iodide at room temperature up to the reflux temperature for the solvent used for 5 minutes to 72 h.

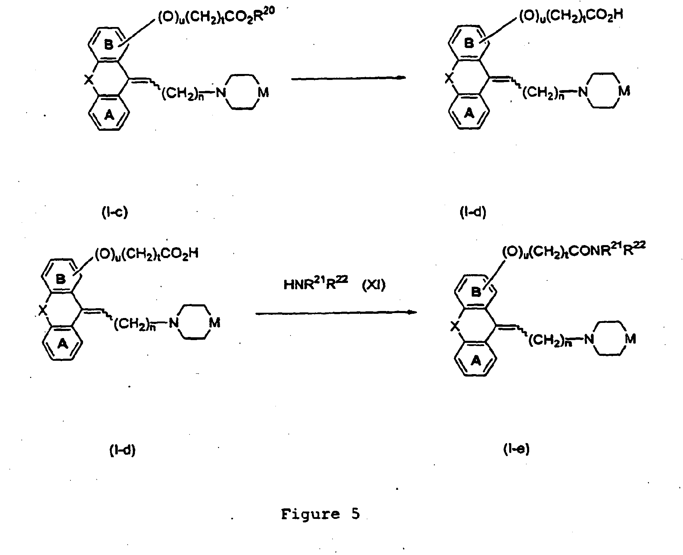

Figure 5 is a schematic showing the preparation of the compounds represented by Structural Formula (I), wherein Z is represented by Structural Formulas (III) and wherein Ring A and/or Ring B in Z is substituted with -(O)ᵤ-(CH₂)ₜ-COOR²⁰, -(O)ᵤ-(CH₂)ₜ-OC(O)R²⁰, - (O)ᵤ-(CH₂)ₜ-C(O) -NR²¹R²² or -(O)ᵤ-(CH₂)ₜ-NHC(O)O-R²⁰. In Figure 5, the hydrolysis reaction may be carried out in a mixture of aqueous alkali metal hydroxide solution and a solvent such as methanol, ethanol, tetrahydrofuran (THF) or dioxane at room temperature up to the reflux temperature for the solvent used for 5 minutes to 72 h. The acylation reaction can be carried out using dicyclohexylcarbodiimide (DCC) or (1-ethyl-3-(3- dimethylaminopropyl)carbodiimide (DEC) in a solvent such as tetrahydrofuran (THF), dimethylformamide (DMF) or methylene chloride in the presence of a base such as pyridine or triethylamine (when necessary) at temperatures of 0 to 100°C for 5 minutes to 72 h.

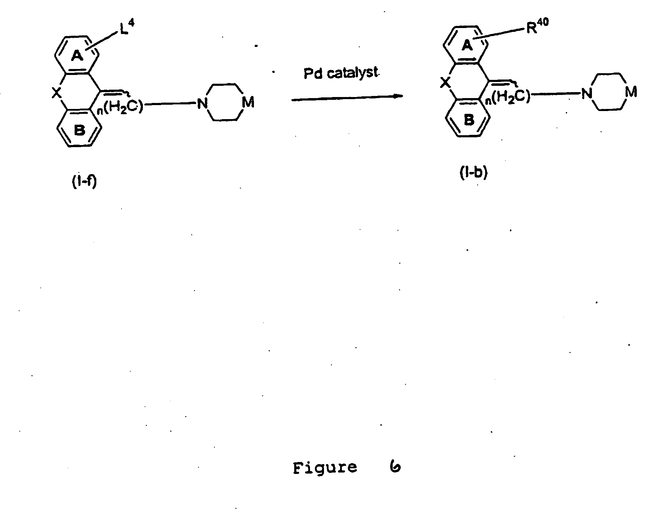

Figure 6 shows the preparation of compounds represented by Structural Formula (I), wherein Z is represented by Structural Formulas (III) and wherein Ring A or Ring B in Z is substituted with R⁴⁰. L4 is a suitable leaving group such as halogen or trifluoromethylsulfonate.

In Figure 6, a palladium coupling reaction such as Stille coupling, Suzuki coupling, Heck reaction, or carboxylation using carbon monoxide may be carried out using a palladium catalyst such as tetrakis(triphenylphosphine)palladium, bis(triphenylphosphine)palladium chloride, and palladium acetate in a solvent such as tetrahydrofuran (THF), 1,4-dioxane, toluene, dimethylformamide (DMF), or dimethylsufoxide (DMSO) in the presence of additive (when necessary) such as triphenylphosphine, 1,1'-bis(diphenylphosphino)ferrocene, triethylamine, sodium bicarbonate, tetraethylammonium chloride, or lithium chloride at room temperature up to the reflux temperature for the solvent used for 5 minutes to 72 h.

Figure 9c shows three procedures for the preparation of compounds represented by Structural Formulas (I),(VII), (VIII) and (IX), wherein Z is represented by Structural Formula (III) and wherein Ring A or Ring B in Z is substituted with R^{40.} In Figure 9c, R⁴⁰ is represented by - (O)ᵤ-(CH₂)ₜ-C(O)-NR²¹R²², u is one, t is zero.

In Figure 9c a compound containing a phenol can be reacted with a carbonate equivalent, such as a carbamoyl chloride (method A), an isocyanate (method B) or an acylimidazole (method C), in the presence of a base such as sodium hydroxide, potassium carbonate or sodium carbonate in a solvent such as dimethylformamide or tetrahydrofuran, at a temperature from 0°C to reflux temperature for a period of about 5 minutes to about 72 hours.

Compounds represented by Structural Formula (I), wherein Z is represented by Structural Formulas (III) or (IV), X is -CO-NR_{c}- and R_{c} is -(CH₂)ₛ-COOR³⁰, -(CH₂)ₛ-C(O) -NR³¹R³² or -(CH₂)ₛ-NHC(O)-O-R³⁰, can be prepared by suitable modification of the scheme shown in Figure 1-6. One modification utilizes the starting material shown in Figure 1, wherein X is -CO-NH-. The amide is then alkylated with L³-(CH₂)₃-COOR³⁰, wherein L³ is a suitable leaving group, using the alkylation procedures described above. The remainder of the synthesis is as described in Figures 1-6.

Figure 11 shows the preparation of compounds of formula (VI-c). The Friedel-Crafts acylation can be carried out using an acid chloride in the presence of a Lewis acid, such as aluminum trichloride or titanium tetrachloride, in a solvent such as dichloromethane, dichloroethane, nitrobenzene or carbon disulfide. The acylation reaction can be run at a temperature of about room temperature up to the reflux temperature of the chosen solvent, and for a period of about 5 minutes to about 72 hours.

Figure 12 shows the preparation of compounds of formula (VI-e). In Step 1 of Figure 12, a chlorosulfonylation can be carried out using chlorosulfonic acid in a solvent, such as dichloromethane, or in the absence of a solvent at a temperature of about 0°C to about 60°C for a period of about 5 minutes to about 72 hours. In Step 2 of Figure 12, a coupling reaction can be carried out using an amine in the presence of a base, such as triethylamine, in a solvent such as dichloromethane, acetone, ethanol, THF or DMF. The reaction can be carried out at a temperature of about room temperature up to the reflux temperature of the selected solvent, and for a period of about 5 minutes to about 72 hours.

Although Figures 1- 6, 11 and 12 show the preparation of compounds in which Rings A and B are phenyl rings, analogous compounds with heteroaryl groups for Rings A and B can be prepared by using starting materials with heteroaryl groups in the corresponding positions. These starting materials can be prepared according to methods disclosed in JP 61/152673, U.S. Patent 5089496, WO 89/10369, WO 92/20681 and WO 93/02081.

The invention is illustrated by the following examples which are not intended to be limiting in any way.

### EXEMPLIFICATION

### Membrane Preparations for Chemokine Binding and Binding Assays

Membranes were prepared from THP-1 cells (ATCC #TIB202). Cells were harvested by centrifugation, washed twice with PBS (phosphate-buffered saline), and the cell pellets were frozen at -70 to -8.5°C. The frozen pellet was thawed in ice-cold lysis buffer consisting of 5 mM HEPES (N-2-hydroxyethylpiperazine-N'-2-ethane-sulfonic acid) pH 7.5, 2 mM EDTA (ethylenediaminetetraacetic acid), 5 µg/ml each aprotinin, leupeptin, and chymostatin (protease inhibitors), and 100 µg/ml PMSF (phenyl methane sulfonyl fluoride - also a protease inhibitor), at a concentration of 1 to 5 x 10⁷ cells/ml. This procedure results in cell lysis. The suspension was mixed well to resuspend all of the frozen cell pellet. Nuclei and cell debris were removed by centrifugation of 400 x g for 10 minutes at 4°C. The supernatant was transferred to a fresh tube and the membrane fragments were collected by centrifugation at 25,000 x g for 30 minutes at 4°C. The supernatant was aspirated and the pellet was resuspended in freezing buffer consisting of 10 mM HEPES pH 7.5, 300 mM sucrose, 1µg/ml each aprotinin, leupeptin, and chymostatin, and 10 µg/ml PMSF (approximately 0.1 ml per each 10⁸ cells). All clumps were resolved using a minihomogenizer, and the total protein concentration was determined using a protein assay kit (Bio-Rad, Hercules, CA, cat #500-0002). The membrane solution was then aliquoted and frozen at -70 to -85°C until needed. Binding Assays utilized the membranes described above. Membrane protein (2 to 20 µg total membrane protein) was incubated with 0.1 to 0.2 nM ¹²⁵I-labeled RANTES or MIP-1α with or without unlabeled competitor (RANTES or MIP-1α) or various concentrations of compounds. The binding reactions were performed in 60 to 100 µl of a binding buffer consisting of 10 mM HEPES pH 7.2, 1 mM CaCl₂, 5 mM MgCl₂, and 0.5% BSA (bovine serum albumin), for 60 min at room temperature. The binding reactions were terminated by harvesting the membranes by rapid filtration through glass fiber filters (GF/B or GF/C, Packard) which were presoaked in 0.3% polyethyleneimine. The filters were rinsed with approximately 600 µl of binding buffer containing 0.5 M NaCl, dried, and the amount of bound radioactivity was determined by scintillation counting in a Topcount beta-plate counter.

The activities of test compounds are reported in the Table below as IC₅₀ values or the inhibitor concentration required for 50% inhibition of specific binding in receptor binding assays using ¹²⁵I-RANTES or ¹²⁵MIP-1α as ligand and THP-1 cell membranes. Specific binding is defined as the total binding minus the non-specific binding; non-specific binding is the amount of cpm still detected in the presence of excess unlabeled Rantes or ¹²⁵MIP-1α.

**Table**

| BIOLOGICAL DATA | |
|---|---|
| Example | IC₅₀ (µM) |
| 1 | <1 |
| 51 | <1 |
| 52 | <1 |
| 53 | <1 |

### Reference Example 1-4-(4-Chlorophenyl)-1-[3-(6,11-dihydro-2-hydroxydibenz[b,e]oxepin-11-ylidene)propyl]piperidin-4-ol

### Step 1

11-(3-Bromopropylidene)-6,11-dihydro-2-hydroxydibenz[b,e]oxepine was prepared by following the procedure of reference example 4, step 1 and 2, but replacing 5,11-dihydro-7-methoxypyrido[2,3-c][1]benzoxepin-5-one with 6,11-dihydro-2-hydroxydibenz[b,e]oxepin-11-one. ¹H-NMR (CDCl₃) δ: 2.69(2H,q), 3.39 (2H,t), 5.20(2H,brs), 5.92(1H,t), 6.50-6.81(4H,m), 7.17-7.37(4H,m).

### Step 2

The titled compound was prepared by following the procedure of reference example 4, step 3, but replacing 5-(3-bromopropylidene)-10,11-dihydro-5H-dibenzo[a,d]cycloheptene with the product of step 1.
¹H-NMR (CDCl₃) δ: 1.60-1.75(3H,m), 1.95-2.10(2H,m), 2.35-2.80(8H,m), 5.10(2H,brs), 5.93(1H,t), 6.56(2H,brs), 6.71(1H,brs), 7.11-7.35(8H,m).
MS m/z: 462 (M+1)

### Reference Example 2-4-(4-Chlorophenyl)-1-[3-(6,11-dihydro-2-ethoxydibenz[b,e]oxepin-11-ylidene)propyl]piperidin-4-ol

To a solution of 4-(4-chlorophenyl)-1-[3-(6,11-dihydro-2-hydroxydibenz[b,e]oxepin-11-ylidene)propyl]piperidin-4-ol (reference example 1) (200mg) in DMF (5ml) were added sodium hydride (60% in oil, 25mg), ethyl iodide (0.052ml) and the mixture was stirred at room temperature for 1 hour. Water and ethyl acetate were added to the reaction mixture, the organic layer was separated and washed with saturated aqueous sodium chloride, and dried with magnesium sulfate. The solvent was distilled off under reduced pressure. The residue was purified by silica gel chromatography eluting with ethyl acetate-hexane (1:1) to give the titled compound (170mg).
¹H-NMR (CDCl₃) δ: 1.37(3H,t), 1.60-1.65(2H,m), 1.95-2.08(3H,m), 2.28-75(8H,m), 3.96(2H,q), 5.15(2H,brs), 6.02(1H,t), 6.68(2H,brs), 6.82(1H,brs), 7.19-7.42(8H,m).
MS m/z: 490(M+1)

### Reference Example 3-4-(4-Chlorophenyl)-1-[3-(5,11-dihydro-7-hydroxy[1]benzoxepino[2,3-b]pyridin-5-ylidene)propyl]piperidin-4-ol

### Step 1

To a solution of the product of reference example 4; step 1 (4.3g) in dichloroethane (100ml) was added boron tribromide-methyl sulfide complex (19.3g) and the mixture was heated to reflux for 3 hour. Water and ethyl acetate were added to the reaction mixture and neutralized with dilute NaOH solution. The organic layer was separated and washed with saturated aqueous sodium chloride, and dried over magnesium sulfate. The solvent was distilled off under reduced pressure. The residue was purified by silica gel chromatography eluting with ethyl acetate-hexane (1:2) to give 5-(3-bromopropylidene)-5,11-dihydro-7-hydroxy [1]benzoxepino[2,3-b]pyridine (3.2g).
¹H-NMR (CDCl₃) δ: 2.72(2H,q), 3.45(2H,t), 5.28(2H,brs), 6.03(1H,t), 6.66-6.80(3H,m), 7.26(1H,dd), 7.58(1H,dd), 8.51(1H,dd).

### Step 2

The titled compound was prepared by following the procedure of reference example 4, step 3, but replacing 5-(3-bromopropylidene)-5,11-dihydro-7-methoxy [1]benzoxepino[2,3-b]pyridine with the product of step 1.
¹H-NMR (DMSO-d₆) δ: 1.46-1.51(2H,m), 1.74-1.85(2H,m), 2.29-2.51(8H,m), 5.15(2H,brs), 6.07(1H, t), 6.61-6.70(3H,m), 7.33-7.48(5H,m), 7.73(1H,dd), 8.47(1H,dd), 9.06(1H,s).
MS m/z: 463(M+1)

### Reference Example 4 -4-(4-Chlorophenyl)-1-[3-(5,11-dihydro-7-methoxy[1]benzoxepino[2,3-b]pyridin-5-ylidene)propyl]piperidin-4-ol

### Step 1

To a solution of 5,11-dihydro-7-methoxy [1]benzoxepino[2,3-b]pyridin-5-one (5.0g) in THF (50ml) was added 1.1M cyclopropylmagnesium bromide THF solution (25ml) at 0°C. The reaction mixture was warmed to room temperature, and stirred for 30 minutes. Aqueous ammonium chloride and ethyl acetate were added to the reaction mixture, the organic layer was separated and washed with saturated aqueous sodium chloride, and dried with magnesium sulfate. The solvent was distilled off under reduced pressure. The residue was filtered and washed with ethyl acetate-hexane (1:2) to give 5-cyclopropyl-5,11-dihydro-7-methoxy[1]benzoxepino[2,3-b]pyridin-5-ol (5.0g).

### Step 2

To a solution of the product of step 1 (4.3g) in acetic acid (30ml) was added 48% aqueous HBr (25ml) at 10°C. The reaction mixture was warmed to room temperature, and stirred for 12 hours. Water and ethyl acetate were added to the reaction mixture and neutralized with dilute NaOH solution. The organic layer was separated and washed with saturated aqueous sodium chloride, and dried over magnesium sulfate. The solvent was distilled off under reduced pressure. The residue was purified by silica gel chromatography eluting with ethyl acetate-hexane (1:4) to give 5-(3-bromopropylidene)-5,11-dihydro-7-methoxy [1]benzoxepino[2,3-b]pyridine (5.6g).
¹H-NMR (CDCl₃) δ: 2.74(2H,q), 3.46(2H,t), 3.78(3H,s), 5.25 (2H,brs), 6.07(1H,t), 6.72-6.82(3H,m), 7.21-7.42(5H,m), 7.56(1H,dd), 8.45(1H,dd).

### Step 3

To a solution the product of step 2 (1.1g) in DMF (15ml) were added 4-(4-chlorophenyl)-4-hydroxypiperidine (0.81g) and potassium carbonate (0.53g) and the mixture was stirred at room temperature for 3 hours. Water and ethyl acetate were added to the reaction mixture, the organic layer was separated and washed with saturated aqueous sodium chloride, and dried with magnesium sulfate. The solvent was distilled off under reduced pressure. The residue was purified by silica gel chromatography eluting with methylene chloride-methanol (10:1) to give the titled compound as major regioisomer (0.86g) and minor one (0.05g).
Major isomer
¹H-NMR (CDCl₃) δ: 1.64-1.69 (2H,m) , 1.91-2.08 (3H ), 2.34-2.69(8H,m), 3.77(3H,s), 5.25(2H,brs), 6.07(1H,t), 6.72-6.82(3H,m), 7.21-7.42(5H,m), 7.56(1H,dd), 8.45(1H,dd).
MS m/z: 477(M+1)
Minor isomer
¹H-NMR (CDCl₃) δ: 1.65-1.79 (3H,m) , 2.01-2.13 (2H,m) , 2.35-2.76(8H,m), 3.76(3H,s), 5.22(2H,brs), 5.95(1H,t), 6.72-6.80(2H,m), 7.06(1H,d), 7.16(1H,dd), 7.28(2H,d), 7.42(2H,d), 7.66(1H,dd), 8.39(1H,dd).
MS m/z: 477(M+1)

### Reference Example 5 -4-(4-Chlorophenyl)-1-[3-(5,11-dihydro-7-ethoxy [1]benzoxepino[2,3-b]pyridin-5-ylidene)propyl]piperidin-4-ol

The titled compound was prepared by following the procedure of reference example 2, but replacing 4-(4-chlorophenyl)-1-[3-(6,11-dihydro-2-hydroxydibenz[b,e]oxepin-11-ylidene)propyl]piperidin-4-ol with 4-(4-chlorophenyl)-1-[3-(5,11-dihydro-7-hydroxy[1]benzoxepino[2,3-b]pyridin-5-ylidene)propyl]piperidin-4-ol (reference example 3).
¹H-NMR (CDCl₃) δ: 1.38(3H,t), 1.67-1.72(3H,m), 2.05-2.16(2H,m), 2.40-2.80(8H,m), 3.99(2H,q), 5.26(2H,brs), 6.05(1H,t), 6.71-6.82(3H,m), 7.23-7.43(5H,m), 7.57(1H,dd), 8.47(1H,dd).
MS m/z: 491(M+1)

### Reference Example 6 -4-(4-Chlorophenyl)-1-[3-(5,11-dihydro-7-ethoxycarbonylmethyloxy[1]benzoxepino[2,3-b]pyridin-5-ylidene)propyl]piperidin-4-ol

The titled compound was prepared by following the procedure of reference example 5, but replacing ethyl iodide with ethyl bromoacetate.
¹H=NMR (CDCl₃) δ: 1.28(3H,t), 1.63-1.68(2H,m), 1.97-2.02(3H,m), 2.33-2.68(8H,m), 4.24(2H,q), 4.55(2H,s), 5.26(2H,brs), 6.06(1H,t), 6.73-6.88(3H,m), 7.21-7.42(5H,m), 7.55(1H,dd), 8.44(1H,dd).
MS m/z: 549(M+1)

### Reference Example 7 - 4-(4-Chlorophenyl)-1-[3-(5,11-dihydro [1]benzoxepino[2,3-b]pyridin-5-ylidene)propyl]piperidin-4-ol

### Step 1

5-(3-Bromopropylidene)-5,11-dihydro [1]benzoxepino[2,3-b]pyridine was prepared by following the procedure of reference example 4, step 1 and 2, but replacing 5,11-dihydro-7-methoxy[1]benzoxepino[2,3-b]pyridin-5-one with 5,11-dihydro[1]benzoxepino[2,3-b]pyridin-5-one.
¹H-NMR (CDCl₃) δ: 2.71(2H,q), 3.-46(2H,t), 5.33(2H,brs), 6.04(1H,t), 7.01-7.17(3H,m), 7.29(1H,dd), 7.56(1H,dd), 8.53(1H,dd).

### Step 2

The titled compound was prepared by following the procedure of reference example 4, step 3, but replacing 5-(3-bromopropylidene)-5,11-dihydro-7-methoxy [1]benzoxepino[2,3-b]pyridine with the product of step 1.
¹H-NMR (CDCl₃) δ: 1.66-1.71(2H,m), 2.00-2.20(3H,m), 2.36-2.69(8H,m), 5.34(2H,brs), 6.10(1H,t), 6.83-6.96(3H,m), 7.17-7.44(6H,m), 7.60(1H,dd), 8.46(1H,dd).
MS m/z: 447(M+1)

### Reference Example 8 - 1-[3-(7-Carboxy-5,11-dihydro[1]benzoxepino[2,3-b]pyridin-5-ylidene)propyl]-4-(4-chlorophenyl)piperidin-4-ol

A mixture of the product of reference example 10 (500 mg), potassium acetate (330 mg), palladium(II) diacetate (10 mg), 1,1'-bis(diphenylphosphino)ferrocene (93 mg), in dimethylsulfoxide (10 ml) was purged with carbon monoxide for 5 minutes and stirred under a carbon monoxide balloon at 60°C for 3 hours. Water was added to the reaction mixture, the precipitation was filtered. The solid were dissolved with ethyl acetate and dilute sodium hydroxide solution. The aqueous layer was separated and neutralized with dilute hydrochloric acid. The precipitation was filtered to give the titled compound (250 mg).
1H-NMR (DMSO-d₆) δ: 1.45-1.55(2H,m), 1.75-1.85(2H,m), 2.36-2.62(8H,m), 5.42(2H,brs), 6.21(1H,t), 6.90(1H,d), 7.40-7.52(5H,m), 7.75(1H,dd), 7.83(1H,dd), 7.95(1H,d), 8.56(1H,dd).
MS m/z: 491(M+1)

### Reference Example 9 -1-(3-(7-Carboxymethyloxy-5,11-dihydro[1]benzoxepino[2,3-b]pyridin-5-ylidene)propyl]-4-(4-chlorophenyl)piperidin-4-ol

To a solution of product of reference example 6 (3.0 g) in methanol (50 ml) was added 1N sodium hydroxide solution (8 ml) and the mixture stirred at room temperature for 1 hour. The reaction mixture was distilled off under reduced pressure. The residue was dissolved with water and neutralized with 1N hydrochloric acid. The precipitation was filtered and washed with water to give the titled compound (2.6 g).
¹H-NMR (DMSO-d₆) δ: 1.48-1.53(2H,m), 1.76-1.88(2H,m), 2.32-2.60(8H,m), 4.60(2H,s), 5.18(2H,brs), 6.16(1H,t), 6.72-6.84(3H,m), 7.34-7.48(5H,m), 7.73(1H,dd), 8.50(1H,dd).
MS m/z: 521(M+1)

### Reference Example 10 - 4-(4-Chlorophenyl)-1-[3-(5,11-dihydro-7-trifluoromethanesulfonyloxy[1]benzoxepino[2,3-b]pyridin-5-ylidene)propyl]piperidin-4-ol

To a solution of product of reference example 3 (1.0 g) in pyridine (10 ml) was added trifluoromethanesulfonic acid anhydride (0.55 ml) at 0°C, and the mixture was stirred at room temperature for 1 hour. Water and diethyl ether were added to the reaction mixture, the organic layer was separated and washed with saturated aqueous sodium chloride, and dried with magnesium sulfate. The solvent' was distilled off under reduced pressure, and the residue was purified by silica gel chromatography eluting with ethyl acetate-methanol (10:1) to give the titled compound (1.1 g).
1H-NMR (CDCl₃) δ: 1.56(1H,brs), 1.66-1.71(2H,m), 1.97-2.09(2H,m), 2.35-2.69(8H,m), 5.35(2H,brs) 6.15(1H,t), 6.88(1H,d), 7.05(1H,dd), 7.21-7.44(6H,m), 7.60(1H,dd), 8.54(1H,dd).
MS m/z: 595(M+1)

### Example 1 -5-(4-Chlorophenyl)-1-[3-(5, 11-dihydro-7-methoxy[1]benzoxepino[2,3-b]pyridin-5-ylidene)propyl]-4,6-dioxazacane.

5-(4-Chlorophenyl)-1-[3-(5,11-dihydro-7-methoxy[1]benzoxepino[2,3-b]pyridin-5-ylidene)propyl]-4,6-diazacyclooctylamine

### Step1

5-(3-(N,N'-Bis (2-hydroxyethyl)amino)propylidene) - 5,11-dihydro-7-methoxy[1]benzoxepino[2,3-b]pyridine was prepared by following the procedure of reference example 4, step 3, but replacing 4-(4-chlorophenyl)-4-hydroxypiperidine with diethanolamine.
¹H-NMR (CD₃OD) δ: 2.46(2H,m), 2.84(4H,t), 2.98(2H,m), 3.67(4H,t), 3.75(3H,s), 5.20(2H,brs), 6.16(1H,t), 6.68-6.80(2H,m), 6.87(1H,d), 7.46(1H,dd), 7.81(1H,dd), 8.45(1H,dd).

### Step2

To a mixture of product of step1 (78mg) and 4-chlorobenzaldehyde dimethyl acetal (0.1ml) in 1,2-dichloroethane (60ml) was added p-toluenesulfonic acid monohydrate (5mg) at room temperature, and the mixture was stirred at reflux for 12 hours. Dichloromethane and saturated aqueous sodium bicarbonate was added to the cooled reaction mixture, the organic layer was separated and washed with saturated aqueous sodium chloride, and dried with magnesium sulfate. The solvent was distilled off under reduced pressure, and the residue was purified by silica gel chromatography eluting with dichloromethane-methanol (20:1) to give the titled compound (40mg).
H-NMR (CDCl₃) δ: 2.35(2H,m), 2.64-2.94(6H, m), 3.52-3.68(2H, m), 3.78(3H,s), 3.72-3.90(2H,m), 5.27(2H,brs), 5.66(1H,s), 6.08(1H,t), 6.68-6.88(3H,m), 7.18-7.46(5H,m), 7.58(1H,dd), 8.50(1H,dd).

### Example 4

### 3-(4-Chlorophenyl)-8-[3-(5,11-dihydro-7-hydroxy[1]benzoxepino[2,3-b]pyridin-5-ylidene)propyl]-8-azabicyclo[3.2.1]octan-3-ol

The titled compound was prepared by following the procedure of reference example 3, step 2, but replacing 4-(4-chlorophenyl)-4-hydroxypiperidine with 3-(4-chlorophenyl)-8-azabicyclo[3.2.1]octan-3-ol
¹H-NMR(CDCl₃) δ:1.65-2.10(4H,m), 2.1-2.7(8H,m), 3.32(2H,bs), 3.78(3H,s), 5.24(2H,bs), 6.10(1H,dd), 6.70-6.90(3H,m), 7.15-7.31(3H,m), 7.45(bd,2H), 7.64(dd,1H) 8.46(dd,1H)
MS m/z: 503(M+1)

### Reference Example 11 -4-(4-Chlorophenyl)-1-[3-(7-ethoxycarbonylamino-5,11-dihydro[1]benzoxepino[2,3-b]pyridin-5-ylidine)propyl]piperidin-4-ol

A mixture of product of reference example 8 (490mg) and diphenylphosphonic azide (0.28ml) was stirred at 110°C for 30minutes. After the mixture was cooled, and triethylamine (0.14ml) and ethanol (5ml) were added, and the mixture was heated to reflux for 8 hours. The reaction mixture was diluted with ethyl acetate and filterd through Celite. The filtrate was washed with saturated aqueous sodium bicarbonate, and dried over magnesium sulfate. The solvent was removed under reduced pressure and the residue was purified by silica gel column chromatography (chloroform : methanol = 10 : 1) to give the titled compound (210mg).
¹H-NMR (CDCl₃) δ: 1.31(3H,t), 1.65-1.70(2H,m), 2.01-2.09(2H,m), 2.36-2.70(8H,m), 4.21(2H,q), 5.30(2H,brs), 6.13(1H,t), 6.46(1H,brs), 6.80(1H,d), 7.02(1H,dd), 7.28-7.50(6H,m), 7.57(1H,dd), 8.50(1H,dd).
MS m/z: 534(M+H)

### Example 5

### 4-(4-Chlorophenyl)-1-[3-(5,11-dihydro-7-(1-cyclohexyloxycarbonyloxy)ethyloxycarbonyl[1]benzoxepino[2, 3-b]pyridin-5-ylidene)propyl]piperidin-4-ol dihydrochloride

To a solution of product of reference example 8 (1.1g) in dimethylformamide (15ml) were added sodium iodide(0.17g), potassium carbonate (0.38 g) and cyclohexyl 1-chloroethyl carbonate (J. Antibiotics, 1987, 40, 81.) (0.57g) at room temperature. The mixture was stirred at 70°C for 1 hour. Water and ethyl acetate were added to the reaction mixture, the organic layer was separated and washed with saturated aqueous sodium chloride, and dried with magnesium sulfate. The solvent was distilled off under reduced pressure and the residue was purified by silica gel column chromatography (ethyl acetate : methanol = 100 : 3). The obtained oil was dissolved with ethyl acetate, and 4 N hydrochloric acid ethyl acetate solution (0.8ml) was added. The precipitation was filtered to give the titled compound (0.96g).
¹H-NMR (DMSO-d₆) δ: 1.22-1.47(6H,m), 1.58(3H,d), 1.63-1.81(6H,m), 2.38-3.30(10H,m), 4.07-4.59(1H,m), 5.80(2H,brs), 6.28(1H,t), 6.87(1H, q), 6.97(1H,d), 7.40-7.49(4H,m), 7.69(1H,dd), 7.79(1H,dd), 7.96(1H,d), 8.03(1H,dd), 8.65(1H,dd), 11.07(1H,brs).
MS m/z: 661[(M-2HCl)+1]

### Example 12

To a stirred solution of phenol containing the product of reference Example 3 (1.0 mmol) and K₂CO₃ (1.5 mmol) in THF (10 mL) at RT was added *N, N*-dimethylcarbamoylchloride (1.2 mmol). The reaction was stirred at reflux for 24 hrs. Excess solvent was removed and pure compound was isolated via silica gel chromatography eluting with 5% MeOH/CH₂Cl₂- MS
m/z: (M+ 535)

### Example 13

To a stirred solution of phenol containing the product of reference Example 3 (1.0 mmol) and K₂CO₃ (1.5 mmol) in THF (10 mL) at RT was added morpholinocarbamoylchloride (1.2 mmol). The reaction was stirred at reflux for 24 hrs. Excess solvent was removed and pure compound was isolated via silica gel chromatography eluting with 5% MeOH/CH₂Cl₂. MS
m/z: (M+ 577)

### Example 14

To a stirred solution of phenol containing the product of reference Example 3 (1.0 mmol) in DMF at RT was added NaH (1.5 mmol) followed by the addition of *N*-isopropylisocyanate (1.5 mmol). The reaction was heated to 60°C for 6 hrs. The reaction was quenched with 1.5 equivalents of H₂O and excess DMF was removed under reduced pressure. Residue was charged on a silica gel column and eluted off with 5% MeOH/CH₂Cl₂. MS m/z: (M+548)

### Example 15

To a stirred solution of phenol containing the product of reference Example 3 (1.0 mmol) and K₂CO₃ (1.5 mmol) in THF (10 mL) at RT was added *N*-methyl-*N*-phenylcarbamoylchloride (1.2 mmol). The reaction was stirred at reflux for 24 hrs. Excess solvent was removed and pure compound was isolated via silica gel chromatography eluting with 5% MeOH/CH₂Cl₂. MS m/z: (M+ 597)

### Example 16

To a stirred solution of phenol containing the product of reference Example 3 (1.0 mmol) in DMF at RT was added NaH (1.5 mmol) followed by the addition of *N*-phenylisocyanate(1.5 mmol). The reaction was heated to 60°C for 6 hrs. The reaction was quenched with 1.5 equivalents of H₂O and excess DMF was removed under reduced pressure. Residue was charged on a silica gel column and eluted off with 5% MeOH/CH₂Cl₂. MS m/z: (M+ 583)

### Example 17

To a stirred solution of phenol containing the product of reference Example 3 (1.0 mmol) in DMF at RT was added NaH (1.5 mmol) followed by the addition of *N*-(3-pyridyl)isoeyanate(1.5 mmol). The reaction was heated to 60°C for 6 hrs. The reaction was quenched with 1.5 equivalents of H₂O and excess DMF was removed under reduced pressure. Residue was charged on a silica gel column and eluted off with 5% MeOH/CH₂Cl₂. MS m/z: (M+ 584)

### Example 18

To a stirred solution of phenol containing the product of reference Example 3 (1.0 mmol) and K₂CO₃ (1.5 mmol) in THF (10 mL) at RT was added pyrolidinylcarbamoylchloride (1.2 mmol). The reaction was stirred at reflux for 24 hrs. Excess solvent was removed and pure compound was isolated via silica gel chromatography eluting with 5% MeOH/CH₂Cl₂.
MS m/z: (M+ 560)

### Reference Example 12

The compound was prepared by following the procedure for reference example 4, step 3, but replacing 4-(4-chlorophenyl)-4-hydroxypiperidine with 4-(4-chlorophenyl)-4-cyanopiperidine. MS m/z: (M+ 486).

### Example 13

To a cold (0°C) stirred solution or reference Example 12 (0.50 g, 0.104 mmol) in anhydrous THF (5 mL) was added lithium aluminum hydride (8 mg, 0.21 mmol). The reaction was stirred at RT for 2 hrs. The reaction was then quenched by the careful addition of H₂O (0.21 mL), 15% aqueous KOH (0.21 mL), then H₂O (0.21 mL). The organic layer was separated and dried over Na₂SO₄. The compound was purified via silica gel flash chromatography eluting with 10% methanol/methylene chloride. MS m/z: (M+ 490).

### Example 14

### Step 1

A mixture of epichlorohydrin (5.92 g, 64 mmol) and benzhydrylamine (11.7 g, 64 mmol) in MeOH (120 mL) was stirred under the protection of argon at room temperature for 48 hours. The mixture was then stirred at 50°C for 72 hours. The reaction mixture was then stirred at room temperature for 72 hours. The reaction mixture was concentrated in vacuo and partitioned between EtOAc and H₂O. The aqueous layer was extracted with EtOAc (200 mL x 3), dried over MgSO₄ and concentrated in vacuo. Chromatographic purification on silica gel (CH₂Cl₂/MeOH= 95/5) provided 10.0 g (65%) of 1-benzhydril-3-hydroxyazetidine. m/z 240 (m+1).

### Step 2

A mixture 1-benzhydril-3-hydroxyazetidine (2.6 g, 11 mmol) and palladium hydroxide on active carbon (0.26 g, w/w 20%) in EtOH (40 mL) was shaken in hydrogenation parr under 60 psi for 24 hours. The reaction mixture was filtered through celite and concentrated under vacuum.
Concentration in vacuo provided 0.75 (95%) 3-hydroxyazetidine. ¹H NMR (250 MHz, CD30D) 3.81-3.92 (2H, m), 4.14-4.25 (2H, m), 4.61-4.69 (1H, m).

### Step 3

The compound 1-[3-(5,11-dihydro-7-(methoxy[1]benzoxepino[2,3-b]pyridin-5-ylidene)propyl]azetidin-3-ol was prepared by following the procedure for reference example 4, step 3, but replacing 4-(4-chlorophenyl)-4-hydroxypiperidine with 3-hydroxyazetidine. m/z 339 (m+1).

### Step 4

To a mixture of morpholine N-oxide (0.028 g, 0.244 mmol), crushed molecular sieves (0.066 g) and Pr₄N⁺RO₄ (0.01 g, 0.024 mmol) in CH₂Cl₂ was added the 1-[3-(5,11-dihydro-7-(methoxy[l]benzoxepino[2,3-b]pyridin-5-ylidene)propyl]azetidin-3-ol (0.055 g, 0.16 mmol) under the protection of argon. The mixture was stirring over night at room temperature. The reaction mixture was filtered off through celite and concentrated under vacuum. Chromatographic purification on silica gel (CH₂Cl₂/MeOH = 95/5 to 9/1) provided 0.033 g 1-[3-(5,11-dihydro-7-(methoxy[1]benzoxepino[2,3-b]pyridin-5-ylidene)propyl]azetidin-3-one ( 60%) of the desired product. m/z 337 (m+1)

### Step 5

To a solution of 1-[3-(5,11-dihydro-7-(methoxy[1]benzoxepino[2,3-b]pyridin-5-ylidene)propyl]azetidin-3-one (0.06 g, 0.18 mmol) in THF (8 mL) was added dropwise a solution of 4-chlorophenyl magnesium bromide in diethyl ether (1.0 M, 0.27 mL) under the the protection of argon at 0°C. The reaction was stirred at room temperature for 1.5 hours and quenched by the addition of saturated aqueous NH₄OH (4 mL). The aqueous layer was extracted with EtOAc (10 mL x 2), dried over MgS04 and concentrated in vacuo. Chromatographic purification on silica gel (CH₂Cl₂/MeOH = 95/5) provided 0.048 g 3-(4-chlorophenyl)-1-[3-(5,11-dihydro-7-(methoxy[1]benzoxepino[2,3-b]pyridin-5-ylidene)propyl]azetidine (51%) m/z 449 (m+1)

### Example 15

### Step 1

### tert-Butyl 3-(4-chlorobenzoyl)-1-(2-aminoethyl) carbamate: Fig. 9b

*tert*-Butyl *N*-(2-aminoethyl) carbamate (1, 0.50 g g, 3.12 mmol) was added to the mixture of 4-chlorobenzoic acid chloride (0.547 g, 3.12 mmol) and Et₃N (1.74 mL, 12.5 mmol) in CH₂Cl₂ (20 mL) under the protection of argon. Stirring at room temperature for 2 hours. The reaction mixture was diluted with H₂O (25 mL), extracted with CH₂Cl₂ (50 mL x 2), dried over MgSO₄ and concentrated in vacuo. Chromatographic purification on silica gel (CH₂Cl₂/MeOH = 95/5) to provide 0.86 g (2, 93%) of the desired product *tert*-Butyl 3-(4-chlorobenzoyl)-1-(2-aminoethyl) carbamate. MS m/z: (M+ 299) .

### Step 2

1-(4-chlorobenzoyl)-1,2-ethylenediamine: Fig. 9b Trifluoroacetic acid (7.5 mL) was added to the solution of tert-Butyl 3-(4-chlorobenzoyl)-1-(2-aminoethyl)carbamate (2, 0.86 g, 2.89 mmol) in CH₂Cl₂ (35 mL) at 0°C. Stirring at room temperature for 30 minutes. Concentration in vacuo provided 0.88 g (95%) of the desired product 1-(4-chlorobenzoyl)-1,2-ethylenediamine (3). MS m/z: (M+ 199).

### Step 3

The compound was prepared by following the procedure for reference example 4, step 3, but replacing 4-(4-chlorophenyl)-4-hydroxypiperidine with 1-(4-chlorobenzoyl)-1,3-propylenediamine. MS m/z: (M+ 465).

### Example 1b

### Step 1

### 2-(4-Chlorophenyl)-1-bromoethylene: Fig. 8c

To a solution of AlCl₃ (1.96 g, 14.7 mmol) in anhydrous CH₂Cl₂ (50 mL), Borane-tert-butyl amine complex (2.57 g, 29.6 mmol) was added at 0°C under argon protection, stirred for 10 minutes and clear solution was formed. 4-Chlorophenacyl bromide (1, 1.11 g, 4.91 mmol) in CH₂Cl₂ (5 mL) was added to the resulted mixture at 0°C. The reaction was stirred for 1.5 hours and then quenched by the addition of 0.1 N HCl (25 mL). The mixture was extracted with EtOAc (80 mL x 3), dried over MgS04 and concentrated in vacuo. Chromatographic purification on silica gel (Hexane/EtOAc = 9:1) provided 0.85 g (84%) of 2-(4-chlorophenyl)-1-bromoethylene (2). MS m/z: (M+ 219).

### Step 2

### 2-(4-chlorophenyl)-1-(N-methyl)ethylamine: Fig. 8c

A mixture of 2-(4-chlorophenyl)-1-bromoethylene (2, 1.02 g, 4.62 mmol), EtOH (3 mL) and H₂NMe in H₂O (6 mL, 40% w/w) was heated at 135 0°C over night. The mixture was cooled down to room temperature. The mixture was extracted with Et₂O (5mL x 2), dried over MgSO₄ and concentrated in vacuo. Chromatographic purification on silica gel (CH₂Cl₂/MeOH/NH₄OH = 9/1/0.1) provided 0.61 g 2-(4-chlorophenyl)-1-(*N*-methyl)ethylamine (3, 79%). MS m/z: (M+ 170).

### Step 3

The compound was prepared by following the procedure for reference example 4, step 3, but replacing 4-(4-chlorophenyl)-4-hydroxypiperidine with 2-(4-chlorophenyl)-1-(*N-*methyl)ethylamine. MS m/z: (M+ 451).

### Example 17

### Step 1

3-(4-chlorophenyl)-1-*N*-methylaminopropane: Fig. 8e A mixture of 3-(4-chlorophenyl)-1-bromoropane (1, 0.70 g, 3.73 mmol), EtOH (3 mL) and H₂NMe in H₂O (6 mL, 40% w/w) was heated at 135 0°C overnight. The mixture was then cooled down to room temperature. The mixture was extracted with Et₂O (5 mL x 2), dried over MgSO₄ and concentrated in vacuo. Chromatographic purification on silica gel (CH₂Cl₂/MeOH/NH₄OH = 9/1/0.1) provided 0.5 g (76%) of 3-(4-chlorophenyl)-1-*N*-methylaminopropane (2). MS m/z: (M+ 189).

### Step 2

The compound was prepared by following the procedure for reference example 4, step 3, but replacing 4-(4-chlorophenyl)-4-hydroxypiperidine with 3-(4-chlorophenyl)-1-*N-*methylaminopropane. MS m/z: (M+ 450).

### Example 18

### Step 1

3-(4-chlorophenyl)-3-chloro-1-hydroxypropane: Fig. 8d To 3,4'-Dichloropropylphenone (0.52 g, 2.53 mmol) in anhydrous MeOH (10 mL) at 0°C under the protection of argon, NaBH₄ (0.23 g, 3.03 mmol) was added to the solution by several portions. The reaction was stirred under the same condition for 15 minutes. The mixture was warmed up to room temperature, stirred an additional 30 minutes, then concentration in vacuo. The residue was partitioned between EtOAc and H₂O. The aqueous layer was re-extracted with EtOAc (30 mL x 2), dried over MgSO₄ and concentrated in vacuo. Chromatographic purification on silica gel (Hexane/EtOAc = (1/1) provided 0.52 g (99%) of 3-(4-chlorophenyl)-3-chloro-1-hydroxypropane. MS m/z: (M+205).

### Step 2

The compound was prepared by following the procedure for reference example 4, step 3, but replacing 4-(4-chlorophenyl)-4-hydroxypiperidine with 3-(4-chlorophenyl)-3-chloro-1-hydroxypropane. MS m/z: (M+ 481).

### Example 19

### Step 1

### 3-(4-chlorophenyl)-3-hydroxy-3-methyl-1-chloropropane: Fig. 9a

To 3,4'-Dichloropropylphenone (1, 1.10 g, 5.40 mmol) in anhydrous THF at 0°C under the protection of argon, was added MeMgBr (2.50 mL, 7.35 mmol) dropwise at 0°C. The reaction was stirred at room temperature for an additional hour. The reaction was quenched by adding saturated aqueous NH₄Cl. The reaction was then extracted with Et₂O (60 mL x 2), dried over MgSO₄ and concentrated in vacuo. Chromatographic purification on silica gel (Hexane/EtOAc = 10/1) provided 1.0 g (85%) of 3-(4-chlorophenyl)-3-hydroxy-3-methyl-1-bromoropane (2). MS m/z: (M+ 219).

### Step 2

### 3-(4-chlorophenyl)-3-hydroxyl-3-methyl-1-N-methylaminopropane: Fig. 9a

A mixture of 3,3,3-(4-Chlorophenyl)-hydroxylmethyl-1-bromoropane (2, 1.04 g, 4.74 mmol), EtOH (5 mL) and H₂NMe in H₂O (10 mL, 40% w/w) was heated at 135 0°C for 3 hours. The mixture was cooled down to room temperature. The mixture was extracted with Et₂O (5mL x 2), dried over MgSO₄ and concentrated in vauco. Chromatographic purification on silica gel (CH₂Cl₂/MeOH/NH₂OH = 9/1/0.1) provided 1.01 g 3-(4-chlorophenyl)-3-hydroxyl-3-methyl-1-*N-*methylaminopropane (3, 99%). MS m/z: (M+ 214).

### Step 3

The compound was prepared by following the procedure for reference example 4, step 3, but replacing 4-(4-chlorophenyl)-4-hydroxypiperidine with 3-(4-chlorophenyl)-3-hydroxyl-3-methyl-1-*N*-methylaminopropane. MS m/z: (M+ 480).

### Example 28

### 4-(4-Chlorophenyl)-1-[3-(5,11-dihydro-7-sulfamoyl[1]benzoxepino[2,3-b]pyridin-5-ylidene)propyl]piperidin-4-ol

### Step 1

To the product of reference example 7, step 1 (5.4g) was added chlorosulfonic acid (50ml) and the mixture stirred at 0°C for 1 hour. The reaction mixture was poured to ice, and ethyl acetate was added to the mixture, the organic layer was separated and washed with saturated aqueous sodium chloride, and dried with magnesium sulfate. The solvent was distilled off under reduced pressure. To the residue were added THF (250ml) and ammonium hydroxide (30ml) and the mixture stirred at room temperature for 10 minutes. Ethyl acetate and water were added to the mixture, the organic layer was separated and washed with saturated aqueous sodium chloride, and dried with magnesium sulfate. The solvent was distilled off under reduced pressure. The residue was purified by silica gel chromatography eluting with ethyl acetate-hexane (1:1) to give 5-(3-bromopropylidene)-5,11-dihydro-7-sulfamoyl[1]benzoxepino[2,3-b]pyridine(5.0g).
¹H-NMR (CDCl₃) δ: 2.70-2.75 (2H, m), 3.48 (2H, t), 5.39-5.49(4H, m), 6.16(1H, t), 6.88(1H, d), 7.25-7.34(2H, m), 7.53(1H, dd), 7.68(1H, dd), 7.93(1H, d), 8.53(1H, dd).

### Step 2

The titled compound was prepared by following the procedure of reference example 3, step 2,but replacing the product of reference example 3, step 1 with the product of step 1.
¹H-NMR (DMSO-d6) δ: 1.65-1.70(3H, m), 1.98-2.07(2H, m), 2.35-2.64(8H, m), 4.98(2H, brs), 5.39(2H, brs), 6.22(1H, t), 6.92(1H, d) 7.26-7.43(5H, m), 7.55-7.69(2H, m), 7.91(1H, d), 8.53(1H, dd).
MS m/z: 526(M+1)

### Example 29

### 4-(4-Chlorophenyl)-1-[3-(5,11-dihydro-7-(N-methylsulfamoyl)[1]benzoxepino[2,3-b]pyridin-5-ylidene)propyl]piperidin-4-ol

The titled compound was prepared by following the procedure of Example 28, but replacing ammonium hydroxide with methylamine.
¹H-NMR (CDCI₃) δ: 1.57-1.70(3H, m), 1.93-2.08(2H, m), 2.34-2.73(11H, m), 4.33(1H, q), 5.36(2H, brs), 6.21(1H, t), 6.91(1H, d), 7.29-7.45(6H, m), 7.58-7.65(2H, m), 7.83(1H, dd), 8.53(1H, dd).
MS m/z: 540 (M+1)

### Example 30

### 4-(4-Chlorophenyl)-1-[3-(5,11-dihydro-7-(N,N-dimethylsulfamoyl)[1]benzoxepino[2,3-b]pyridin-5-ylidene)propyl]piperidin-4-ol

The titled compound was prepared by following the procedure of Example 28, but replacing ammonium hydroxide with dimethylamine.
¹H-NMR (CDCI₃) δ: 1.55-1.75(3H, m), 1.96-2.07(2H, m), 2.35-2.67(8H, m), 2.71(6H, s), 5.51(2H, brs), 6.19(1H, t), 6.92(1H, d), 7.29-7.73(8H, m), 8.55(1H, dd).
MS m/z: 554(M+1)

### Example 31

### 4-(4-Chlorophenyl)-1-[3-(5,11-dihydro-7-(N-methoxycarbonylmethylsulfamoyl) [1]benzoxepino[2,3-b]pyridin-5-ylidene)propyl]piperidin-4-ol

The titled compound was prepared by following the procedure of Example 28, but replacing ammonium hydroxide with glycine methyl ester hydrochloride.
¹H-NMR (CDCl₃) δ: 1.66-1.74(3H, m), 1.97-2.15(2H, m), 2.37-2.80(8H, m), 3.63(3H, s), 3.78(2H, s) 5.40(2H, brs), 6.22(1H, t), 6.92(1H, d), 7.28-7,45(5H, m), 7:62(2H, dd), 7.83(1H, d), 8.53(1H, dd).
MS m/z: 598(M+1)

### Example 32

### 1-[3-(7-(N-Carboxymethylsulfamoyl-5,11-dihydro[1]benzoxepino[2,3-b]pyridin-5-ylidene)propyl] -4-(4-chlorophenyl)piperidin-4-ol

The titled compound was prepared by following the procedure of reference example 9, but replacing the product of reference example 6 with the product of Example 31.
¹H-NMR (DMSO-d6) δ: 1.60-1.65(2H, m), 2.16-2.25(2H, m), 2.43-3.03(8H, m), 3.45(2H, s), 5.33(2H, brs), 6.39(1H, t), 6.94(1H, d), 7.41-7.57(6H, m), 7.83(1H, dd), 8.00(1H, d), 8.54(1H, dd).

### Example 33

### 4-(4-Chlorophenyl)-1-[3-(5,11-dihydro-7-(N-(2,2,2-trifluoroethyl)sulfamoyl)[1]benzoxepino[2,3-b]pyridin-5-ylidene)propyl]piperidin-4-ol

The titled compound was prepared by following the procedure of Example 28, but replacing ammonium hydroxide with 2,2,2-trifluoroethylamine hydrochloride.
¹H-NMR (CDCl₃) δ: 1.64-1.77(2H, m), 1.97-2.18(2H, m), 2.35-2.80(8H, m), 3.63(2H, q), 5.41(2H, brs), 6.21(1H, t), 6.91(1H, d), 7.22-7.65(7H, m), 7.84(1H, d), 8.57(1H, dd).
MS m/z: 608(M+1)

### Example 34

### 4-(4-Chlorophenyl)-1-[3-(5,11-dihydro-7-ureido[1]benzoxepino[2,3-b]pyridin-5-ylidene)propyl]piperidin-4-ol

### Step 1

### 4-(4-Chlorophenyl)-1-[3-(5,11-dihydro-7-pheoxycarbonylamino[1]benzoxepino[2,3-b]pyridin-5-ylidene)propyl]piperidin-4-ol

The titled compound was prepared by following the procedure of reference Example 11, but replacing ethanol with phenol.
¹H-NMR (CDCl₃) δ: 1.62-1.68(2H, m), 1.96-2.08(2H, m), 2.35-2.65(8H, m), 5.28(2H, brs), 6.10(1H, t), 6.78(1H, m), 7.08-7.40(6H, m), 7.52(1H, dd), 7.62(1H, s), 8.44(1H, dd).
MS m/z: 582(M+1)

### Step 2

To a solution of the product of Step 1 (300mg) in DMF (3ml) was added ammonium hydroxide (1.5ml) and the mixture was stirred at room temperature for 2 hours. Ethyl acetate and water were added to the mixture, the organic layer was separated and washed with saturated aqueous sodium chloride, and dried with magnesium sulfate. The solvent was distilled off under reduced pressure. The residue was purified by silica gel chromatography eluting with (chloroform : methanol = 10 : 1) to give the titled compound (140mg).
¹H-NMR (DMSO-d6) δ: 1.45-1.50(2H, m), 1.72-1.88(2H, m), 2.28-2.51(8H, m), 4.82(1H, s), 5.19(1H, brs), 5.74(2H, brs), 6.09(1H, t), 6.69(1H, d), 7.12(1H, dd), 7.32-7.48(6H, m), 7.74(1H, dd), 8.37(1H, s), 8.50(1H, dd).
MS m/z: 505(M+1)

### Example 35

### 4- (4-Chlorophenyl) -1-[3-(5,11-dihydro-7-(1-morpholinocarbonylamino[1]benzoxepino[2,3-b]pyridin-5-ylidene)propyl]piperidin-4-ol

The titled compound was prepared by following the procedure of Example 34, step 2, but replacing ammonium hydroxide with morpholine.
¹H-NMR (CDCl₃) δ: 1.62-1.67(2H, m), 1.95-2.16(2H, m), 2.28-2.64(8H, m), 3.41(4H, t), 3.69(4H, t), 5.26 (2H, brs), 6.08(1H, t), 6.69-6.76(2H, m), 6.98(1H, dd), 7.21-7.51(7H, m), 8.42(1H, dd).
MS m/z: 575(M+1)

### Example 36

### 4-(4-Chlorophenyl)-1-[3-(5,11-dihydro-7-(3-(2-ethoxy)carbonylethyl)ureido[1]benzoxepino[2,3-b]pyridin-5-ylidene)propyl]piperidin-4-ol

The titled compound was prepared by following the procedure of Example 34, step 2, but replacing ammonium hydroxide with beta-alanine ethyl ester hydrochloride.
¹H-NMR (CDCl₃) δ: 1.18-1.39(3H, t), 1.62-1.66(2H, m), 1.92-2.01(2H, m), 2.21-2.62(10H, m), 3.47-3.50(2H, m), 4.08(2H, q), 5.22(2H, brs), 5.98-6.03(2H, m), 6.68-6.92(2H, m), 7.15-7.42(7H, m), 7.62(1H, s), 8.36(1H, dd).
MS m/z: 605(M+1)

### Example 37

### 1-[3-(7-(E)-(2-Carboxy-1-methyl)ethenyl-5,11-dihydro[1]benzoxepino[2,3-b]pyridin-5-ylidene)propyl]-4-(4-chlorophenyl)piperidin-4-ol

### Step 1

4-(4-Chlorophenyl)-1-[3-(7-(E)-(2-ethoxycarboxy-1-methyl)ethenyl-5,11-dihydro[1]benzoxepino[2,3-b]pyridin-5-ylidene)propyl]piperidin-4-ol was prepared by following the procedure of reference Example 13, but replacing ethyl cyanoformate with ethyl (trimethylsilyl)acetate.
¹H-NMR (CDCl₃) δ: 1.30(3H, t), 1.67-1.72(3H, m), 1.98-2.05(2H, m), 2.42-2.67(11H, m), 4.23(2H, q), 5.36(2H, brs), 6.14-6.19(2H, m), 6.85(1H, d), 7.20-7.61(8H, m), 8.52(1H, dd).

### Step 2

The titled compound was prepared by following the procedure of reference Example 9, but replacing the product of reference Example 6 with the product of step 1.
¹H-NMR (DMSO-d6) δ: 1.50-1.55(2H, m), 1.87-1.99(2H, m), 2.34-2.61(11H, m), 5.29(2H, brs), 6.12(1H, s), 6.31(1H, t), 6.83(1H, d), 7.35-7.49(7H, m), 7.76(1H, dd), 8.52(1H, dd).
MS m/z: 530(M+1)

### Example 37

### 1-[3-(7-(3-(2-Carboxy)ethyl)ureido-5,11-dihydro[1]benzoxepino[2,3-b]pyridin-5-ylidene)propyl]-4-(4-chlorophenyl)piperidin-4-ol

The titled compound was prepared by following the procedure of reference Example 9, but replacing the product of reference Example 6 with the product of Example 36.
¹H-NMR (DMSO-d6) δ: 1.45-1.55(2H, m), 1.72-1.85(2H, m), 2.32-2.49(10H, m), 3.29(2H, q), 4.88(1H, s), 5.19(2H, brs), 6.06-6.14(2H, m), 6.69(1H, d), 7.07(1H, dd), 7.33-7.48(6H, m), 7.73(1H, dd), 8.43(1H, s), 8.49(1H, dd).
MS m/z: 577(M+1)

### Example 38

### 1- [3-(7-(3- (2-Hydroxy)ethyl)ureido-5,11-dihydro [1] benzoxepino [2,3-b]pyridin-5-ylidene) propyl]-4-(4-chlorophenyl)piperidin-4-ol

The titled compound was prepared by following the procedure of Example 34, step 2, but replacing ammonium hydroxide with 2-aminoethanol.
¹H-NMR (DMSO-d6) δ : 1.45-1.51(2H, m), 1.72-1.84(2H, m), 2.24-2.51(8H, m), 3.11-3.46(4H, m), 4.71(1H, t), 4.83(1H, s), 5.19(2H, brs), 6.08(1H, t), 6.69(1H, d), 7.08(1H, dd), 7.33-7.48(6H, m), 7.73(1H, dd), 8.41(1H, s), 8.50(1H, dd).
MS m/z: 549(M+1)

### Example 39

### 1-[3-(7-(E)-(2-Carboxy-2-methyl)ethenyl-5,11-dihydro[1]benzoxepino[2,3-b]pyridin-5-ylidene)propyl]-4-(4-chlorophenyl)piperidin-4-ol

The titled compound was prepared by following the procedure of reference Example 9, but replacing the product of reference Example 6 with the product of step 1.
¹H-NMR (DMSO-d6) δ: 1.62-1.67(2H, m), 1.91-2.05(5H, m), 2.50-2.94(8H, m), 5.28(2H, brs), 6.23(1H, t), 6.87(1H, d), 7.34-7.55(8H, m), 7.79(1H, dd), 8.54(1H, dd).
MS m/z: 531(M+1)

### Example 41

### 4-(4-Chlorophenyl)-1-[3-(5,11-dihydro-7-(3-phenylureido) sulfonyl [1]benzoxepino [2,3-b]pyridin-5-ylidene)propyl]piperidin-4-ol

The titled compound was prepared by following the procedure of Example 16, but replacing compound of reference Example 3, step 2 with compound of Example 28, step 2.
¹H-NMR (DMSO-db) δ : 1.65-1.69(2H, m), 1.95-2.05(2H, m), 2.89-3.06(8H, m), 5.31 (2H, brs), 6.14(1H, t), 6.74-6.85(2H, m), 7.08-7.12(2H, m), 7.37-7.64(8H, m), 7.80-7.84(2H, m), 8.44(1H, s), 8.54(1H, dd).
MS m/z: 645(M+1)

### Example 42

### 4-(4-Chlorophenyl)-1-[3-(7-(3-cyclohexylureido)sulfonyl-5,11-dihydro[1]benzoxepino[2,3-b]pyridin-5-ylidene)propyl]piperidin-4-ol

The titled compound was prepared by following the procedure of Example 41, but replacing phenyl isocyanate with cyclohexyl isocyanate.
¹H-NMR (DMSO-d6) δ: 1.07-1.81(14H, m), 2.23-2.58(8H, m), 3.22-3.35(1H, m), 4.91(1H, s), 5.38(2H, brs), 6.17-6.29(2H, m), 6.96(1H, d), 7.34-7.51(5H, m), 7.62-7.84(3H, m), 8.53(1H, dd).
MS m/z: 651(M+1)

### Example 43

### 4-(4-Chlorophenyl)-1-[3-(5,11-dihydro-7-(3-propylureido)sulfonyl[1]benzoxepino[2,3-b]pyridin-5-ylidene)propyl]piperidin-4-ol

The titled compound was prepared by following the procedure of Example 41, but replacing phenyl isocyanate with propyl isocyanate.
¹H-NMR (DMSIO-d6) δ: 0.74(3H, t), 1.25-1.53(4H, m), 1.81-1.91(2H, m), 2.33-2.59(10H, m), 2.89(2H, q), 4.92(1H, s), 5.35(2H, brs), 6.20(1H, t), 6.44(1H, brs), 6.96(1H, d), 7.34-7.51(5H, m), 7.64(1H, dd), 7.78-7.85(2H, m), 8.54(1H, dd).
MS m/z: 611(M+1)

### Example 44

### 1-[3-(7-Benzoylsulfamoyl-5,11-dihydro[1]benzoxepino[2,3-b]pyridin-5-ylidene)propyl]-4-(4-chlorophenyl)piperidin-4-ol

The titled compound was prepared by following the procedure of Example 41, but replacing phenyl isocyanate with benzoyl chloride.
MS m/z: 630 (M+1)

### Reference Example 13

### 4-(4-Chlorophenyl)-1-[3-(5,11-dihydro-7-ethoxycarbonylacetyl[1]benzoxepino[2,3-b]pyridin-5-ylidene)propyl]piperidin-4-ol

To a solution of the product of reference Example 14 (250 mg) in THF (3.0 ml) was added LDA (0.51 mol/L THF-hexane solution, 3.0 ml) at -78°C, and the mixture stirred at room temperature for 20 minutes. The reaction mixture was cooled to -78°C again, and added ethyl cyanoformate (76 µl), stirred at room temperature for 1 hour. Saturated aqueous ammonium chloride and aqueous sodium chloride were added to the mixture, and the aqueous layer was extracted with ethyl acetate. The organic layer was washed with saturated aqueous sodium chloride, and dried with magnesium sulfate. The solvent was distilled off under reduced pressure. The residue was purified by silica gel chromatography eluting with chloroform-methanol (10:1) to give the titled compound (280 mg).
¹H-NMR (CDCl₃) δ: 1.26(3H, t), 1.67-1.85(2H, m), 1.93-2.13(2H, m), 2.28-2.47(4H, m), 2.47-2.60(2H, m), 2.60-2.76(2H, m) 3.94(2H, s), 4.21(2H, q), 5.60(2H, brs), 6.22(1H, t), 6.88(1H, d), 7.29-7.34(3H, m), 7.43(2H, d), 7.59(1H, d), 7.71(1H, dd), 7.97(1H, d), 8.53(1H, d).
MS m/z: 561(M+1)

### Reference Example 14

### 1-[3-(7-Acetyl-5,11-dihydro[1]benzoxepino[2,3-b]pyridin-5-ylidene)propyl]-4-(4-chlorophenyl)piperidin-4-ol

### Step 1

To a solution of reference example 7, step 1 (7.2g) in dichloromethane (70 ml) was added aluminum chloride (9.1 g) and acetyl chloride (3.2 ml), and the mixture stirred at 0°C for 10 minutes. The reaction mixture was poured into ice. The aqueous layer was extracted with ethyl acetate, and the organic layer was washed with saturated aqueous sodium chloride, and dried with magnesium sulfate. The solvent was distilled off under reduced pressure. The Residue was purified by silica gel chromatography, eluting with ethyl acetate-hexane (1:2) to give 7-acetyl-5-(3-bromopropylidene)-5,11-dihydro[1]benzoxepino[2,3-b]pyridine (7.9 g).
¹H-NMR (CDCl₃) δ:2.57(3H, s), 2.77(2H, m), 3.49(2H, t), 5.40(2H, brs), 6.16(1H, t), 6.88(1H, d), 8.33(1H, dd), 7.58(1H, dd), 7.77(1H, dd), 7.96(1H, d), 8.56(1H, dd).

### Step 2

The titled compound was prepared by following the procedure of reference example 3, step 2, but replacing the product of reference example 3, step 1 with the product of step 1.
¹H-NMR (CDCl₃) δ:1.52-1.79(2H, m), 1.93-2.11(2H, m), 2.27-2.49(4H, m), 2.49-2.60(5H, m), 2.60-2.73(2H, m), 5.40(2H, brs), 6.22(1H, t), 6.87(1H, d), 7.29-7.34(3H, m), 7.42(2H, d), 7.59(1H, dd), 7.75 (1H, dd), 7.96(1H, d), 8.53(1H, dd).
MS m/z: 489(M+1)

### Example 45

### 4-(4-Chlorophenyl)-1-[3-(5,11-dihydro-7-diethylcarbamoyl-[1]benzoxepino[2,3-b]pyridin-5-ylidene)propyl]piperidine-4-ol

The titled compound was prepared by following the procedure of Example 12, but replacing dimethylamine with diethylamine.
¹H-NMR (CDCl₃) δ: 1.18-1.30 (6H, m), 1.65 (2H, d), 1.80 (1H, s), 2.05 (2H, dt), 2.30-2.45 (4H, m), 2.50 (2H, t), 2.60-2.70 (2H, m), 3.35-3.50 (4H, m), 5.30 (2H, brs), 6.15 (1H, t), 6.83 (1H, d), 6.90 (1H, dd), 7.10 (1H, dd), 7.23-7.35 (3H, m), 7.40 (2H, d), 7.56 (1H, dd), 8.50 (1H, dd).
MS m/z: 563

### Example 46

### 4-(4-Chlorophenyl)-1-[3-(5,11-dihydro-7-phenylsulfonylcarbamoyl-[1]benzoxepino[2,3-b]pyridin-5-ylidene)propyl]piperidine-4-ol

To a solution of the compound of reference Example 3 (0.511 g, 1.1 mmol) in dry THF (20 mL) was added sodium hydride (60% in mineral oil, 48 mg, 1.2 mmol), and the slurry heated at 40°C under argon with stirring for 20 minutes.
Phenylsulfonylisocyanate (160 µL, 1.2 mmol) was added and the mixture was stirred for 14 hours. The solvent was then removed by rotary evaporation to give the crude product. The solid material was washed twice with 20 mL CH₂Cl₂, and then twice with 20 mL MeoH: CH₂Cl₂ (1:1) to give the title compound (274 mg).
MS m/z:647

### Example 47

### 4-(4-Chlorophenyl)-1-[3-(5,11-dihydro-7-methoxycarbonyl-carbamoyl-[1]benzoxepono[2,3-b]pyridin-5-ylidene) propyl]piperidine-4-ol

To a solution of the compound of reference Example 3 (0.214 g, 0.46 mmol) in dry THF (5mL) was added sodium hydride (60% in mineral oil, 28 mg, 0.7 mmol), and the slurry heated at 50°C under argon with stirring for 20 minutes. Methyl isocyanatoformate (56 µl, 0.7 mmol) was added and the mixture was stirred for 14 hours. The solvent was then removed by rotary evaporation to give the crude product. The residue was purified by silica gel chromatography eluting with a dichloromethane/2.0 M ammonia in methanol gradient (0 to 4% MeOH over 1 hour) to give the title compound (102 mg).
¹H-NMR (CDCl₃) δ: 1.60-1.65 (2H, m), 1.80 (1H, s), 2.05 (2H, dt), 2.30-2.45(4H, m), 2.50 (2H, t), 2.60-2.70 (2H, m), 3.35 (3H, s), 5.30 (2H, brs), 6.15 (1H, t), 6.83 (1H, d), 6.90 (1H, dd), 7.10 (1H, dd), 7.23-7.35 (3H, m), 7.40 (2H, d), 7.56 (1H, dd), 8.50 (1H, dd).
MS m/z: 565

### Example 48

### 4- (4-Chlorophenyl) -1- [3- (5,11-dihydro-7-(R-3-ethoxycarbonyl-piperidine-1-yl)-carbamoyl-[1]benzoxepino[2,3-b]pyridin-5-ylidene) propyl]piperidine-4-ol

### Step 1:

R-ethyl nipecotate-L-tartrate (1.53 g) was freebased with aqueous sodium hydroxide and ethyl acetate. The organic layers were evaporated, and the resulting amine was redissolved in THF (10 mL) and treated with carbonyldiimidazole (0.81 g). The resulting solution was stirred at room temperature for 23 hours, concentrated *in vacuo,* and redissolved in acetonitrile (5 mL). This solution was treated with methyl iodide (0.347 mL) and stirred for 18 hours at room temperature.

### Step 2:

The compound of reference Example 3 (0.7 g) was suspended in THF (25 mL) and treated with sodium hydride (0.036 g) and stirred at room temperature for one hour. The resulting anion was added to the imidazolium salt prepared in Step 1, and the solution was heated to reflux for 18 hr. The crude material was then loaded on silica gel and purified by silica gel chromatography (87:10:3 ethyl acetate:methanol:triethylamine) to yield 0.278 g (64%) of the title compound:
¹H-NMR (DMSO) δ: 1.11-1.21 (3H, m), 1.45-2.0 (8H, m), 2.15-2.40 (6H, m), 3.05-3.15 (2H, m), 3.31 (2H, m), 3.95-4.15 (3H, m), 5.31 (2H, brs), 6.14 (1H, t), 6.78 (1H, d), 6.92 (1H, dd), 7.05 (1H, d), 7.33 (2H, d), 7.42-7.47 (3H, m), 7.72 (1H, dd), 8.50 (1H, dd).
ESI-MS m/z: 646 (M + 1).

### Example 49

### 4-(4-Chlorophenyl)-1-[3-(5,11-dihydro-7-(R-3-ethoxycarbonyl-piperidine-1-yl)-carbamoyl-[1]benzoxepino[2,3-b]pyridin-5-ylidene) propyl]piperidine-4-ol

The compound of Example 48 (0.195 g) was dissolved in THF (1 mL) and treated with aqueous lithium hydroxide (0.0084 g) and stirred at room temperature for 18 hours. The resulting solution was concentrated *in vacuo,* and the residue was purified by chromatography on a reverse-phase solid-phase-extraction column, eluting with water-acetonitrile, 0.1% formic acid, to yield 0.153 g (77%) of the title compound.
¹H-NMR (DMSO) δ: 1.55-2.25 (8H, m), 2.30-2.80 (10H, m), 3.22 (1H, m), 4.15-4.35 (2H, m), 5.41 (2H, brs), 6.35 (1H, t), 6.98 (1H, d), 7.13 (1H, dd), 7.25 (1H, d), 7.54 (2H, d), 7.64 (3H, m), 7.90 (1H, dd), 8.50 (1H, s), 8.70 (1H, dd).
ESI-MS m/z: 618 (M + 1).

### Example 50

### 4-(4-Chlorophenyl)-1-[3-(5,11-dihydro-7-(4-ethoxycarbonyl-piperidine-1-yl)-carbamoyl-[1]benzoxepino[2,3-b]pyridin-5-ylidene) propyl]piperidine-4-ol

The titled compound was prepared by following the procedure of Example 48, but replacing R-ethyl nipecotate-L-tartrate with ethyl isonipecotate.
¹H-NMR (CDCl₃) δ: 1.25(3H, t), 1.60-1.80 (4H, m), 1.90-2.05 (4H, m), 2.25-2.65 (10H, m), 2.90-3.15 (2H, m), 4.05-4.25 (9H, m), 5.30 (2H, brs), 6.15 (1H, t), 6.75-6.90 (2H, m), 7.05 (1H, d), 7.20-7.40 (3H, m), 7.40 (2H, d), 7.56 (1H, dd), 8.45 (1H, dd).
MS m/z: 647

### Example 51

### 4-(4-Chlorophenyl)-1-[3-(5,11-dihydro-7-(4-carboxy-piperidine-1-yl)-carbamoyl-[1]benzoxepino [2,3-b]pyridin-5-ylidene) propyl]piperidine-4-ol

A solution of the compound of Example 50 (91 mg, 0.14 mmol) in MeOH (5 mL) was treated with a 0.4 M solution of lithium hydroxide (5 mL, 2 mmol) and stirred for 3 hours. After addition of 5 mL of 0.4 N HCl, the solvent was removed under reduced pressure to give the crude product. The residue was purified using silica gel chromatography eluting with a dichloromethane:methanol gradient (0 to 50% MeOH over 1 hour) to give the title compound (48 mg).
¹H-NMR (MeOD) δ: 1.60-1.65 (2H, m) 2.10-2.70 (10H, m), 5.30 (2H, brs), 6.15 (1H, t), 6.80-6.90 (2H, m), 7.20-7.50 (6H, m), 7.62 (1H, dd), 8.48 (1H, dd).
MS m/z:619.

### Example 52

### 4-(4-Chlorophenyl)-1-[3-(5,11-dihydro-7-(S-3-ethoxycarbonyl-piperidine-1-yl)- carbamoyl-[1]benzoxepino[2,3-b]pyridin-5-ylidene) propyl]piperidine-4-ol

The titled compound was prepared by following the procedure of Example 48, but replacing R-ethyl nipecotate-L-tartrate with ethyl (S)-nipecotate-D-tartrate.
¹H-NMR (CDCl₃) δ: 1.25 (3H, t), 1.30-1.70 (5H, m), 1.94-2.05 (3H, m), 2.25-2.65 (11H, m), 3.05-3.15 (1H, m), 4.05-4.25 (4H, m), 5.30 (2H, brs), 6.15 (1H, t), 6.75-6.90 (2H, m), 7.05 (1H, d), 7.20-7.40 (3H, m), 7.40 (2H, d), 7.56 (1H, dd), 8.45 (1H, dd).
MS m/z: 647

### Reference Exemple 15

### 4-(4-Chlorophenyl)-1-[3-(5,11-dihydro-7-(1-propoxy) carbonyl- [1] benzoxepino [2,3-b] pyridin-5-ylidene) propyl]piperidine-4-ol

To a solution of the compound of reference Example 8 (109 mg, 0.22 mmol) in dry DMF (5 mL) was added potassium carbonate (91 mg) followed by propyl iodide (24 µL, 0.66 mmol) . The mixture was heated to 55°C for 14 hours. The mixture was diluted with ethyl acetate (200 mL), washed twice with water (200 mL) and then with brine (100 mL), and dried with sodium sulfate. The organic solvent was removed under reduced pressure and the residue subjected to silica gel chromatography using a dichloromethane : methanol gradient (0 to 5% MeOH over 1 hour) to give the title compound (103 mg).
¹H-NMR (CDCl₃) δ: 1.06 (3H, t), 1.50-2.10 (4H, m), 2.14-2.25 (2H, m), 2.31-2.75 (10H, m), 4.28 (2H, t), 6.15 (1H, t), 6.83 (1H, d), 7.24-7.38 (3H, m), 7.42 (2H, d), 7.59 (1H, dd), 7.78 (1H, dd), 8.00 (1H, d), 8.50 (1H, dd) .
MS m/z: 533

### Example 53

### 4-(4-Chlorophenyl)-1-[3-(5,11-dihydro-7-(1-2,2-dimethylpropionyl-oxymethyl) -oxycarbonyl-[1] benzoxepino [2,3-b] pyridin-5-ylidene) propyl] piperidine-4-ol

The procedure of reference Example 15 was followed, but replacing with chloromethyl pivalate to yield 0.36 g (77%) of the title compound.
¹H-NMR (CDCl₃) δ: 1.18 (9H, s), 1.58-1.72 (2H, m), 1.85-2.85 (10H, m), 5.00-5.60 (2H, brs), 5.94 (2H, s), 6.17 (1H, t), 6.82 (1H, d), 7.22-7.42 (5H, m), 7.56 (1H, dd), 7.80 (1H, dd), 7.99 (1H, d), 8.05 (1H, d), 8.46 (1H, dd). ESI-MS m/z: 605 (M + 1).

Those skilled in the art will be able to recognize, or be able to ascertain, using no more than routine experimentation, many equivalents to the specific embodiments of the invention described herein. Such equivalents are intended to be encompassed by the following claims.

## Claims

1. A compound which is a tertiary amine of the following formula: 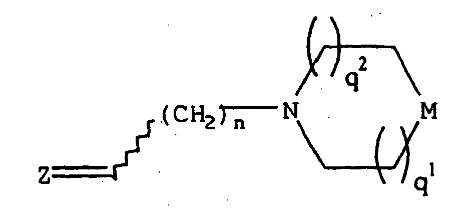 wherein:
n is one to four,
M is >NR², >CR¹R², -O-CR¹R²-O- or -CH₂-CR¹R²-O-;
q¹ is zero to three;
q² is zero or one;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴ -NR³R⁴ or R¹ is a covalent bond between the ring atom at M and an adjacent carbon atom in the ring which contains M;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is: 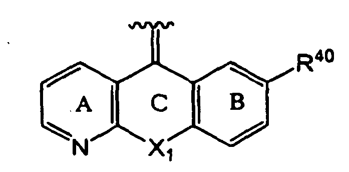
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂-, or a bond;
R⁴⁰ is -S(O₂)-NR²¹R²², -NH-C(O)-NR²¹R²², -O-C(O)-NR²¹R²⁶, 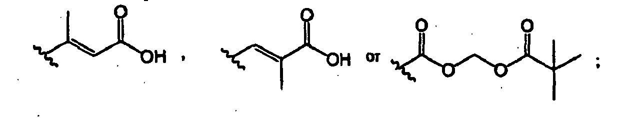
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵,-S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², (O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)p-(non-aromatic heterocyclic group), oxo, epoxy, non-aromatic heterocycle, benzyl, substituted benzyl, aromatic group and substituted aromatic group;
said substituted benzyl group and said substituted aromatic group comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic goup), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵,-S(O)₂NR²⁴R²⁵, -SO₃H, -S(O)₂NH₂, guanidino, -(O)ᵤ-(CH₂)t-C(O)OR²⁰, -(O)ᵤ-(CH₂)ₜOC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group);
R²⁶ is -H, aliphatic group, substituted aliphatic group, aromatic group, substituted aromatic group, non-aromatic heterocyclic group, -C(O)-O-(substituted or unsubstituted aliphatic group), -C(O)-O-(substituted or unsubstituted aromatic group),
-S(O)₂-(substituted or unsubstituted aliphatic group), -S(O)₂-substituted or unsubstituted aromatic group; or
R²¹ and R²², taken together with the nitrogen atom to which they are bonded, form a non-aromatic heterocyclic ring; or
R²¹ taken together with R²⁶ and the nitrogen atom to which they are bonded form a substituted or unsubstituted non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings;
or a physiologically acceptable salt thereof.

2. A compound according to claim 1 wherein q¹ and q² are each one.

3. A compound according to claim 2 wherein M is >CR¹R².

4. A compound according to claim 3 wherein R¹ is -OH and R² is a substituted aromatic group.

5. A compound according to claim 4 wherein R² is 4-chlorophenyl.

6. A compound which is a tertiary amine of the following formula: 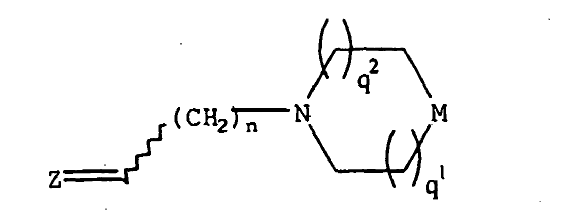 wherein:
n is one to four;
M is >CR¹R², -O-CR¹R²-O- or -CH₂-CR¹R²-O-;
q¹ is zero to three;
q² is zero or one;
R¹ is a substituted aliphatic group or an aminoalkyl group;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R⁵ and R⁶ taken together with the atom to which they are bonded, form a substituted or unsubstituted non-aromatic carbocyclic or heterocyclic ring;
Z is: 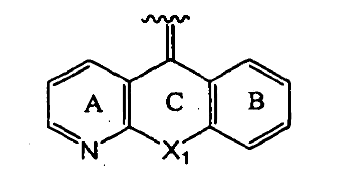
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond;
ring A and ring B are independently substituted or unsubstituted,
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰ -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group and rings A and B when substituted comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)p-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring;
R ²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings;
or a physiologically acceptable salt thereof, other than:

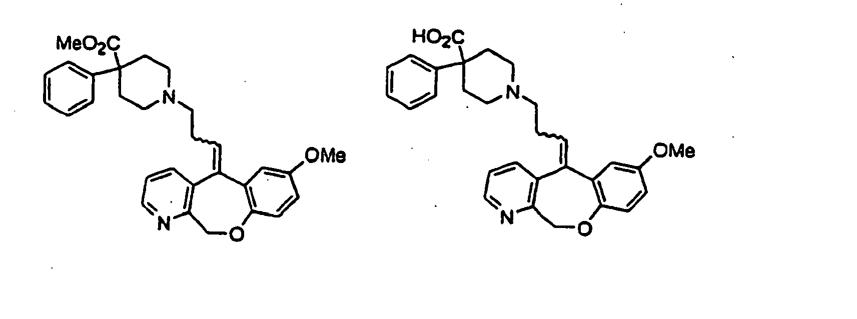

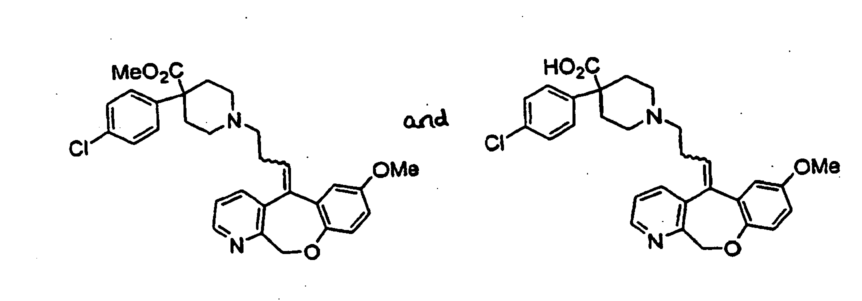

7. A compound according to claim 6 wherein q¹ and q² are each one.

8. A compound according to claim 7 wherein M is >CR¹R²

9. A compound according to claim 8 wherein R² is a substituted aromatic group.

10. A compound according to claim 9 wherein R² is 4-chlorophenyl.

11. A compound according to any one of claims 6-10 wherein R¹ is an alkylamino group selected from the group consisting of aminomethyl, 2-aminoethyL 3-aminopropyl, 4-aminobutyl, dimethylaminoethyl, diethylaminomethyl, methylaminohexyl and aminoethylenyl.

12. A compound which is a tertiary amine of the following formula: 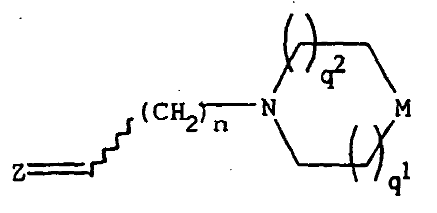 wherein:
n is one to four;
M is >NR², >CR¹R², -O-CR¹R²-O- or -CH₂-CR¹R²-O-;
q¹ is zero to three;
q² is zero or one;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -O-(substituted or unsubstituted aromatic group);
R³ and R⁴ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R³ and R⁴ taken together with the atom to which they are bonded, form a substituted or unsubstituted non-aromatic carbocyclic or heterocyclic ring;
Z is: 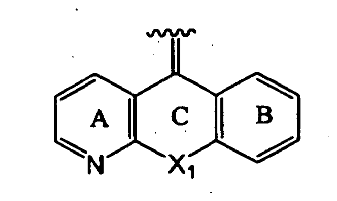 wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond;
ring A and ring B are independently substituted or unsubstituted,
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂. guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group),-Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group and rings A and B when substituted comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring; or
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings;
or a physiologically acceptable salt thereof.

13. A compound according to claim 12 wherein q¹ and q² are each one.

14. A compound according to claim 12 wherein M is >CR¹R²

15. A compound according to claim 8 wherein R¹ is -OH.

16. A compound which is a tertiary amine of the following formula: 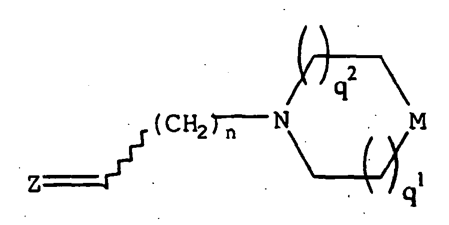 wherein:
n is one to four;
M is -O-CR¹R²-O- or -CH₂-CR¹R²-O-;
q¹ is zero to three;
q² is zero or one;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is: 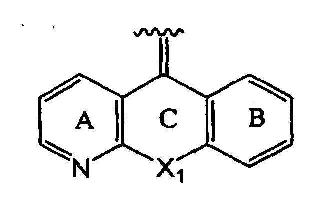
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond;
ring A and ring B are independently substituted or unsubstituted;
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂N²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H., -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of 0, S and N, and a C₆ aromatic carbocycle or a C₅ - C₆ aromatic heterocycle that is fused to one or more other rings;
or a physiologically acceptable salt thereof.

17. A compound according to claim 16 wherein q¹ and q² are each one.

18. A compound according to claim 17 wherein R¹ is -OH and R² is a substituted aromatic group.

19. A compound according to claim 18 wherein R² is 4-chlorophenyl.

20. A compound which is a tertiary amine of the following formula: 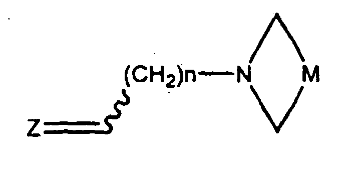 wherein:
n is one to four;
M is >NR² or >CR¹R²_{;}
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group); -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is: 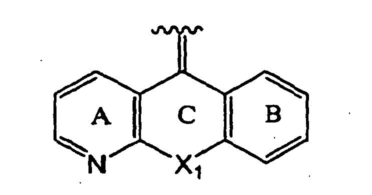
wherein:
X₁ is -CH₂-S-, -S-CH₂-, -O-CH₂-, -CH₂O-, -SO-CH₂-, -CH₂-SO-, -S(O)₂-CH₂-, -CH₂-S(O)₂- or a bond;
ring A and ring B are independently substituted or unsubstituted;
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic.group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic goup, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR¹⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N; and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings;
or a physiologically acceptable salt thereof.

21. A compound according to claim 20 wherein M is >CR¹R².

22. A compound according to claim 21 wherein R¹ is -OH and R² is a substituted aromatic group.

23. A compound according to claim 22 wherein R² is 4-chlorophenyl.

24. A compound which is a tertiary amino of the following formula: 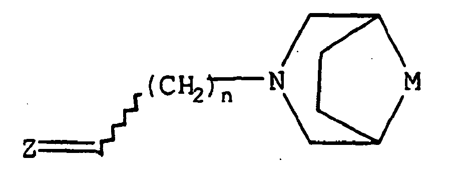 wherein:
n is one to four;
M is >NR², >CR¹R², -O-CR¹R²-O- or -CH₂-CR¹R²-O-;
the ring containing M is substituted or unsubstituted;
R¹ is -H, -OH, -N₃, a halogen, an aliphatic group, a substituted aliphatic group, an aminoalkyl group, -O-(aliphatic group), -O-(substituted aliphatic group), -SH, -S-(aliphatic group), -S-(substituted aliphatic group), -OC(O)-(aliphatic group), -O-C(O)-(substituted aliphatic group), -C(O)O-(aliphatic group), -C(O)O-(substituted aliphatic group), -COOH, -CN, -CO-NR³R⁴, -NR³R⁴ or R¹ is a covalent bond between the ring atom at M and an adjacent carbon atom in the ring which contains M;
R² is -OH, an acyl group, a substituted acyl group, -NR⁵R⁶, an aliphatic group, a substituted aliphatic group, an aromatic group, a substituted aromatic group, a benzyl group, a substituted benzyl group, a non-aromatic heterocyclic group, a substituted non-aromatic heterocyclic group, -O-(substituted or unsubstituted aromatic group) or -O-(substituted or unsubstituted aliphatic group);
R³, R⁴, R⁵ and R⁶ are independently -H, an acyl group, a substituted acyl group, an aliphatic group, a substituted aliphatic group, an aromatic group, a substituted aromatic group, a benzyl group, a substituted benzyl group, a non-aromatic heterocyclic group or a substituted non-aromatic heterocyclic group; or
R¹ and R², R³ and R⁴, or R⁵ and R⁶ taken together with the atom to which they are bonded, form a substituted or unsubstituted non-aromatic carbocyclic or heterocyclic ring;
Z is: 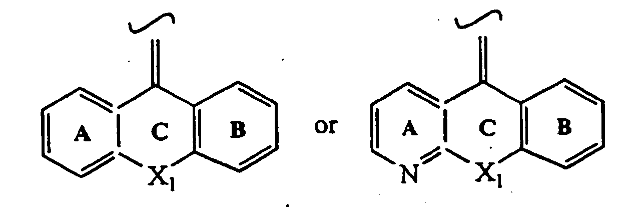
wherein:
X₁ is -CH₂-S-, -S-CH₂-, -O-CH₂-, -CH₂-O-, -SO-CH₂-, -CH₂-SO-, -S(O)rCH₂-, -CH₂-S(O)₂- or a bond; and
ring A and ring B are independently substituted or unsubstituted,
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH_{2,} guanidino, -(O)ᵤ-(CH₂)ₜ-C(O)OR²⁰, -(O)ᵤ-(CH₂)ₜ-OC(O)R²⁰, -(O)ᵤ(CH₂)ᵣC(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group and rings A and B when substituted comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings,
or a physiologically acceptable salt thereof.

25. A compound which is a tertiary amino of the following formula: 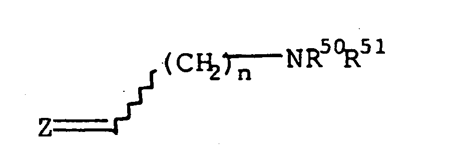 wherein:
n is one to four,
R⁵⁰ and R⁵¹ are each, independently, -H, an aliphatic group, a substituted aliphatic group, an aminoalkyl group, -NR³R⁴, an aromatic group, a substituted aromatic group, a benzyl group, a substituted benzyl group, a non-aromatic heterocyclic group, a substituted non-aromatic heterocyclic group, or a covalent bond between the nitrogen atom an adjacent carbon atom;
R³ and R⁴ are independently -H, an acyl group, a substituted acyl group, an aliphatic group, a substituted aliphatic group, an aromatic group, a substituted aromatic group, a benzyl group, a.substituted benzyl group, a non-aromatic heterocyclic group or a substituted non-aromatic heterocyclic group;
Z is: 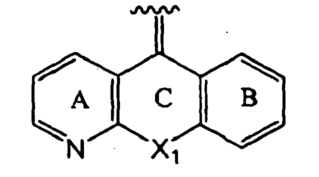
wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond;
ring A and ring B are independently substituted or unsubstituted,
wherein:
said substituted aliphatic group comprises one or more substituents selected from the group consisting of an electron withdrawing group, halo, azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), oxo, epoxy, non-aromatic heterocycle, benzyl, substituted benzyl, aromatic group and substituted aromatic group;
said substituted benzyl group and said substituted aromatic group and rings A and B when substituted comprise one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(C)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ(CH₂)ₜ-C(O)-NR²¹R²², -(O)ᵤ-(CH₂)ₜ-NHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic group), aliphatic group and substituted aliphatic group;
said substituted non-aromatic heterocyclic group comprises one or more substitutents selected from the group consisting of an electron withdrawing group, halo, azido, -CN, -COOH, -OH, -CONR²⁴R²⁵, -NR²⁴R²⁵, -OS(O)₂NR²⁴R²⁵, -S(O)₂NR¹⁴R¹¹, -SO₃H, -S(O)₂NH₂, guanidino, -(O)ᵤ-(CH₂)ₜ-C(O)OR²⁰, -(O)ᵤ-(CH₂)ₜOC(O)R²⁰, -(O)ᵤ-(CH₂)ₜC(O)-NR²¹R²², -(O)ᵤ-(CH₂)ₜNHC(O)O-R²⁰, -Q-H, -Q-(aliphatic group), -Q-(substituted aliphatic group), -Q-(aryl), -Q-(aromatic group), -Q-(substituted aromatic group), -Q-(CH₂)ₚ-(substituted or unsubstituted aromatic group), -Q-(non-aromatic heterocyclic group), -Q-(CH₂)ₚ-(non-aromatic heterocyclic goup), aliphatic group, substituted aliphatic group, =O, =S, =NH, =N(aliphatic), =N(aromatic) and =N(substituted aromatic);
R²⁰, R²¹ and R²² are independently -H, an aliphatic group, a substituted aliphatic group, an aromatic group, a substituted aromatic group, a non-aromatic heterocyclic group, -NHC(O)-O-(aliphatic group), -NHC(O)-O-(aromatic group) or -NHC(O)-O-(non-aromatic heterocyclic group); or
R²¹ and R²², taken together with the nitrogen atom to which they are bonded, form a non-aromatic heterocyclic ring;
R²³ is -H, an aliphatic group, a benzyl group, an aryl group or non-aromatic heterocyclic group;
R²⁴ and R²⁵ are independently -H, -OH, aliphatic group, substituted aliphatic group, benzyl group, an aryl group or non-aromatic heterocyclic group;
t is zero to three;
u is zero or one;
p is one to five;
Q is -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- or -NR²⁴S(O)₂-;
said aminoalkyl group is a C₁ to C₁₂ alkyl group substituted with -NR²⁴R²⁵;
said aliphatic group is a saturated or unsaturated C₁-C₂₀ hydrocarbon;
said electron withdrawing group is alkylimino, alkylsulfonyl, carboxamido, carboxylic alkyl ester, -CH=NH or -NO₂;
said acyl group is aliphatic carbonyl, aromatic carbonyl, aliphatic sulfonyl or aromatic sulfonyl;
said substituted acyl group is substituted aliphatic carbonyl, substituted aromatic carbonyl, substituted aliphatic sulfonyl or substituted aromatic sulfonyl;
the heterocycle of said non-aromatic heterocyclic group is a 5 to 8 membered non-aromatic ring that contains one or more heteroatoms selected from the group consisting of O, N, and S; and
said aromatic group is selected from the group consisting of a C₆ aromatic carbocycle, a C₅ - C₆ aromatic heterocycle comprising one or more heteroatoms selected from the group consisting of O, S and N, and a C₆ aromatic carbocycle or a C₅ - C₆ aromatic heterocycle that is fused to one or more other rings,
or a physiologically acceptable salt thereof.

26. A compound according to claim 24 wherein Z is: 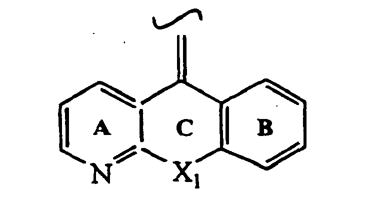 wherein:
X₁ is -CH₂-S-, -S-CH₂-, -O-CH₂-, -CH₂-O-, -SO-CH₂-, -CH₂-SO-, -S(O)₂-CH₂-, -CH₂-S(O)₂- or a bond; and
ring A and ring B are independently substituted or unsubstituted,

27. A compound according to any one of claims 6-23, 25 and 26 wherein ring B is substituted para to the carbon atom of ring B that is bonded to X₁ in ring C, such that Z is: 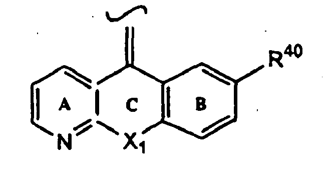 wherein:
R⁴⁰ is -OH, -COOH, -NO₂, halogen, aliphatic group, substituted aliphatic group, an aromatic group, a substituted aromatic group, -NR²⁴R²⁵, -CONR²⁴R²⁵, Q-(aliphatic group), Q-(substituted aliphatic group), -O-(aliphatic group), -O-(substituted aliphatic group), -O-(aromatic group), -O-(substituted aromatic group), an electron withdrawing group, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²² or -(O)ᵤ-(CH₂)ₜ-NHC(O)O-R²⁰;
R²⁰, R²¹ or R²² are independently -H, an aliphatic group, a substituted aliphatic group, an aromatic group, a substituted aromatic group or a non-aromatic heterocyclic group; or
R²¹ and R²², taken together with the nitrogen atom to which they are bonded form a non-aromatic heterocyclic ring;
Q is -NR²⁴C(O)-, -NR²⁴S(O)₂- or -C(O)O-;
R²⁴ and R²⁵ are independently -H, -OH, an aliphatic group or a substituted aliphatic group;
u is zero or one; and
t is an integer from zero to 3.

28. A compound according to one of claims 6-23, 25 and 26 wherein ring B is substituted para to the carbon atom of ring B that is bonded to X₁ in ring C, such that Z is: 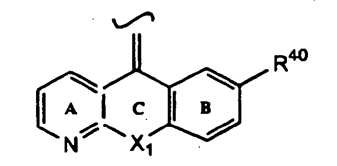 wherein:
R⁴⁰ is -C(=NR⁶⁰)NR²¹R²², -O-C(O)-NR²¹R²⁶, -S(O)₂-NR²¹R²² or -NH-C(O)-NR²¹R²²; wherein
R²¹ and R²² are independently -H, an aliphatic group, a substituted aliphatic group, an aromatic group, a substituted aromatic group or a non-aromatic heterocyclic group; or
R²¹ and R²², taken together with the nitrogen atom to which they are bonded, form a substituted or unsubstituted non-aromatic heterocyclic ring;
R²⁶ is -H, an aliphatic group, a substituted aliphatic group, an aromatic group, a substituted aromatic group, a non-aromatic heterocyclic group, -C(O)-O-(substituted or unsubstituted aliphatic group), -C(O)-O-(substituted or unsubstituted aromatic group), -S(O)₂-(substituted or unsubstituted aliphatic group), -S(O)₂-(substituted or unsubstituted aromatic group); or
R²⁶ and R²¹, taken together with the nitrogen atom to which they are bonded, form a substituted or unsubstituted non-aromatic heterocyclic ring.

29. A compound according to any one of claims 1-28 wherein X₁ is -CH₂-O-

30. A compound which is a tertiary amine of the following formula: 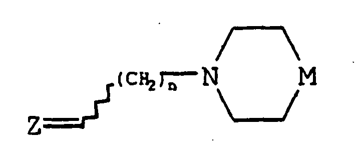 wherein:
M is CR¹R²;
R¹ is -OH;
R² is 4-chlorophenyl;
n is two;
Z is: 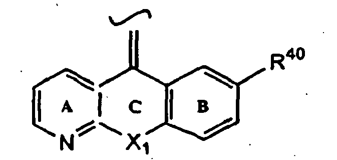
X₁ is -CH₂-O-; and
R⁴⁰ is 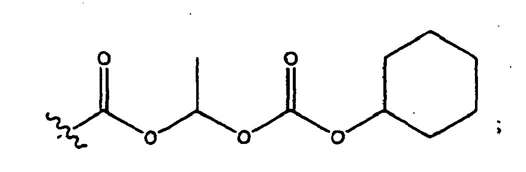
or a physiologically acceptable salt thereof.

31. A compound according to any one of claims 6 to 10 wherein R' is a substituted linear, branched or cyclic C₁-C₂₀ alkyl, alkanyl or alkynyl group.

32. A compound according to any one of claims 1 to 31 for use in a method for treatment of the human or animal body by therapy.

33. A compound as claimed in claim 32 for use in the treatment of a disease associated with aberrant leukocyte recruitment and/or activation.

34. A compound as claimed in claim 32 for use in the treatment of a chronic inflammatory disease.

35. A compound as claimed in claim 32 for use in the treatment of rheumatoid arthritis.

36. A compound as claimed in claim 32 for use in the treatment of multiple sclerosis.

37. Use of a compound defined in any one of claims 1 to 31 in the manufacture of a medicament for the treatment of a disease associated with aberrant leukocyte recruitment and/or activation.

38. Use of a compound defined in any one of claims 1 to 31 in the manufacture of a medicament for the treatment of a chronic inflammatory disease.

39. Use of a compound defined in any one of claims 1 to 31 in the manufacture of a medicament for the treatment of rheumatoid arthritis.

40. Use of a compound defined in any one of claims 1 to 31 in the manufacture of a medicament for the treatment of multiple sclerosis.

## Patentansprüche

1. Verbindung, die ein tertiäres Amin der folgenden Formel ist: 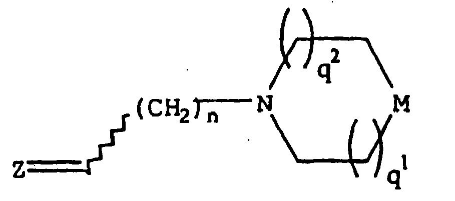 worin bedeuten:
n eins bis vier;
M >NR², >CR¹R² -O-CR¹R², -O-CR¹R²-O- oder-CH₂-CR¹R²-O-;
q¹ Null bis drei
q² Null oder eins;
R¹ -H, -OH, -N₃, ein Halogen, eine aliphatische Gruppe, eine substituierte aliphatische Gruppe, eine Aminoalkylgruppe, -O-(aliphatische Gruppe), -O-(substituierte aliphatische Gruppe), -SH, -S-(aliphatische Gruppe), -S-(substituierte aliphatische Gruppe), -OC(O)-(aliphatische Gruppe), -O-C(O)-(substituierte aliphatische Gruppe), -OC(O)-(aliphatische Gruppe), -O-C(O)-(substituierte aliphatische Gruppe), -COOH, -CN, -CO-NR³R⁴, -NR³R⁴, oder R¹ ist eine kovalente Bindung zwischen dem Ringatom bei M und einem benachbarten Kohlenstoffatom im Ring, der M enthält;
R² -OH, eine Acylgruppe, eine substituierte Acylgruppe, -NR⁵R⁶, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe, eine substituierte nicht-aromatische heterocyclische Gruppe, -O-(substituierte oder unsubstituierte aromatische Gruppe) oder -O-(substituierte oder unsubstituierte aliphatische Gruppe);
R³, R⁴, R⁵ und R⁶ unabhängig von einander -H, eine Acylgruppe, eine substituierte Acylgruppe, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe oder eine substituierte nicht-aromatische heterocyclische Gruppe; oder
R¹ und R², R³ und R⁴ oder R⁵ und R⁶ bilden zusammen mit dem Atom, an das sie gebunden sind, einen substituierten oder unsubstituierten nicht-aromatischen carbocyclischen heterocyclischen Ring;
Z: 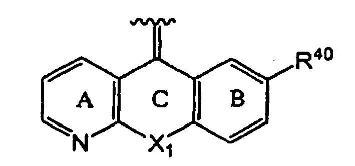
worin bedeuten:
X₁ -CH₂-S-, -S-CH₂-, -O-CH₂-, -CH₂-O-, -SO-CH₂-, -CH₂-SO-, -S(O)₂-CH₂-, -CH₂-S(O)₂- oder eine Bindung;
R⁴⁰ -S(O₂)-NR²¹R²², -NH-C(O)-NR²¹R²², -O-C(O)-NR²¹R²⁶, 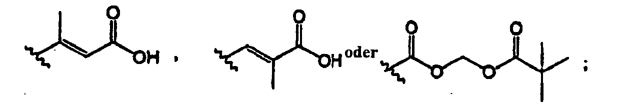
worin:
die substituierte aliphatische Gruppe einen oder mehrere Substituenten aufweist, ausgewählt aus der Gruppe bestehend aus einer Elektronen-anziehenden Gruppe, Halogen, Azido, -COOH, -OH, -CONR²⁴R²⁵, -NR²⁴R²⁵, -OS(O)₂NR²⁴R²⁵, -S(O)₂NR²⁴-R²⁵, -SO₃H, -S(O)₂NH₂, Guanidino, -(O)ᵤ-(CH₂)ₜ-C(O)OR²⁰, -(O)ᵤ-(CH₂)ₜOC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituirte aromatische Gruppe), -Q-(nicht-aromatische heterocyclische Gruppe), -Q-(CH₂)ₚ-(nicht-aromatische heterocyclische Gruppe), Oxo, Epoxy, nicht-aromatischer Heterocyclus, Benzyl, substituiertes Benzyl, aromatische Gruppe und substituierte aromatische Gruppe; wobei die substituierte Benzylgruppe und die substituierte aromatische Gruppe ein oder mehrere Substituenten aufweist, ausgewählt aus der Gruppe bestehend aus einer Elektronen-anziehenden Gruppe, Halogen, Azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituirte aromatische Gruppe), -Q-(nicht-aromatische heterocyclische Gruppe), -Q-(CH₂)ₚ-(nicht-aromatische heterocyclische Gruppe), aliphatische Gruppe und substituierte aliphatische Gruppe;
die substituierte nicht-aromatische heterocyclische Gruppe eines oder mehrere Substituenten aufweist, ausgewählt aus der Gruppe bestehend aus einer Elektronen-anziehenden Gruppe, Halogen, Azido, -COOH, -OH, -CONR²⁴R²⁵, -NR²⁴R²⁵, -OS(O)₂-NR²⁴R²⁵, -S(O)₂NR²⁴-R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜC(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituirte aromatische Gruppe), -Q-(nicht-aromatische heterocyclische Gruppe), -Q-(CH₂)ₚ-(nicht-aromatische heterocyclische Gruppe), aliphatische Gruppe, substituierte aliphatische Gruppe, =O, =S, =NH, =N(aliphatisch), =N(aromatisch) und =N(substituiert aromatisch);
R²⁰, R²¹ und R²² unabhängig von einander -H, eine aliphatische Gruppe, eine substituierte aliphatische Gruppe, eine aromatische Gruppe, eine substituierte aromatische Gruppe, eine nicht-aromatische heterocyclische Gruppe, -NHC(O)-O-(aliphatische Gruppe), -NHC(O)-O-(aromatische Gruppe) oder -NHC(O)-O-(nicht-aromatische heterocyclische Gruppe) sind;
R²⁶ -H, aliphatische Gruppe, substituierte aliphatische Gruppe, aromatische Gruppe, substituierte aromatische Gruppe, nicht-aromatische heterocyclische Gruppe, -C(O)-O-(substituierte oder unsubstituierte aliphatische Gruppe), -C(O)-O-(substituierte oder unsubstituierte aromatische Gruppe), -S(O)₂-(substituierte oder unsubstituierte aliphatische Gruppe), -S(O)₂-substituierte oder unsubstituierte aromatische Gruppe ist; oder
R²¹ und R²² zusammen mit dem Stickstoffatom, an das sie gebunden sind, einen nicht-aromatischen heterocyclischen Ring bilden; oder
R²¹ zusammen mit R²⁶ und dem Stickstoffatom, an das sie gebunden sind, einen substituierten oder unsubstituierten nicht-aromatischen heterocyclischen Ring bilden;
R²³ -H, eine aliphatische Gruppe, eine Benzylgruppe, eine Arylgruppe oder nicht-aromatische heterocyclische Gruppe ist;
R²⁴ und R²⁵ unabhängig von einander -H, -OH, eine aliphatische Gruppe, eine substituierte aliphatische Gruppe, eine Benzylgruppe, eine Arylgruppe oder eine nicht-aromatische heterocyclische Gruppe sind;
t Null bis drei ist;
u Null oder eins ist;
p eins bis fünf ist;
Q -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- oder NR²⁴S(O)₂- ist;
die Aminoalkylgruppe eine mit -NR²⁴R²⁵ substituierte C₁-C₁₂-Alkylgruppe ist;
die aliphatische Gruppe ein gesättigter oder ungesättigter C₁-C₂₀-Kohlenwasserstoff ist;
die Elektronen-anziehende Gruppe Alkylimino, Alkylsulfonyl, Carboxamido, Carbonsäurealkylester, -CH=NH oder NO₂ ist;
die Acylgruppe aliphatisches Carbonyl, aromatisches Carbonyl, aliphatisches Sulfonyl oder aromatisches Sulfonyl ist;
die substituierte Acylgruppe substituiertes aliphatisches Carbonyl, substituiertes aromatisches Carbonyl, substituiertes aliphatisches Sulfonyl oder substituiertes aromatisches Sulfonyl ist;
der Heterocyclus der nicht-aromatischen heterocyclischen Gruppe ein 5- bis 8-gliedriger nicht-aromatischer Ring ist, der ein oder mehrere Heteroatome enthält, ausgewählt aus der Gruppe bestehend aus O, N und S; und
die aromatische Gruppe ausgewählt ist aus der Gruppe bestehend aus einem C₆-aromatischen Carbocyclus, einem C₅-C₆-aromatische Heterocyclus, der ein oder mehrere Heteroatome aufweist, ausgewählt aus der Gruppe bestehend aus O, S und N, und einem C₆-aromatischen Carbocyclus oder einem C₅-C₆-aromatischen Heterocyclus, der mit einem oder mehreren Ringen kondensiert ist;
oder ein physiologisch annehmbares Salz davon.

2. Verbindung nach Anspruch 1, worin q¹ und q² jeweils eins sind.

3. Verbindung nach Anspruch 2, worin M >CR¹R² ist.

4. Verbindung nach Anspruch 3, worin R¹ -OH ist, und R² eine substituierte aromatische Gruppe ist.

5. Verbindung nach Anspruch 4, worin R² 4-Chlorphenyl ist.

6. Verbindung, die ein tertiäres Amin der folgenden Formel ist: 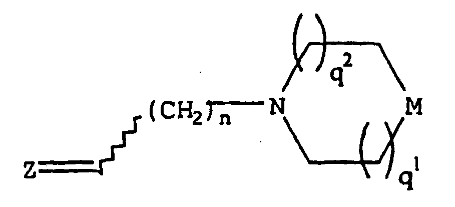 worin bedeuten:
n eins bis vier;
M >CR¹R², -O-CR¹R²-O- oder -CH₂-CR¹R²-O-;
q¹ Null bis drei
q² Null oder eins;
R¹ eine substituierte aliphatische Gruppe oder eine Aminoalkylgruppe;
R² -OH, eine Acylgruppe, eine substituierte Acylgruppe, -NR⁵R⁶, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe, eine substituierte nicht-aromatische heterocyclische Gruppe, -O-(substituierte oder unsubstituierte aromatische Gruppe) oder -O-(substituierte oder unsubstituierte aliphatische Gruppe);
R⁵ und R⁶ unabhängig von einander -H, eine Acylgruppe, eine substituierte Acylgruppe, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe oder eine substituierte nicht-aromatische heterocyclische Gruppe; oder
R⁵ und R⁶ zusammen mit dem Atom, an das sie gebunden sind, einen substituierten oder unsubstituierten nicht-aromatischen carbocyclischen oder heterocyclischen Ring bilden;
Z: 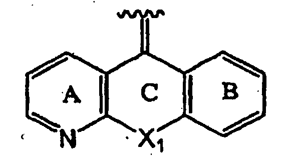
worin bedeuten:
X₁ -CH₂-S-, -S-CH₂-, -O-CH₂-, -CH₂-O-, -SO-CH₂-, -CH₂-SO-, -S(O)₂-CH₂-, -CH₂-S(O)₂- oder eine Bindung;
Ring A und Ring B unabhängig von einander substituiert oder unsubstituiert sind,
worin :
die substituierte aliphatische Gruppe einen oder mehrere Substituenten aufweist, ausgewählt aus der Gruppe bestehend aus einer Elektronen-anziehenden Gruppe, Halogen, Azido, -COOH, -OH, -CONR²⁴R²⁵, -NR²⁴R²⁵, -OS(O)₂-NR²⁴R²⁵, -S(O)₂NR²⁴-R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), Oxo, Epoxy, nicht-aromatischer Heterocyclus, Benzyl, substituiertes Benzyl, aromatische Gruppe und substituierte aromatische Gruppe;
die substituierte Benzylgruppe und die substituierte aromatische Gruppe und die Ringe A und B, wenn substituiert, einen oder mehrere Substituenten aufweisen, ausgewählt aus der Gruppe bestehend aus einer Elektronen-anziehenden Gruppe, Halogen, Azido, CN, -COOH, -OH, -CONR²⁴R²⁵, -NR²⁴R²⁵, -OS(O)₂-NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), aliphatische Gruppe, substituierte aliphatische Gruppe;
die substituierte nicht-aromatische heterocyclische Gruppe einen oder mehrere Substituenten aufweist, ausgewählt aus der Gruppe bestehend aus einer Elektronen-anziehenden Gruppe, Halogen, Azido, CN, -COOH, -OH, -CONR²⁴R²⁵, -NR²⁴R²⁵, -OS(O)₂-NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), aliphatische Gruppe, substituierte aliphatische Gruppe, =O, =S, =NH, =N(aliphatisch), =N(aromatisch) und =N(substituiert aromatisch);
R²⁰, R²¹ und R²² unabhängig von einander -H, eine aliphatische Gruppe, eine substituierte aliphatische Gruppe, eine aromatische Gruppe, eine substituierte aromatische Gruppe, eine nicht-aromatische heterocyclische Gruppe, -NHC(O)-O-(aliphatische Gruppe), -NHC(O)-O-(aromatische Gruppe) oder -NHC(O)-O-(nicht-aromatische heterocyclische Gruppe) sind; oder
R²¹ und R²² zusammen mit dem Stickstoffatom, an das sie gebunden sind, einen nicht-aromatischen heterocyclischen Ring bilden,
R²³ -H, eine aliphatische Gruppe, eine Benzylgruppe, eine Arylgruppe oder nicht-aromatische heterocyclische Gruppe ist;
R²⁴ und R²⁵ unabhängig von einander -H, -OH, eine aliphatische Gruppe, substituierte aliphatische Gruppe, Benzylgruppe, eine Arylgruppe oder eine nicht-aromatische heterocyclische Gruppe sind;
t Null bis drei ist;
u Null oder eins ist;
p eins bis fünf ist;
Q -O-, -S-, -S(O)-, -S(O)₂-, -OS(O)₂-, -C(O)-,-OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- oder -NR²⁴S(O)₂- ist;
die Aminoalkylgruppe eine mit -NR²⁴R²⁵ substituierte C₁-C₁₂-Alkylgruppe ist;
die aliphatische Gruppe ein gesättigter oder ungesättigter C₁-C₂₀-Kohlenwasserstoff ist;
die Elektronen-anziehende Gruppe Alkylamino, Alkylsulfonyl, Carboxamido, Carbonsäurealkylester, -CH=NH oder -NO₂ ist;
die Acylgruppe ein aliphatisches Carbonyl, aromatisches Carbonyl, aliphatische Sulfonyl oder aromatisches Sulfonyl ist;
die substituierte Acylgruppe substituiertes aliphatisches Carbonyl, substituiertes aromatisches Carbonyl, substituiertes aliphatisches Sulfonyl oder substituiertes aromatisches Sulfonyl;
der Heterocyclus der nicht-aromatischen heterocyclischen Gruppe ein 5- bis 8-gliedriger nicht-aromatischer Ring ist, der ein oder mehrere Heteroatome enthält, ausgewählt aus der Gruppe bestehend aus O, N und S; und
die aromatische Gruppe ausgewählt ist aus der Gruppe bestehend aus einem C₆-aromatischer Carbocyclus, einem C₅-C₆-aromatische Heterocyclus, der ein oder mehrere Heteroatome aufweist, ausgewählt aus der Gruppe bestehend aus O, S und N, und einem C₆-aromatischen Carbocyclus oder einem C₅-C₆-aromatischen Heterocyclus, der mit einem oder mehreren Ringen kondensiert ist;
oder ein physiologisch annehmbares Salz davon;
verschieden von:

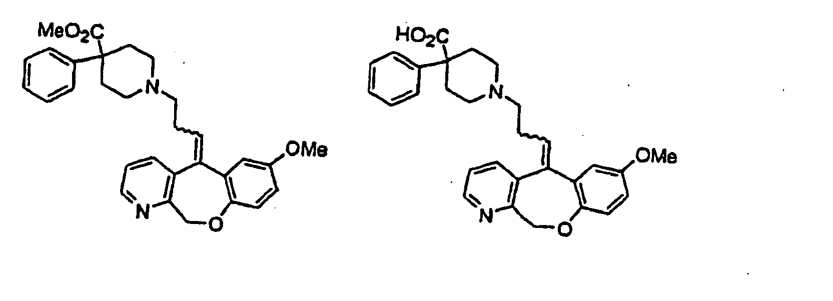

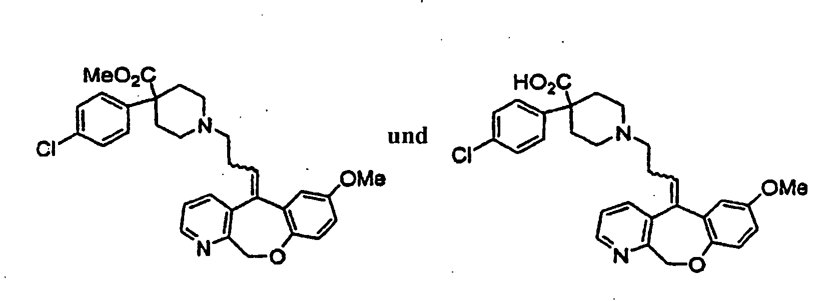

7. Verbindung nach Anspruch 6, worin q¹ und q² jeweils eins sind.

8. Verbindung nach Anspruch 7, worin M >CR¹R² ist.

9. Verbindung nach Anspruch 8, worin R² eine substituierte aromatische Gruppe ist.

10. Verbindung nach Anspruch 9, worin R² 4-Chlorphenyl ist.

11. Verbindung nach einem der Ansprüche 6 bis 10, worin R¹ eine Alkylaminogruppe ist, ausgewählt aus der Gruppe bestehend aus Aminomethyl, 2-Aminoethyl, 3-Aminopropyl, 4-Aminobutyl, Dimethylaminoethyl, Diethylaminomethyl, Methylaminohexyl und Aminoethylenyl.

12. Verbindung, die ein tertiäres Amin der folgenden Formel ist: 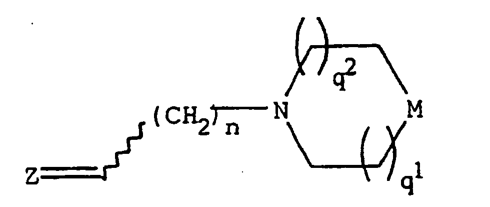 worin bedeuten:
n eins bis vier;
M >NR², >CR¹R², -O-CR¹R²-O- oder -CH₂-CR¹R²-O-;
q¹ Null bis drei;
q² Null oder eins;
R¹ -H, -OH, -N₃, ein Halogen, eine aliphatische Gruppe, eine substituierte aliphatische Gruppe, eine Aminoalkylgruppe, -O-(aliphatische Gruppe), -O-(substituierte aliphatische Gruppe), -SH, -S-(aliphatische Gruppe), -S-(substituierte aliphatische Gruppe), -OC(O)-(aliphatische Gruppe), -O-C(O)-(substituierte aliphatische Gruppe), -C(O)O-(aliphatische Gruppe), -C(O)O-(substituierte aliphatische Gruppe), -COOH, -CN, -CO-NR³R⁴, -NR³R⁴, oder R¹ ist eine kovalente Bindung zwischen dem Ringatom bei M und einem benachbarten Kohlenstoffatom im Ring, der M enthält;
R² -O-(substituierte oder unsubstituierte aromatische Gruppe);
R³ und R⁴ unabhängig von einander -H, eine Acylgruppe, eine substituierte Acylgruppe, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe, eine substituierte nicht-aromatische heterocyclische Gruppe; oder
R³ und R⁴ zusammen mit dem Atom, an das sie gebunden sind, einen substituierten oder unsubstituierten nicht-aromatischen carbocyclischen oder heterocyclischen Ring bilden;
Z: 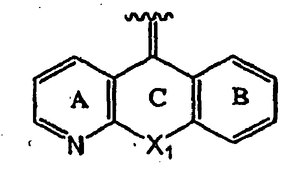
worin bedeuten:
X₁ -CH₂-S-, -S-CH₂-, -O-CH₂-, -CH₂-O-, -SO-CH₂-, -CH₂-SO-, -S(O)₂-CH₂-, -CH₂-S(O)₂- oder eine Bindung;
Ring A und Ring B unabhängig von einander substituiert oder unsubstituiert sind,
worin:
die substituierte aliphatische Gruppe einen oder mehrere Substituenten aufweist, ausgewählt aus der Gruppe bestehend aus einer Elektronen-anziehenden Gruppe, Halogen, Azido, -COOH, -OH, -CONR²⁴R²⁵, -NR²⁴R²⁵, -OS(O)₂NR²⁴R²⁵, -S(O)₂NR²⁴-R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituirte aromatische Gruppe), -Q-(nicht-aromatische heterocyclische Gruppe), -Q-(CH₂)ₚ-(nicht-aromatische heterocyclische Gruppe), Oxo, Epoxy, nicht-aromatischer Heterocyclus, Benzyl, substituiertes Benzyl, aromatische Gruppe und substituierte aromatische Gruppe; wobei die substituierte Benzylgruppe und die substituierte aromatische Gruppe und die Ringe A und B, wenn substituiert, einen oder mehrere Substituenten aufweist, ausgewählt aus der Gruppe bestehend aus einer Elektronen-anziehenden Gruppe, Halogen, Azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituirte aromatische Gruppe), -Q-(nicht-aromatische heterocyclische Gruppe), -Q-(CH₂)ₚ-(nicht-aromatische heterocyclische Gruppe), aliphatische Gruppe und substituierte aliphatische Gruppe;
die substituierte nicht-aromatische heterocyclische Gruppe einen oder mehrere Substituenten aufweist, ausgewählt aus der Gruppe bestehend aus einer Elektronen-anziehenden Gruppe, Halogen, Azido, -CN -COOH, -OH, -CONR²⁴R²⁵, -NR²⁴R²⁵, -OS(O)₂- NR²⁴R²⁵, -S(O)₂NR²⁴-R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²¹, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituirte aromatische Gruppe), -Q-(nicht-aromatische heterocyclische Gruppe), -Q-(CH₂)ₚ-(nicht-aromatische heterocyclische Gruppe), aliphatische Gruppe, substituierte aliphatische Gruppe, =O, =S, =NH, =N(aliphatisch), =N(aromatisch) und =N(substituiert aromatisch);
R²⁰, R²¹ und R²² unabhängig von einander -H, eine aliphatische Gruppe, eine substituierte aliphatische Gruppe, eine aromatische Gruppe, eine substituierte aromatische Gruppe, eine nicht-aromatische heterocyclische Gruppe, -NHC(O)-O-(aliphatische Gruppe), -NHC(O)-O-(aromatische Gruppe oder -NHC(O)-O-(nicht-aromatische heterocyclische Gruppe) sind;
R²¹ und R²² zusammen mit dem Stickstoffatom, an das sie gebunden sind, einen nicht-aromatischen heterocyclischen Ring bilden; oder
R²³ -H, eine aliphatische Gruppe, eine Benzylgruppe, eine Arylgruppe oder nicht-aromatische heterocyclische Gruppe ist;
R²⁴ und R²⁵ unabhängig von einander -H, -OH, eine aliphatische Gruppe, eine substituierte aliphatische Gruppe, eine Benzylgruppe, eine Arylgruppe oder eine nicht-aromatische heterocyclische Gruppe sind;
t Null bis drei ist;
u Null oder eins ist;
p eins bis fünf ist;
Q -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- oder -NR²⁴S(O)₂- ist;
die Aminoalkylgruppe eine mit -NR²⁴R²⁵ substituierte C₁-C₁₂-Alkylgruppe ist;
die aliphatische Gruppe ein gesättigter oder ungesättigter C₁-C₂₀-Kohlenwasserstoff ist;
die Elektronen-anziehende Gruppe Alkylimino, Alkylsulfonyl, Carboxamido, Carbonsäurealkylester, -CH=NH oder -NO₂ ist;
die Acylgruppe aliphatisches Carbonyl, aromatisches Carbonyl, aliphatisches Sulfonyl oder aromatisches Sulfonyl ist;
die substituierte Acylgruppe substituiertes aliphatisches Carbonyl, substituiertes aromatisches Carbonyl, substituiertes aliphatisches Sulfonyl oder substituiertes aromatisches Sulfonyl ist;
der Heterocyclus der nicht-aromatischen heterocyclischen Gruppe ein 5- bis 8-gliedriger nicht-aromatischer Ring ist, der ein oder mehrere Heteroatome enthält, ausgewählt aus der Gruppe bestehend aus O, N und S; und
die aromatische Gruppe ausgewählt ist aus der Gruppe bestehend aus einem C₆-aromatischen Carbocyclus, einem C₅-C₆-aromatischen Heterocyclus, der ein oder mehrere Heteroatome aufweist, ausgewählt aus der Gruppe bestehend aus O, S und N, und einem C₆-aromatischen Carbocyclus oder einem C₅-C₆-aromatischen Heterocyclus, der mit einem oder mehreren Ringen kondensiert ist;
oder ein physiologisch annehmbares Salz davon.

13. Verbindung nach Anspruch 12, worin q¹ und q² jeweils eins sind.

14. Verbindung nach Anspruch 12, worin M >CR¹R² ist.

15. Verbindung nach Anspruch 8, worin R¹ -OH ist.

16. Verbindung die ein tertiäres Amin der folgenden Formel ist: 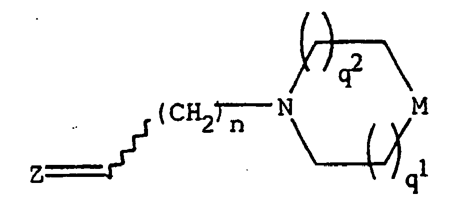 worin bedeuten:
n eins bis vier;
M -O-CR¹R²-O- oder -CH₂-CR¹R²-O-;
q¹ Null bis drei;
q² Null oder eins;
R¹ -H, -OH, -N₃, ein Halogen, eine aliphatische Gruppe, eine substituierte aliphatische Gruppe, eine Aminoalkylgruppe, -O-(aliphatische Gruppe), -O-(substituierte aliphatische Gruppe), -SH, -S-(aliphatische Gruppe), -S-(substituierte aliphatische Gruppe), -OC(O)-(aliphatische Gruppe), -O-C(O)-(substituierte aliphatische Gruppe), -C(O)O-(aliphatische Gruppe), -C(O)O-(substituierte aliphatische Gruppe), -COOH, -CN, -CO-NR³R⁴, -NR³R⁴, oder R¹ ist eine kovalente Bindung zwischen dem Ringatom bei M und einem benachbarten Kohlenstoffatom im Ring, der M enthält;
R² -OH, eine Acylgruppe, eine substituierte Acylgruppe, -NR⁵R⁶, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe, eine substituierte nicht-aromatische heterocyclische Gruppe; -O-(substituierte oder unsubstituierte aromatische Gruppe) oder -O-(substituierte oder unsubstituierte aliphatische Gruppe);
R³, R⁴, R⁵ und R⁶ unabhängig von einander -H, eine Acylgruppe, eine substituierte Acylgruppe, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe, eine substituierte nicht-aromatische heterocyclische Gruppe; oder
R¹ und R², R³ und R⁴ oder R⁵ und R⁶ zusammen mit dem Atom, an das sie gebunden sind, einen substituierten oder unsubstituierten nicht-aromatischen carbocyclischen heterocyclischen Ring bilden;
Z: 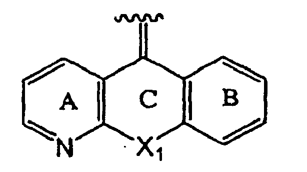
worin bedeuten:
X₁ -CH₂-S-, -S-CH₂-, -O-CH₂-, -CH₂-O-, -SO-CH₂-, -CH₂-SO-, -S(O)₂-CH₂-, -CH₂-S(O)₂- oder eine Bindung;
Ring A und Ring B unabhängig von einander substituiert oder unsubstituiert sind,
worin:
die substituierte aliphatische Gruppe einen oder mehrere Substituenten aufweist, ausgewählt aus der Gruppe bestehend aus einer Elektronen-anziehenden Gruppe, Halogen, Azido, -COOH, -OH, -CONR²⁴R²⁵ -NR²⁴R²⁵ -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), Oxo, Epoxy, nicht-aromatischer Heterocyclus, Benzyl, substituiertes Benzyl, aromatische Gruppe und substituierte aromatische Gruppe;
wobei die substituierte Benzylgruppe und die substituierte aromatische Gruppe einen oder mehrere Substituenten aufweist, ausgewählt aus der Gruppe bestehend aus einer Elektronen-anziehenden Gruppe, Halogen, Azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), aliphatische Gruppe und substituierte aliphatische Gruppe;
die substituierte nicht-aromatische heterocyclische Gruppe einen oder mehrere Substituenten aufweist, ausgewählt aus der Gruppe bestehend aus einer Elektronen-anziehenden Gruppe, Halogen, Azido, -CN, -COOH, -OH, -CONR²⁴R²⁵, -NR²⁴R²⁵, -OS(O)₂-NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituirte aromatische Gruppe), -Q-(nicht-aromatische heterocyclische Gruppe), -Q-(CH₂)ₚ-(nicht-aromatische heterocyclische Gruppe), aliphatische Gruppe, substituierte aliphatische Gruppe, =O, =S, =NH, =N(aliphatisch), =N(aromatisch) und =N(substituiert aromatisch);
R²⁰, R²¹ und R²² unabhängig von einander -H, eine aliphatische Gruppe, eine substituierte aliphatische Gruppe, eine aromatische Gruppe, eine substituierte aromatische Gruppe, eine nicht-aromatische heterocyclische Gruppe, -NHC(O)-O-(aliphatische Gruppe), -NHC(O)-O-(aromatische Gruppe oder NHC(O)-O-(nicht-aromatische heterocyclische Gruppe) sind; oder
R²¹ und R²² zusammen mit dem Stickstoffatom, an das sie gebunden sind, einen nicht-aromatischen heterocyclischen Ring bilden; oder
R²³ -H, eine aliphatische Gruppe, eine Benzylgruppe, eine Arylgruppe oder nicht-aromatische heterocyclische Gruppe ist;
R²⁴ und R²⁵ unabhängig von einander -H, -OH, eine aliphatische Gruppe, eine substituierte aliphatische Gruppe, eine Benzylgruppe, eine Arylgruppe oder eine nicht-aromatische heterocyclische Gruppe sind;
t Null bis drei ist;
u Null oder eins ist;
p eins bis fünf ist;
Q -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- oder -NR²⁴S(O)₂- ist;
die Aminoalkylgruppe eine mit -NR²⁴R²⁵ substituierte C₁-C₁₂-Alkylgruppe ist;
die aliphatische Gruppe ein gesättigter oder ungesättigter C₁-C₂₀-Kohlenwasserstoff ist;
die Elektronen-anziehende Gruppe Alkylimino, Alkylsulfonyl, Carboxamido, Carbonsäurealkylester, -CH=NH oder -NO₂ ist;
die Acylgruppe aliphatisches Carbonyl, aromatisches Carbonyl, aliphatisches Sulfonyl oder aromatisches Sulfonyl ist;
die substituierte Acylgruppe substituiertes aliphatisches Carbonyl, substituiertes aromatisches Carbonyl, substituiertes aliphatisches Sulfonyl oder substituiertes aromatisches Sulfonyl ist;
der Heterocyclus der nicht-aromatischen heterocyclischen Gruppe ein 5- bis 8-gliedriger nicht-aromatischer Ring ist, der ein oder mehrere Heteroatome enthält, ausgewählt aus der Gruppe bestehend aus O, N und S; und
die aromatische Gruppe ausgewählt ist aus der Gruppe bestehend aus einem C₆-aromatischen Carbocyclus, einem C₅-C₆-aromatischen Heterocyclus, der ein oder mehrere Heteroatome aufweist, ausgewählt aus der Gruppe bestehend aus O, S und N, und einem C₆-aromatischen Carbocyclus oder einem C₅-C₆-aromatischen Heterocyclus, der mit einem oder mehreren Ringen kondensiert ist;
oder ein physiologisch annehmbares Salz davon.

17. Verbindung nach Anspruch 16 worin q¹ und q² jeweils eins sind.

18. Verbindung nach Anspruch 17, worin R¹ -OH ist, und R² eine substituierte aromatische Gruppe ist.

19. Verbindung nach Anspruch 18, worin R² 4-Chlorphenyl ist.

20. Verbindung, die ein tertiäres Amin der folgenden Formel ist: 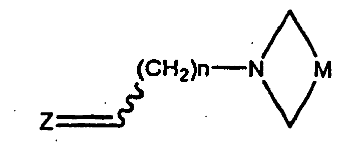 worin bedeuten:
n eins bis vier;
M >NR² oder >CR¹R²;
R¹ -H, -OH, -N₃, ein Halogen, eine aliphatische Gruppe, eine substituierte aliphatische Gruppe, eine Aminoalkylgruppe, -O-(aliphatische Gruppe), -O-(substituierte aliphatische Gruppe), -SH, -S-(aliphatische Gruppe), -S-(substituierte aliphatische Gruppe), -OC(O)-(aliphatische Gruppe), -O-C(O)-(substituierte aliphatische Gruppe), -C(O)O-(aliphatische Gruppe), -C(O)O-(substituierte aliphatische Gruppe), -COOH, -CN, -CO-NR³R⁴, -NR³R⁴, oder R¹ ist eine kovalente Bindung zwischen dem Ringatom bei M und einem benachbarten Kohlenstoffatom im Ring, der M enthält;
R² -OH, eine Acylgruppe, eine substituierte Acylgruppe, -NR⁵R⁶, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe, eine substituierte nicht-aromatische heterocyclische Gruppe; -O-(substituierte oder unsubstituierte aromatische Gruppe) oder -O-(substituierte oder unsubstituierte aliphatische Gruppe);
R³, R⁴, R⁵ und R⁶ unabhängig von einander -H, eine Acylgruppe, eine substituierte Acylgruppe, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe oder eine substituierte nicht-aromatische heterocyclische Gruppe; oder
R¹ und R², R³ und R⁴ oder R⁵ und R⁶ zusammen mit dem Atom, an das sie gebunden sind, einen substituierten oder unsubstituierten nicht-aromatischen carbocyclischen oder heterocyclischen Ring bilden;
Z: 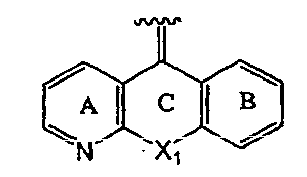
worin bedeuten:
X₁ -CH₂-S-, -S-CH₂-, -O-CH₂-, -CH₂-O-, -SO-CH₂-, -CH₂-SO-, -S(O)₂-CH₂-, -CH₂-S(O)₂- oder eine Bindung;
Ring A und Ring B unabhängig von einander substituiert oder unsubstituiert sind,
worin:
die substituierte aliphatische Gruppe einen oder mehrere Substituenten aufweist, ausgewählt aus der Gruppe bestehend aus einer Elektronen-anziehenden Gruppe, Halogen, Azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), Oxo, Epoxy, nicht-aromatischer Heterocyclus, Benzyl, substituiertes Benzyl, aromatische Gruppe und substituierte aromatische Gruppe;
wobei die substituierte Benzylgruppe und die substituierte aromatische Gruppe einen oder mehrere Substituenten aufweist, ausgewählt aus der Gruppe bestehend aus einer Elektronen-anziehenden Gruppe, Halogen, Azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), aliphatische Gruppe und substituierte aliphatische Gruppe;
die substituierte nicht-aromatische heterocyclische Gruppe einen oder mehrere Substituenten aufweist, ausgewählt aus der Gruppe bestehend aus einer Elektronen-anziehenden Gruppe, Halogen, Azido, -CN -COOH, -OH, -CONR²⁴R²⁵, -NR²⁴R²⁵, -OS(O)₂-NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), aliphatische Gruppe, substituierte aliphatische Gruppe, =O, =S, =NH, =N(aliphatisch), =N(aromatisch) und =N(substituiert aromatisch);
R²⁰, R²¹ und R²² unabhängig von einander -H, eine aliphatische Gruppe, eine substituierte aliphatische Gruppe, eine aromatische Gruppe, eine substituierte aromatische Gruppe, eine nicht-aromatische heterocyclische Gruppe, -NHC(O)-O-(aliphatische Gruppe), -NHC(O)-O-(aromatische Gruppe oder -NHC(O)-O-(nicht-aromatische heterocyclische Gruppe) sind; oder
R²¹ und R²² zusammen mit dem Stickstoffatom, an das sie gebunden sind, einen nicht-aromatischen heterocyclischen Ring bilden;
R²³ -H, eine aliphatische Gruppe, eine Benzylgruppe, eine Arylgruppe oder nicht-aromatische heterocyclische Gruppe ist;
R²⁴ und R²⁵ unabhängig von einander -H, -OH, eine aliphatische Gruppe, eine substituierte aliphatische Gruppe, eine Benzylgruppe, eine Arylgruppe oder eine nicht-aromatische heterocyclische Gruppe sind;
t Null bis drei ist;
u Null oder eins ist;
p eins bis fünf ist;
Q -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- oder NR²⁴S(O)₂- ist;
die Aminoalkylgruppe eine mit -NR²⁴R²⁵ substituierte C₁-C₁₂-Alkylgruppe ist;
die aliphatische Gruppe ein gesättigter oder ungesättigter C₁-C₂₀-Kohlenwasserstoff ist;
die Elektronen-anziehende Gruppe Alkylimino, Alkylsulfonyl, Carboxamido, Carbonsäurealkylester, -CH=NH oder -NO₂ ist;
die Acylgruppe aliphatisches Carbonyl, aromatisches Carbonyl, aliphatisches Sulfonyl oder aromatisches Sulfonyl ist;
die substituierte Acylgruppe substituiertes aliphatisches Carbonyl, substituiertes aromatisches Carbonyl, substituiertes aliphatisches Sulfonyl oder substituiertes aromatisches Sulfonyl ist;
der Heterocyclus der nicht-aromatischen heterocyclischen Gruppe ein 5- bis 8-gliedriger nicht-aromatischer Ring ist, der ein oder mehrere Heteroatome enthält, ausgewählt aus der Gruppe bestehend aus O, N und S; und
die aromatische Gruppe ausgewählt ist aus der Gruppe bestehend aus einem C₆-aromatischen Carbocyclus, einem C₅-C₆-aromatischen Heterocyclus, der ein oder mehrere Heteroatome aufweist, ausgewählt aus der Gruppe bestehend aus O, S und N, und einem C₆-aromatischen Carbocyclus oder einem C₅-C₆-aromatischen Heterocyclus, der mit einem oder mehreren Ringen kondensiert ist;
oder ein physiologisch annehmbares Salz davon.

21. Verbindung nach Anspruch 20, worin M >CR¹R² ist.

22. Verbindung nach Anspruch 21, worin R¹ -OH ist, und R² eine substituierte aromatische Gruppe ist.

23. Verbindung nach Anspruch 22, worin R² ein 4-Chlorphenyl ist.

24. Verbindung, die ein tertiäres Amin der folgenden Formel ist: 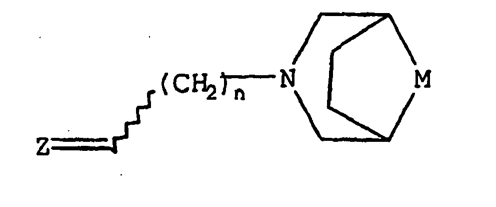 worin bedeuten:
n eins bis vier;
M >NR², >CR¹R², -O-CR¹R²-O- oder -CH₂-CR¹R²-O-;
der M enthaltende Ring substituiert oder unsubstituiert ist;
R¹ -H, -OH, -N₃, ein Halogen, eine aliphatische Gruppe, eine substituierte aliphatische Gruppe, eine Aminoalkylgruppe, -O-(aliphatische Gruppe), -O-(substituierte aliphatische Gruppe), -SH, -S-(aliphatische Gruppe), -S-(substituierte aliphatische Gruppe), -OC(O)-(aliphatische Gruppe), -O-C(O)-(substituierte aliphatische Gruppe), -C(O)O-(aliphatische Gruppe), -C(O)O-(substituierte aliphatische Gruppe), -COOH, -CN, -CO-NR³R⁴, -NR³R⁴, oder R¹ ist eine kovalente Bindung zwischen dem Ringatom bei M und einem benachbarten Kohlenstoffatom im Ring, der M enthält;
R² -OH, eine Acylgruppe, eine substituierte Acylgruppe, -NR⁵R⁶, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe, eine substituierte nicht-aromatische heterocyclische Gruppe; -O-(substituierte oder unsubstituierte aromatische Gruppe) oder -O-(substituierte oder unsubstituierte aliphatische Gruppe);
R³, R⁴, R⁵ und R⁶ unabhängig von einander -H, eine Acylgruppe, eine substituierte Acylgruppe, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe oder eine substituierte nicht-aromatische heterocyclische Gruppe; oder
R¹ und R², R³ und R⁴ oder R⁵ und R⁶ zusammen mit dem Atom, an das sie gebunden sind, einen substituierten oder unsubstituierten nicht-aromatischen carbocyclischen oder heterocyclischen Ring bilden;
Z: 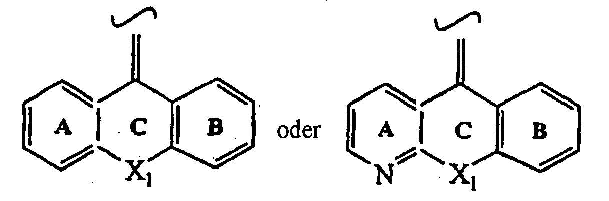
worin bedeuten:
X₁ -CH₂-S-, -S-CH₂-, -O-CH₂-, -CH₂-O-, -SO-CH₂-, -CH₂-SO-, -S(O)₂-CH₂-, -CH₂-S(O)₂- oder eine Bindung;
Ring A und Ring B unabhängig von einander substituiert oder unsubstituiert sind,
worin:
die substituierte aliphatische Gruppe einen oder mehrere Substituenten aufweist, ausgewählt aus der Gruppe bestehend aus einer Elektronen-anziehenden Gruppe, Halogen, Azido, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), Oxo, Epoxy, nicht-aromatischer Heterocyclus, Benzyl, substituiertes Benzyl, aromatische Gruppe und substituierte aromatische Gruppe;
wobei die substituierte Benzylgruppe und die substituierte aromatische Gruppe einen oder mehrere Substituenten aufweist, ausgewählt aus der Gruppe bestehend aus einer Elektronen-anziehenden Gruppe, Halogen, Azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), aliphatische Gruppe und substituierte aliphatische Gruppe;
die substituierte nicht-aromatische heterocyclische Gruppe einen oder mehrere Substituenten aufweist, ausgewählt aus der Gruppe bestehend aus einer Elektronen-anziehenden Gruppe, Halogen, Azido, -CN -COOH, -OH, -CONR²⁴R²⁵, -NR²⁴R²⁵, -OS(O)₂- NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituirte aromatische Gruppe), -Q-(nicht-aromatische heterocyclische Gruppe), -Q-(CH₂)ₚ-(nicht-aromatische heterocyclische Gruppe), aliphatische Gruppe, substituierte aliphatische Gruppe, =O, =S, =NH, =N(aliphatisch), =N(aromatisch) und =N(substituiert aromatisch);
R²¹, R²¹ und R²² unabhängig von einander -H, eine aliphatische Gruppe, eine substituierte aliphatische Gruppe, eine aromatische Gruppe, eine substituierte aromatische Gruppe, eine nicht-aromatische heterocyclische Gruppe, -NHC(O)-O-(aliphatische Gruppe), -NHC(O)-O-(aromatische Gruppe) oder NHC(O)-O-(nicht-aromatische heterocyclische Gruppe) sind; oder
R²¹ und R²² zusammen mit dem Stickstoffatom, an das sie gebunden sind, einen nicht-aromatischen heterocyclischen Ring bilden;
R²³ -H, eine aliphatische Gruppe, eine Benzylgruppe, eine Arylgruppe oder nicht-aromatische heterocyclische Gruppe ist;
R²⁴ und R²⁵ unabhängig von einander -H, -OH, eine aliphatische Gruppe, eine substituierte aliphatische Gruppe, eine Benzylgruppe, eine Arylgruppe oder eine nicht-aromatische heterocyclische Gruppe sind;
t Null bis drei ist;
u Null oder eins ist;
p eins bis fünf ist;
Q -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- oder NR²⁴S(O)₂- ist;
die Aminoalkylgruppe eine mit -NR²⁴R²⁵ substituierte C₁-C₁₂-Alkylgruppe ist;
die aliphatische Gruppe ein gesättigter oder ungesättigter C₁-C₂₀-Kohlenwasserstoff ist;
die Elektronen-anziehende Gruppe Alkylimino, Alkylsulfonyl, Carboxamido, Carbonsäurealkylester, -CH=NH oder -NO₂ ist;
die Acylgruppe aliphatisches Carbonyl, aromatisches Carbonyl, aliphatisches Sulfonyl oder aromatisches Sulfonyl ist;
die substituierte Acylgruppe substituiertes aliphatisches Carbonyl, substituiertes aromatisches Carbonyl, substituiertes aliphatisches Sulfonyl oder substituiertes aromatisches Sulfonyl ist;
der Heterocyclus der nicht-aromatischen heterocyclischen Gruppe ein 5- bis 8-gliedriger nicht-aromatischer Ring ist, der ein oder mehrere Heteroatome enthält, ausgewählt aus der Gruppe bestehend aus O, N und S; und
die aromatische Gruppe ausgewählt ist aus der Gruppe bestehend aus einem C₆-aromatischen Carbocyclus, einem C₅-C₆-aromatischen Heterocyclus, der ein oder mehrere Heteroatome aufweist, ausgewählt aus der Gruppe bestehend aus O, S und N, und einem C₆-aromatischen Carbocyclus oder einem C₅-C₆-aromatischen Heterocyclus, der mit einem oder mehreren Ringen kondensiert ist;
oder ein physiologisch annehmbares Salz davon.

25. Verbindung, die ein tertiäres Amin der folgenden Formel ist: 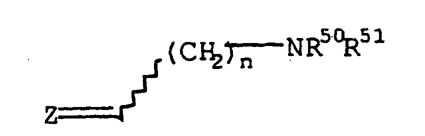 worin bedeuten:
n eins bis vier;
R⁵⁰ und R⁵¹ jeweils unabhängig von einander -H, eine aliphatische Gruppe, eine substituierte aliphatische Gruppe, eine Aminoalkylgruppe, -NR³R⁴, eine aromatische Gruppe, eine substituierte aromatische Gruppe, eine Benzylgruppe, eine substituierte Benzylgruppe, eine nicht-aromatische heterocyclische Gruppe, eine substituierte nicht-aromatische heterocyclische Gruppe oder eine kovalente Bindung zwischen dem Stickstoffatom und einem benachbarten Kohlenstoffatom;
R³ und R⁴ unabhängig von einander -H, eine Acylgruppe, eine substituierte Acylgruppe, eine aliphatische Gruppe, eine substituierte aliphatische Gruppe, eine aromatische Gruppe, eine substituierte aromatische Gruppe, eine Benzylgruppe, eine substituierte Benzylgruppe, eine nicht-aromatische heterocyclische Gruppe oder eine substituierte nicht-aromatische heterocyclische Gruppe;
Z: 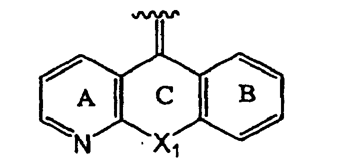
worin bedeuten:
X₁ -CH₂-S-, -S-CH₂-, -O-CH₂-, -CH₂-O-, -SO-CH₂-, -CH₂-SO-, -S(O)₂-CH₂-, -CH₂-S(O)₂- oder eine Bindung;
Ring A und Ring B unabhängig von einander substituiert oder unsubstituiert sind,
worin:
die substituierte aliphatische Gruppe einen oder mehrere Substituenten aufweist, ausgewählt aus der Gruppe bestehend aus einer Elektronen-anziehenden Gruppe, Halogen, Azido, -COOH, -OH, -CONR²⁴R²⁵ -NR²⁴R²⁵ -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), Oxo, Epoxy, nicht-aromatischer Heterocyclus, Benzyl, substituiertes Benzyl, aromatische Gruppe und substituierte aromatische Gruppe;
wobei die substituierte Benzylgruppe und die substituierte aromatische Gruppe und die Ringe A und B, wenn substituiert, einen oder mehrere Substituenten aufweisen, ausgewählt aus der Gruppe bestehend aus einer Elektronen-anziehenden Gruppe, Halogen, Azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituierte aromatische Gruppe), -Q-(nicht-aromatische heterocyclische Gruppe), -Q-(CH₂)ₚ-(nicht-aromatische heterocyclische Gruppe), aliphatische Gruppe und substituierte aliphatische Gruppe;
die substituierte nicht-aromatische heterocyclische Gruppe einen oder mehrere Substituenten aufweist, ausgewählt aus der Gruppe bestehend aus einer Elektronen-anziehenden Gruppe, Halogen, Azido, -CN -COOH, -OH, -CONR²⁴R²⁵, -NR²⁴R²⁵, -OS(O)₂- NR²⁴R²⁵, -S(O)₂NR²⁴R²⁵, -SO₃H, -S(O)₂NH₂, Guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)O-R²⁰, -Q-H, -Q-(aliphatische Gruppe), -Q-(substituierte aliphatische Gruppe), -Q-(Aryl), -Q-(aromatische Gruppe), -Q-(substituierte aromatische Gruppe), -Q-(CH₂)ₚ-(substituierte oder unsubstituirte aromatische Gruppe), -Q-(nicht-aromatische heterocyclische Gruppe), -Q-(CH₂)ₚ-(nicht-aromatische heterocyclische Gruppe), aliphatische Gruppe, substituierte aliphatische Gruppe, =O, =S, =NH, =N(aliphatisch), =N(aromatisch) und =N(substituiert aromatisch);
R²⁰, R²¹ und R²² unabhängig von einander -H, eine aliphatische Gruppe, eine substituierte aliphatische Gruppe, eine aromatische Gruppe, eine substituierte aromatische Gruppe, eine nicht-aromatische heterocyclische Gruppe, -NHC(O)-O-(aliphatische Gruppe), -NHC(O)-O-(aromatische Gruppe) oder -NHC(O)-O-(nicht-aromatische heterocyclische Gruppe) sind; oder
R²¹ und R²² zusammen mit dem Stickstoffatom, an das sie gebunden sind, einen nicht-aromatischen heterocyclischen Ring bilden;
R²³ -H, eine aliphatische Gruppe, eine Benzylgruppe, eine Arylgruppe oder nicht-aromatische heterocyclische Gruppe ist;
R²⁴ und R²⁵ unabhängig von einander -H, -OH, eine aliphatische Gruppe, eine substituierte aliphatische Gruppe, eine Benzylgruppe, eine Arylgruppe oder eine nicht-aromatische heterocyclische Gruppe sind;
t Null bis drei ist;
u Null oder eins ist;
p eins bis fünf ist;
Q -O-, -S-, -S(O)-, -S(O)₂-, -OS(O)₂-, -C(O)-, -OC(O)-, -C(O)O-, -C(O)C(O)-O-, -O-C(O)C(O)-, -C(O)NH-, -NHC(O)-, -OC(O)NH-, -NHC(O)O-, -NH-C(O)-NH-, -S(O)₂NH-, -NHS(O)₂-, -N(R²³)-, -C(NR²³)NHNH-, -NHNHC(NR²³)-, -NR²⁴C(O)- oder -NR²⁴S(O)₂- ist;
die Aminoalkylgruppe eine mit -NR²⁴R²⁵ substituierte C₁-C₁₂-Alkylgruppe ist;
die aliphatische Gruppe ein gesättigter oder ungesättigter C₁-C₂₀-Kohlenwasserstoff ist;
die Elektronen-anziehende Gruppe Alkylimino, Alkylsulfonyl, Carboxamido, Carbonsäurealkylester, -CH=NH oder -NO₂ ist;
die Acylgruppe aliphatisches Carbonyl, aromatisches Carbonyl, aliphatisches Sulfonyl oder aromatisches Sulfonyl ist;
die substituierte Acylgruppe substituiertes aliphatisches Carbonyl, substituiertes aromatisches Carbonyl, substituiertes aliphatisches Sulfonyl oder substituiertes aromatisches Sulfonyl ist;
der Heterocyclus der nicht-aromatischen heterocyclischen Gruppe ein 5- bis 8-gliedriger nicht-aromatischer Ring ist, der ein oder mehrere Heteroatome enthält, ausgewählt aus der Gruppe bestehend aus O, N und S; und
die aromatische Gruppe ausgewählt ist aus der Gruppe bestehend aus einem C₆-aromatischen Carbocyclus, einem C₅-C₆-aromatischen Heterocyclus, der ein oder mehrere Heteroatome aufweist, ausgewählt aus der Gruppe bestehend aus O, S und N, und einem C₆-aromatischen Carbocyclus oder einem C₅-C₆-aromatischen Heterocyclus, der mit einem oder mehreren Ringen kondensiert ist;
oder ein physiologisch annehmbares Salz davon.

26. Verbindung nach Anspruch 24, worin Z: 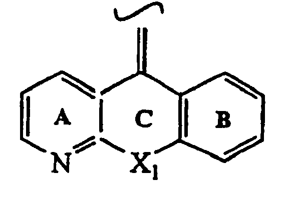 ist, worin
X₁ -CH₂-S-, -S-CH₂-, -O-CH₂-, -CH₂-O-, -SO-CH₂-, -CH₂-SO-, -S(O)₂-CH₂-, -CH₂-S(O)₂- oder eine Bindung bedeutet; und
Ring A und Ring B unabhängig von einander substituiert oder unsubstituiert sind.

27. Verbindung nach einem der Ansprüche 6 bis 23, 25 und 26, worin Ring B zum Kohlenstoffatom des Rings B, das an X₁ im Ring C gebunden ist, in para-Stellung so substituiert ist, dass Z: 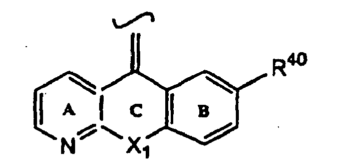 ist, worin:
R⁴⁰ -OH, -COOH, -NO₂, Halogen, eine aliphatische Gruppe, eine substituierte aliphatische Gruppe, eine aromatische Gruppe, eine substituierte aromatische Gruppe, -NR²⁴R²⁵, -CONR²⁴R²⁵, Q-(aliphatische Gruppe), Q-(substituierte aliphatische Gruppe), -O-(aliphatische Gruppe), -O-(substituierte aliphatische Gruppe), -O-(aromatische Gruppe), -O-(substituierte aromatische Gruppe), eine Elektronen-anziehende Gruppe, -(O)ᵤ-(CH₂)ₜ-C(O)OR²⁰, -(O)ᵤ-(CH₂)ₜ-OC(O)R²⁰, -(O)ᵤ(CH₂)ₜ-C(O)-NR²¹R²² oder -(O)ᵤ-(CH₂)ₜ-NHC(O)O-R²⁰ ist;
R²⁰, R²¹ oder R²² unabhängig von einander-H, eine aliphatische Gruppe, eine substituierte aliphatische Gruppe, eine aromatische Gruppe, eine substituierte aromatische Gruppe oder eine nicht-aromatische heterocyclische Gruppe sind; oder
R²¹ und R²² zusammen mit dem Stickstoffatom, an das sie gebunden sind, einen nicht-aromatischen heterocyclischen Ring bilden;
Q -NR²⁴C(O)-, -NR²⁴S(O)₂- oder -C(O)O- ist;
R²⁴ und R²⁵ unabhängig von einander -H, -OH, eine aliphatische Gruppe oder eine substituierte aliphatische Gruppe sind;
u Null oder eins ist; und
t eine ganze Zahl von Null bis 3 ist.

28. Verbindung nach einem der Ansprüche 6 bis 23, 25 und 26, worin Ring B zum Kohlenstoffatom des Rings B, das an X₁ im Ring C gebunden ist, in para-Stellung so substituiert ist, dass Z 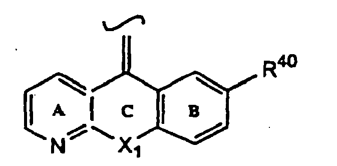 ist, worin:
R⁴⁰ -C(=NR⁶⁰)NR²¹R²², -O-C(O)-NR²¹R²⁶, -S(O)₂-NR²¹R²² oder -NH-C(O)-NR²¹R²² ist;
worin
R²¹ und R²² unabhängig von einander -H, eine aliphatische Gruppe, eine substituierte aliphatische Gruppe, eine aromatische Gruppe, eine substituierte aromatische Gruppe oder eine nicht-aromatische heterocyclische Gruppe sind; oder
R²¹ und R²² zusammen mit dem Stickstoffatom, an das sie gebunden sind, einen substituierten oder nicht-substituierten nicht-aromatischen heterocyclischen Ring bilden;
R²⁶ -H, eine aliphatische Gruppe, eine substituierte aliphatische Gruppe, eine aromatische Gruppe, eine substituierte aromatische Gruppe, eine nicht-aromatische heterocyclische Gruppe, -C(O)-O-(substituierte oder unsubstituierte aliphatische Gruppe), -C(O)-O-(substituierte oder unsubstituierte aromatische Gruppe), -S(O)₂-(substituierte oder unsubstituierte aliphatische Gruppe), -S(O)₂-(substituierte oder unsubstituierte aromatische Gruppe) ist; oder
R²⁶ und R²¹ zusammen mit dem Stickstoffatom, an das sie gebunden sind, einen substituierten oder unsubstituierten nicht-aromatischen heterocyclischen Ring bilden.

29. Verbindung nach einem der Ansprüche 1 bis 28, worin X₁ -CH₂-O- ist.

30. Verbindung, die ein tertiäres Amin der folgenden Formel ist: 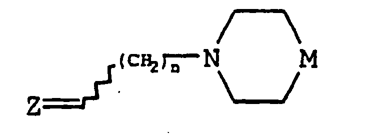 worin bedeuten:
M CR¹R²;
R¹-OH;
R² 4-Chlorphenyl;
n zwei;
Z: 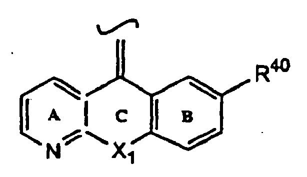
X₁ -CH₂-O-; und
R⁴⁰ 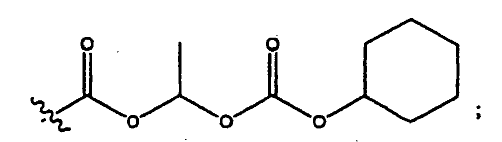
oder ein physiologisch annehmbares Salz davon.

31. Verbindung nach einem der Ansprüche 6 bis 10, worin R¹ eine substituierte lineare, verzweigte oder cyclische C₁-C₂₀-Alkyl-, -Alkenyl- oder -Alkinylgruppe ist.

32. Verbindung nach einem der Ansprüche 1 bis 31 zur Verwendung in einem Verfahren zur therapeutischen Behandlung des menschlichen oder tierischen Körpers.

33. Verbindung nach Anspruch 32 zur Verwendung bei der Behandlung einer mit einer aberrierenden Leukozyten-Stärkung und/oder -Aktivierung assoziierten Erkrankung.

34. Verbindung nach Anspruch 32 zur Verwendung bei der Behandlung einer chronischen entzündlichen Erkrankung.

35. Verbindung nach Anspruch 32 zur Verwendung bei der Behandlung von rheumatoider Arthritis.

36. Verbindung nach Anspruch 32 zur Verwendung bei der Behandlung von Multipler Sklerose.

37. Verwendung einer in einem der Ansprüche 1 bis 31 definierten Verbindung zur Herstellung eines Arzneimittels zur Behandlung einer mit aberrierender Leukozyten-Stärkung und/oder -Aktivierung assoziierten Erkrankung.

38. Verwendung einer in einem der Ansprüche 1 bis 31 definierten Verbindung zur Herstellung eines Arzneimittels zur Behandlung einer chronischen entzündlichen Erkrankung.

39. Verwendung einer in einem der Ansprüche 1 bis 31 definierten Verbindung zur Herstellung eines Arzneimittels zur Behandlung von rheumatoider Arthritis.

40. Verwendung einer in einem der Ansprüche 1 bis 31 definierten Verbindung zur Herstellung eines Arzneimittels zur Behandlung von Multipler Sklerose.

## Revendications

1. Composé qui est une amine tertiaire de formule suivante : 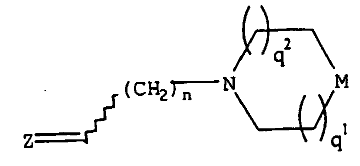 dans laquelle :
n est un à quatre ;
M est >NR², >CR¹R², -O-CR¹R²-O- ou -CH-CR¹R²-O-;
q¹ est zéro à trois ;
q² est zéro à un ;
R¹ est -H, -OH, -N₃, un halogène, un groupe aliphatique, un groupe aliphatique substitué, un groupe aminoalkyle, -O-(groupe aliphatique), -O-(groupe aliphatique substitué), -SH, -S-(groupe aliphatique), -S-(groupe aliphatique substitué), -OC(O)-(groupe aliphatique), -O-C(O)-(groupe aliphatique substitué), - C(O)O-(groupe aliphatique), -C(O)O-(groupe aliphatique substitué), -COOH, -CN, - CO-NR³R⁴, -NR³R⁴ ou bien R¹ est une liaison covalente entre l'atome du noyau en M et un atome de carbone adjacent dans le noyau qui contient M ;
R² est -OH, un groupe acyle, un groupe acyle substitué, -NR⁵R⁶, un groupe aliphatique, un groupe aliphatique substitué, un groupe aromatique, un groupe aromatique substitué, un groupe benzyle, un groupe benzyle substitué, un groupe hétérocyclique non aromatique, un groupe hétérocyclique non aromatique substitué, - O-(groupe aromatique substitué ou non substitué) ou -O-(groupe aliphatique substitué ou non substitué) ;
R³, R⁴, R⁵ et R⁶ sont indépendamment -H, un groupe acyle, un groupe acyle substitué, un groupe aliphatique, un groupe aliphatique substitué, un groupe aromatique, un groupe aromatique substitué, un groupe benzyle, un groupe benzyle substitué, un groupe hétérocyclique non aromatique ou un groupe hétérocyclique non aromatique substitué ; ou
R¹ et R², R³ et R⁴, ou R⁵ et R⁶ pris en association avec l'atome auquel ils sont liés, forment un noyau carbocyclique ou hétérocyclique non aromatique substitué ou non substitué ;
Z est: 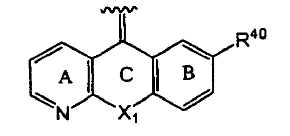
dans lequel :
X₁ est -CH₂-S-, -S-CH₂-, -O-CH₂-, -CH₂-O-, -SO-CH₂-, -CH₂-SO-, -S(O)₂-CH₂-, -CH₂-S(O)₂-, ou une liaison ;
R⁴⁰ est -S(O)₂-NR²¹R²², -NH-C(O)-R²¹R²², -O-C(O)-R²¹R²⁶, 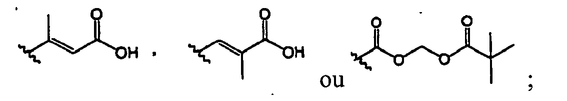
dans lesquels :
ledit groupe aliphatique substitué comprend un ou plusieurs substituants choisis dans le groupe consistant en un groupe électro-attracteur, halo, azido, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), oxo, époxy, hétérocycle non aromatique, benzyle, benzyle substitué, groupe aromatique et groupe aromatique substitué ;
ledit groupe benzyle substitué et ledit groupe aromatique substitué comprennent un ou plusieurs substituants choisis dans le groupe consistant en un groupe électro-attracteur, halo, azido, -CN, -COOH, -OH, -CONR²⁴R²⁵, -NR²⁴R²⁵, - OS(O)₂NR²⁴R²⁵, -S(O)₂NR²⁴R²⁵, -SO₃H, -S(O)₂NE₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), -Q-(CH₂)ₚ-(groupe hétérocyclique non aromatique), groupe aliphatique et groupe aliphatique substitué ;
ledit groupe hétérocyclique non aromatique substitué comprend un ou plusieurs substituants choisis dans le groupe consistant en un groupe électro-attracteur, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, - Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), -Q-(CH₂)ₚ-(groupe hétérocyclique non aromatique), groupe aliphatique, groupe aliphatique substitué, =O, =S, =NH, =N(aliphatique), =N(aromatique) et =N(aromatique substitué) ;
R²⁰, R²¹ et R²² sont indépendamment -H, un groupe aliphatique, un groupe aliphatique substitué, un groupe aromatique, un groupe aromatique substitué, un groupe hétérocyclique non aromatique, -NHC(O)-O-( groupe aliphatique), -NHC(O)-O-(groupe aromatique) ou -NHC(O)-O-( groupe hétérocyclique non aromatique) ;
R²⁶ est -H, un groupe aliphatique, un groupe aliphatique substitué, un groupe aromatique, un groupe aromatique substitué, un groupe hétérocyclique non aromatique, -C(O)-O-(groupe aliphatique substitué ou non substitué), -C(O)-O-(groupe aromatique substitué ou non substitué), -S(O)₂-(groupe aliphatique substitué ou non substitué), -S(O)₂-(groupe aromatique substitué ou non substitué) ; ou
R²¹ et R²², pris en association avec l'atome d'azote auquel ils sont liés, forment un noyau hétérocyclique non aromatique ; ou
R²¹ pris en association avec R²⁶ et l'atome d'azote auquel ils sont liés, forment un noyau hétérocyclique non aromatique substitué ou non substitué ;
R²³ est -H, un groupe aliphatique, un groupe benzyle, un groupe aryle ou un groupe hétérocyclique non aromatique ;
R²⁴ et R²⁵ sont indépendamment -H, -OH, un groupe aliphatique, un groupe aliphatique substitué, un groupe benzyle, un groupe aryle ou un groupe hétérocyclique non aromatique ;
t est zéro à trois ;
u est zéro à un ;
p est un à cinq ;
Q est -O-, -S-, -S(O)-, -S(O)₂-, -OS(O)₂-, -C(O)-, -OC(O)-, -C(O)O-, - C(O)C(O)-O-, -O-C(O)C(O)-, -C(O)NH-, -NHC(O)-, -OC(O)NH-, -NHC(O)O-, - NH-C(O)-NH-, -S(O)₂NH-, -NHS(O)₂-, -N(R²³)-, -C(NR²³)NHNH-, - NHNHC(NR²³)-, -NR²⁴C(O)- ou -NR²⁴S(O)₂- ;
ledit groupe aminoalkyle est un groupe alkyle en C₁ à C₁₂ substitué par - NR²⁴R²⁵;
ledit groupe aliphatique est un hydrocarbure en C₁ à C₂₀ saturé ou non saturé ;
ledit groupe électro-attracteur est un alkylimino, un alkylsulfonyle, un carboxamido, un ester carboxylique d'alkyle, -CH=NH ou -NO₂;
ledit groupe acyle est un carbonyle aliphatique, un carbonyle aromatique, un sulfonyle aliphatique ou un sulfonyle aromatique ;
ledit groupe acyle substitué est un carbonyle aliphatique substitué, un carbonyle aromatique substitué, un sulfonyle aliphatique substitué ou un sulfonyle aromatique substitué ;
l'hétérocycle dudit groupe hétérocyclique non aromatique est un noyau non aromatique comportant cinq à huit chaînons qui contient un ou plusieurs hétéroatomes choisis dans le groupe consistant en O, N, et S ; et
ledit groupe aromatique est choisi dans le groupe consistant en un carbocycle aromatique en C₆, un hétérocycle aromatique en C₅ à C₆ comprenant un ou plusieurs hétéroatomes choisis dans le groupe consistant en O, N, et S et un carbocycle aromatique en C₆ ou un hétérocycle aromatique en C₅ à C₆ qui est fusionné à un ou plusieurs autres noyaux ;
ou un sel de celui-ci acceptable du point de vue physiologique.

2. Composé selon la revendication 1 dans lequel q¹ et q² sont chacun un.

3. Composé selon la revendication 2 dans lequel M est >CR¹R².

4. Composé selon la revendication 3 dans lequel R¹ est -OH et R² est un groupe aromatique substitué.

5. Composé selon la revendication 4 dans lequel R² est 4-chlorophényle.

6. Composé qui est une amine tertiaire de formule suivante : 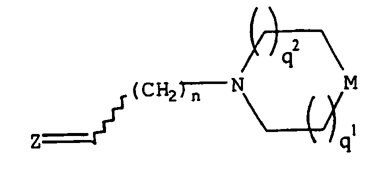 dans laquelle :
n est un à quatre ;
M est >CR¹R², -O-CR¹R²-O- ou -CH-CR¹R²-O- ;
q¹ est zéro à trois ;
q² est zéro à un ;
R¹ est un groupe aliphatique substitué ou un groupe aminoalkyle ;
R² est -OH, un groupe acyle, un groupe acyle substitué, -NR⁵R⁶, un groupe aliphatique, un groupe aliphatique substitué, un groupe aromatique, un groupe aromatique substitué, un groupe benzyle, un groupe benzyle substitué, un groupe hétérocyclique non aromatique, un groupe hétérocyclique non aromatique substitué,-O-(groupe aromatique substitué ou non substitué) ou -O-(groupe aliphatique substitué ou non substitué) ;
R⁵ et R⁶ sont indépendamment -H, un groupe acyle, un groupe acyle substitué, un groupe aliphatique, un groupe aliphatique substitué, un groupe aromatique, un groupe aromatique substitué, un groupe benzyle, un groupe benzyle substitué, un groupe hétérocyclique non aromatique ou un groupe hétérocyclique non aromatique substitué ; ou
R⁵ et R⁶ pris en association avec l'atome auquel ils sont liés, forment un noyau carbocyclique ou hétérocyclique non aromatique substitué ou non substitué ;
Zest: 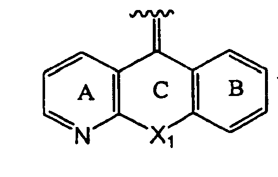
dans lequel :
X₁ est -CH₂-S-, -S-CH₂-, -O-CH₂-, -CH₂-O-, -SO-CH₂-, -CH₂-SO-, -S(O)₂-CH₂-, -CH₂-S(O)₂-, ou une liaison ;
le noyau A et le noyau B sont indépendamment substitués ou non substitués ;
dans lesquels :
ledit groupe aliphatique substitué comprend un ou plusieurs substituants choisis dans le groupe consistant en un groupe électro-attracteur, halo, azido, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜC(O)OR²⁰, -(O)ᵤ₋(CH₂)ₜOC(O)R²⁰, -(O)ᵤ-(CH₂)ₜ-C(O)-NR²¹R²², -(O)ᵤ-(CH₂)ₜNHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), oxo, époxy, hétérocycle non aromatique, benzyle, benzyle substitué, groupe aromatique et groupe aromatique substitué ;
ledit groupe benzyle substitué et ledit groupe aromatique substitué et les noyaux A et B lorsqu'ils sont substitués comprennent un ou plusieurs substituants choisis dans le groupe consistant en un groupe électro-attracteur, halo, azido, -CN, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜ-C(O)OR²⁰, -(O)ᵤ-(CH₂)ₜOC(O)R²⁰, -(O)ᵤ-(CH₂)ₜ-C(O)-NR²¹R²², -(O)ᵤ-(CH₂)ₜNHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), groupe aliphatique et groupe aliphatique substitué ;
ledit groupe hétérocyclique non aromatique substitué comprend un ou plusieurs substituants choisis dans le groupe consistant en un groupe électro-attracteur, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, - Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), -Q-(CH₂)ₚ-(groupe hétérocyclique non aromatique), groupe aliphatique, groupe aliphatique substitué, =O, =S, =NH, =N(aliphatique), =N(aromatique) et =N(aromatique substitué) ;
R²⁰, R²¹ et R²² sont indépendamment -H, un groupe aliphatique, un groupe aliphatique substitué, un groupe aromatique, un groupe aromatique substitué, un groupe hétérocyclique non aromatique, -NHC(O)-O-(groupe aliphatique), -NHC(O)-O-(groupe aromatique) ou -NHC(O)-O-( groupe hétérocyclique non aromatique) ; ou
R²¹ et R²², pris en association avec l'atome d'azote auquel ils sont liés, forment un noyau hétérocyclique non aromatique ;
R²³ est -H, un groupe aliphatique, un groupe benzyle, un groupe aryle ou un groupe hétérocyclique non aromatique ;
R²⁴ et R²⁵ sont indépendamment -H, -OH, un groupe aliphatique, un groupe aliphatique substitué, un groupe benzyle, un groupe aryle ou un groupe hétérocyclique non aromatique ;
t est zéro à trois ;
u est zéro à un :
p est un à cinq
Q est -O-, -S-, -S(O)-, -S(O)₂-, -OS(O)₂-, -C(O)-, -OC(O)-, -C(O)O-, - C(O)C(O)-O-, -O-C(O)C(O)-, -C(O)NH-, -NHC(O)-, -OC(O)NH-, -NHC(O)O-, - NH-C(O)-NH-, -S(O)₂NH-, -NHS(O)₂-, -N(R²³)-, -C(NR²³)NHNH-, - NHNHC(NR²³), -NR²⁴C(O)- ou -NR²⁴S(O)₂- ;
ledit groupe aminoalkyle est un groupe alkyle en C₁ à C₁₂ substitué par - NR²⁴R²⁵.
ledit groupe aliphatique est un hydrocarbure en C₁ à C₂₀ saturé ou non saturé ;
ledit groupe électro-attracteur est un alkylimino, un alkylsulfonyle, un carboxamido, un ester carboxylique d'alkyle, -CH=NH ou -NO₂ ;
ledit groupe acyle est un carbonyle aliphatique, un carbonyle aromatique, un sulfonyle aliphatique ou un sulfonyle aromatique ;
ledit groupe acyle substitué est un carbonyle aliphatique substitué, un carbonyle aromatique substitué, un sulfonyle aliphatique substitué ou un sulfonyle aromatique substitué ;
l'hétérocycle dudit groupe hétérocyclique non aromatique est un noyau non aromatique comportant cinq à huit chaînons qui contient un ou plusieurs hétéroatomes choisis dans le groupe consistant en O, N, et S ; et
ledit groupe aromatique est choisi dans le groupe consistant en un carbocycle aromatique en C₆, un hétérocycle aromatique en C₅ à C₆ comprenant un ou plusieurs hétéroatomes choisis dans le groupe consistant en O, N, et S et un carbocycle aromatique en C₆ ou un hétérocycle aromatique en C₅ à C₆ qui est fusionné à un ou plusieurs autres noyaux ;
ou un sel de celui-ci acceptable du point de vue physiologique,
autre que :

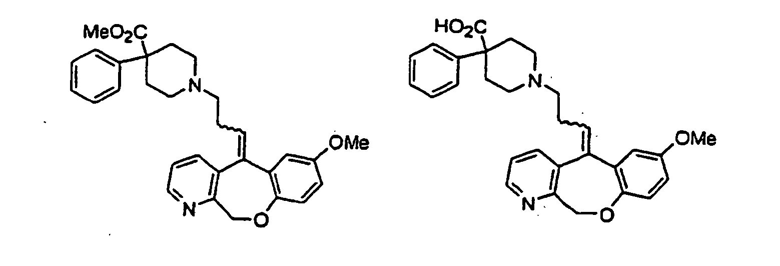

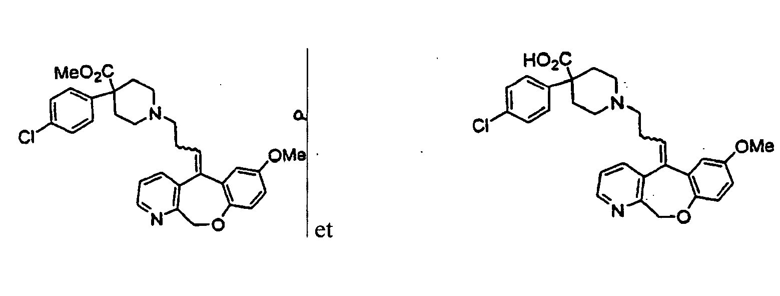

7. Composé selon la revendication 6 dans lequel q¹ et q² sont chacun un.

8. Composé selon la revendication 7 dans lequel M est >CR¹R².

9. Composé selon la revendication 8 dans lequel R² est un groupe aromatique substitué.

10. Composé selon la revendication 9 dans lequel R² est 4-chlorophényle.

11. Composé selon l'une quelconque des revendications 6 à 10, dans lequel R¹ est un groupe alkylamine choisit dans le groupe constitué par l'aminoéthyle, le 2-aminoéthyle, le 3-aminopropyle, le 4-aminobutyle, le diméthylaninoéthyle, le diéthylaminométhyle, le méthylaminohéxyle et l'aminoéthylényle.

12. Composé qui est une amine tertiaire de formule suivante : 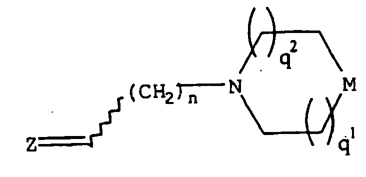 dans laquelle :
n est un à quatre ;
M est >NR², >CR¹R², -O-CR¹R²-O- ou -CH-CR¹R²-O- ;
q¹ est zéro à trois ;
q² est zéro à un ;
R¹ est -H, -OH, -N₃, un halogène, un groupe aliphatique, un groupe aliphatique substitué, un groupe aminoalkyle, -O-(groupe aliphatique), -O-(groupe aliphatique substitué), -SH, -S-(groupe aliphatique), -S-(groupe aliphatique substitué), -OC(O)-(groupe aliphatique), -O-C(O)-(groupe aliphatique substitué), - C(O)O-(groupe aliphatique), -C(O)O-(groupe aliphatique substitué), -COOH, -CN, - CO-NR³R⁴, -NR³R⁴ ou bien R¹ est une liaison covalente entre l'atome du noyau en M et un atome de carbone adjacent dans le noyau qui contient M ;
R² est -O-(groupe aromatique substitué ou non substitué) ;
R³ et R⁴ sont indépendamment -H, un groupe acyle, un groupe acyle substitué, un groupe aliphatique, un groupe aliphatique substitué, un groupe aromatique, un groupe aromatique substitué, un groupe benzyle, un groupe benzyle substitué, un groupe hétérocyclique non aromatique ou un groupe hétérocyclique non aromatique substitué ; ou
R³ et R⁴ pris en association avec l'atome auquel ils sont liés, forment un noyau carbocyclique ou hétérocyclique non aromatique substitué ou non substitué ;
Zest: 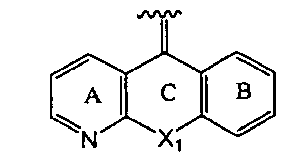
dans lequel :
X₁ est -CH₂-S-, -S-CH₂-, -O-CH₂-, -CH₂-O-, -SO-CH₂-, -CH₂-SO-, -S(O)₂-CH₂-, -CH₂-S(O)₂-, ou une liaison ;
le noyau A et le noyau B sont indépendamment substitués ou non substitués ;
dans lesquels :
ledit groupe aliphatique substitué comprend un ou plusieurs substituants choisis dans le groupe consistant en un groupe électro-attracteur, halo, azido, - COOH, -OH, -CONR²⁴R²⁵, -NR²⁴R²⁵, -OS(O)₂NR²⁴R²⁵, -S(O)₂NR²⁴R²⁵, -SO₃H, S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)Qₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), oxo, époxy, hétérocycle non aromatique, benzyle, benzyle substitué, groupe aromatique et groupe aromatique substitué ;
ledit groupe benzyle substitué et ledit groupe aromatique substitué et les noyaux A et B lorsqu'ils sont substitués comprennent un ou plusieurs substituants choisis dans le groupe consistant en un groupe électro-attracteur, halo, azido, -CN, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), groupe aliphatique et groupe aliphatique substitué ;
ledit groupe hétérocyclique non aromatique substitué comprend un ou plusieurs substituants choisis dans le groupe consistant en un groupe électro-attracteur, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, - Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), -Q-(CH₂)ₚ-(groupe hétérocyclique non aromatique), groupe aliphatique, groupe aliphatique substitué, =O, =S, =NH, =N(aliphatique), =N(aromatique) et =N(aromatique substitué) ;
R²⁰, R²¹ et R²² sont indépendamment -H, un groupe aliphatique, un groupe aliphatique substitué, un groupe aromatique, un groupe aromatique substitué, un groupe hétérocyclique non aromatique, -NHC(O)-O-(groupe aliphatique), -NHC(O)-O-(groupe aromatique) ou -NHC(O)-O-( groupe hétérocyclique non aromatique) ; ou
R²¹ et R²², pris en association avec l'atome d'azote auquel ils sont liés, forment un noyau hétérocyclique non aromatique ; ou
R²³ est -H, un groupe aliphatique, un groupe benzyle, un groupe aryle ou un groupe hétérocyclique non aromatique ;
R²⁴ et R²⁵ sont indépendamment -H, -OH, un groupe aliphatique, un groupe aliphatique substitué, un groupe benzyle, un groupe aryle ou un groupe hétérocyclique non aromatique ;
t est zéro à trois ;
u est zéro à un ;
p est un à cinq ;
Q est -O-, -S-, -S(O)-, -S(O)₂-, -OS(O)₂-, -C(O)-, -OC(O)-, -C(O)O-, - C(O)C(O)-O-, -O-C(O)C(O)-, -C(O)NH-, -NHC(O)-, -OC(O)NH-, -NHC(O)O-, - NH-C(O)-NH-, -S(O)₂NH-, -NHS(O)₂-, -N(R²³)-, -C(NR²³)NHNH-, - NHNHC(NR²³)-, -NR²⁴C(O)- ou -NR²⁴S(O)₂- ;
ledit groupe aminoalkyle est un groupe alkyle en C₁ à C₁₂ substitué par - NR²⁴R²⁵;
ledit groupe aliphatique est un hydrocarbure en C₁ à C₂₀ saturé ou non saturé ;
ledit groupe électro-attracteur est un alkylimino, un alkylsulfonyle, un carboxamido, un ester carboxylique d'alkyle, -CH=NH ou -NO₂ ;
ledit groupe acyle est un carbonyle aliphatique, un carbonyle aromatique, un sulfonyle aliphatique ou un sulfonyle aromatique ;
ledit groupe acyle substitué est un carbonyle aliphatique substitué, un carbonyle aromatique substitué, un sulfonyle aliphatique substitué ou un sulfonyle aromatique substitué ;
l'hétérocycle dudit groupe hétérocyclique non aromatique est un noyau non aromatique comportant cinq à huit chaînons qui contient un ou plusieurs hétéroatomes choisis dans le groupe consistant en O, N, et S ; et
ledit groupe aromatique est choisi dans le groupe consistant en un carbocycle aromatique en C₆, un hétérocycle aromatique en C₅ à C₆ comprenant un ou plusieurs hétéroatomes choisis dans le groupe consistant en O, N, et S et un carbocycle aromatique en C₆ ou un hétérocycle aromatique en C₅ à C₆ qui est fusionné à un ou plusieurs autres noyaux ;
ou un sel de celui-ci acceptable du point de vue physiologique.

13. Composé selon la revendication 12 dans lequel q¹ et q² sont chacun un.

14. Composé selon la revendication 12 dans lequel M est >CR¹R².

15. Composé selon la revendication 8 dans lequel R¹ est -OH.

16. Composé qui est une amine tertiaire de formule suivante : 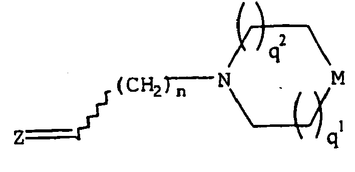 dans laquelle :
n est un à quatre ;
M est -O-CR¹R²-O- ou -CH-CR¹R²-O- ;
q¹ est zéro à trois ;
q² est zéro à un ;
R¹ est -H, -OH, -N₃, un halogène, un groupe aliphatique, un groupe aliphatique substitué, un groupe aminoalkyle, -O-(groupe aliphatique), -O-(groupe aliphatique substitué), -SH, -S-(groupe aliphatique), -S-(groupe aliphatique substitué), -OC(O)-(groupe aliphatique), -O-C(O)-(groupe aliphatique substitué), - C(O)O-(groupe aliphatique), -C(O)O-(groupe aliphatique substitué), -COOH, -CN, - CO-NR³R⁴, -NR³R⁴ ou bien R¹ est une liaison covalente entre l'atome du noyau en M et un atome de carbone adjacent dans le noyau qui contient M ;
R² est -OH, un groupe acyle, un groupe acyle substitué, -NR⁵R⁶, un groupe aliphatique, un groupe aliphatique substitué, un groupe aromatique, un groupe aromatique substitué, un groupe benzyle, un groupe benzyle substitué, un groupe hétérocyclique non aromatique, un groupe hétérocyclique non aromatique substitué, - O-(groupe aromatique substitué ou non substitué) ou -O-(groupe aliphatique substitué ou non substitué) ;
R³, R⁴, R⁵ et R⁶ sont indépendamment -H, un groupe acyle, un groupe acyle substitué, un groupe aliphatique, un groupe aliphatique substitué, un groupe aromatique, un groupe aromatique substitué, un groupe benzyle, un groupe benzyle substitué, un groupe hétérocyclique non aromatique ou un groupe hétérocyclique non aromatique substitué ; ou
R¹ et R², R³ et R⁴, ou R⁵ et R⁶ pris en association avec l'atome auquel ils sont liés, forment un noyau carbocyclique ou hétérocyclique non aromatique substitué ou non substitué ;
Z est : 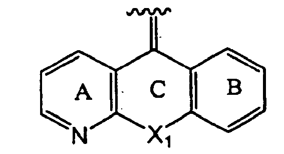
dans lequel :
X₁ est -CH₂-S-, -S-CH₂-, -O-CH₂-, -CH₂-O-, -SO-CH₂-, -CH₂-SO-, -S(O)₂-CH₂-, -CH₂-S(O)₂-, ou une liaison ;
le noyau A et le noyau B sont indépendamment substitués ou non substitués ;
dans lesquels :
ledit groupe aliphatique substitué comprend un ou plusieurs substituants choisis dans le groupe consistant en un groupe électro-attracteur, halo, azido, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), oxo, époxy, hétérocycle non aromatique, benzyle, benzyle substitué, groupe aromatique et groupe aromatique substitué ;
ledit groupe benzyle substitué et ledit groupe aromatique substitué comprennent un ou plusieurs substituants choisis dans le groupe consistant en un groupe électro-attracteur, halo, azido, -CN, -COOH, -OH, -CONR²⁴R²⁵, -NR²⁴R²⁵, - OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), -Q-(CH₂)ₚ-(groupe hétérocyclique non aromatique), groupe aliphatique et groupe aliphatique substitué :
ledit groupe hétérocyclique non aromatique substitué comprend un ou plusieurs substituants choisis dans le groupe consistant en un groupe électro-attracteur, halo, azido, -CN, -COOH, -OH, -CONR²⁴R²⁵ -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, - Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), **-Q-(CH₂)_{P}-**(groupe hétérocyclique non aromatique), groupe aliphatique, groupe aliphatique substitué, =O, =S, =NH, =N(aliphatique), =N(aromatique) et =N(aromatique substitué) ;
R²⁰, R²¹ et R²² sont indépendamment -H, un groupe aliphatique, un groupe aliphatique substitué, un groupe aromatique, un groupe aromatique substitué, un groupe hétérocyclique non aromatique, -NHC(O)-O-( groupe aliphatique), -NHC(O)-O-(groupe aromatique) ou -NHC(O)-O-( groupe hétérocyclique non aromatique) ; ou
R²¹ et R²², pris en association avec l'atome d'azote auquel ils sont liés, forment un noyau hétérocyclique non aromatique ;
R²³ est -H, un groupe aliphatique, un groupe benzyle, un groupe aryle ou un groupe hétérocyclique non aromatique ;
R²⁴ et R²⁵ sont indépendamment -H, -OH, un groupe aliphatique, un groupe aliphatique substitué, un groupe benzyle, un groupe aryle ou un groupe hétérocyclique non aromatique;
t est zéro à trois ;
u est zéro à un ;
p est un à cinq ;
Q est -O-, -S-, -S(O)-, -S(O)₂-, -OS(O)₂-, -C(O)-, -OC(O)-, -C(O)O-, - C(O)C(O)-O-, -O-C(O)C(O)-, -C(O)NH-, -NHC(O)-, -OC(O)NH-, -NHC(O)O-, - NH-C(O)-NH-, -S(O)₂NH-, -NHS(O)₂-, -N(R²³)-, -C(NR²³)NHNH-, - NHNHC(NR²³)-, -NR²⁴C(O)- ou -NR²⁴S(O)₂- ;
ledit groupe aminoalkyle est un groupe alkyle en C₁ à C₁₂ substitué par - NR²⁴R²⁵;
ledit groupe aliphatique est un hydrocarbure en C₁ à C₂₀ saturé ou non saturé ;
ledit groupe électro-attracteur est un alkylimino, un alkylsulfonyle, un carboxamido, un ester carboxylique d'alkyle, -CH=NH ou -NO₂ ;
ledit groupe acyle est un carbonyle aliphatique, un carbonyle aromatique, un sulfonyle aliphatique ou un sulfonyle aromatique ;
ledit groupe acyle substitué est un carbonyle aliphatique substitué, un carbonyle aromatique substitué, un sulfonyle aliphatique substitué ou un sulfonyle aromatique substitué ;
l'hétérocycle dudit groupe hétérocyclique non aromatique est un noyau non aromatique comportant cinq à huit chaînons qui contient un ou plusieurs hétéroatomes choisis dans le groupe consistant en O, N, et S ; et
ledit groupe aromatique est choisi dans le groupe consistant en un carbocycle aromatique en C₆, un hétérocycle aromatique en C₅ à C₆ comprenant un ou plusieurs hétéroatomes choisis dans le groupe consistant en O, N, et S et un carbocycle aromatique en C₆ ou un hétérocycle aromatique en C₅ à C₆ qui est fusionné à un ou plusieurs autres noyaux ;
ou un sel de celui-ci acceptable du point de vue physiologique.

17. Composé selon la revendication 16 dans lequel q¹ et q² sont chacun un.

18. Composé selon la revendication 17 dans lequel R¹ est -OH et R² est un groupe aromatique substitué.

19. Composé selon la revendication 18 dans lequel R² est 4-chlorophényle.

20. Composé qui est une amine tertiaire de formule suivante : 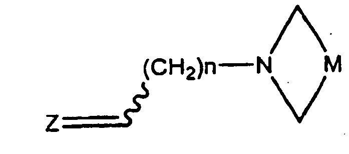 dans laquelle :
n est un à quatre ;
M est >NR² ou >CR¹R² ;
R¹ est -H, -OH, -N₃, un halogène, un groupe aliphatique, un groupe aliphatique substitué, un groupe aminoalkyle, -O-(groupe aliphatique), -O-(groupe aliphatique substitué), -SH, -S-(groupe aliphatique), -S-(groupe aliphatique substitué), -OC(O)-(groupe aliphatique), -O-C(O)-(groupe aliphatique substitué), - C(O)O-(groupe aliphatique), -C(O)O-(groupe aliphatique substitué), -COOH, -CN, - CO-NR³R⁴, -NR³R⁴ ou bien R¹ est une liaison covalente entre l'atome du noyau en M et un atome de carbone adjacent dans le noyau qui contient M ;
R² est -OH, un groupe acyle, un groupe acyle substitué, -NR⁵R⁶, un groupe aliphatique, un groupe aliphatique substitué, un groupe aromatique, un groupe aromatique substitué, un groupe benzyle, un groupe benzyle substitué, un groupe hétérocyclique non aromatique, un groupe hétérocyclique non aromatique substitué, - 0-(groupe aromatique substitué ou non substitué) ou -O-(groupe aliphatique substitué ou non substitué) ;
R³, R⁴, R⁵ et R⁶ sont indépendamment -H, un groupe acyle, un groupe acyle substitué, un groupe aliphatique, un groupe aliphatique substitué, un groupe aromatique, un groupe aromatique substitué, un groupe benzyle, un groupe benzyle substitué, un groupe hétérocyclique non aromatique ou un groupe hétérocyclique non aromatique substitué ; ou
R¹ et R², R³ et R⁴, ou R⁵ et R⁶ pris en association avec l'atome auquel ils sont liés, forment un noyau carbocyclique ou hétérocyclique non aromatique substitué ou non substitué ;
Z est: 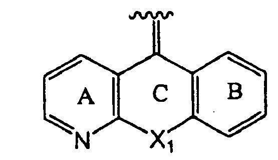
dans lequel :
X₁ est -CH₂-S-, -S-CH₂-, -O-CH₂-, -CH₂-O-, -SO-CH₂-, -CH₂-SO-, -S(O)₂-CH₂-, -CH₂-S(O)₂-, ou une liaison ;
le noyau A et le noyau B sont indépendamment substitués ou non substitués ;
dans lesquels :
ledit groupe aliphatique substitué comprend un ou plusieurs substituants choisis dans le groupe consistant en un groupe électro-attracteur, halo, azido, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), oxo, époxy, hétérocycle non aromatique, benzyle, benzyle substitué, groupe aromatique et groupe aromatique substitué ;
ledit groupe benzyle substitué et ledit groupe aromatique substitué et comprennent un ou plusieurs substituants choisis dans le groupe consistant en un groupe électro-attracteur, halo, azido, -CN, -COOH, -OH, -CONR²⁴R²⁵, -NR²⁴R²⁵, - OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), -Q-(CH₂)ₚ-(groupe hétérocyclique non aromatique), groupe aliphatique et groupe aliphatique substitué ;
ledit groupe hétérocyclique non aromatique substitué comprend un ou plusieurs substituants choisis dans le groupe consistant en un groupe électro-attracteur, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, - Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), -Q-(CH₂)ₚ-(groupe hétérocyclique non aromatique), groupe aliphatique, groupe aliphatique substitué, =O, =S, =NH, =N(aliphatique), =N(aromatique) et =N(aromatique substitué) ;
R²⁰, R²¹ et R²² sont indépendamment -H, un groupe aliphatique, un groupe aliphatique substitué, un groupe aromatique, un groupe aromatique substitué, un groupe hétérocyclique non aromatique, -NHC(O)-O-(groupe aliphatique), -NHC(O)-O-(groupe aromatique) ou -NHC(O)-O-( groupe hétérocyclique non aromatique) ; ou
R²¹ et R²², pris en association avec l'atome d'azote auquel ils sont liés, forment un noyau hétérocyclique non aromatique ;
R²³ est -H, un groupe aliphatique, un groupe benzyle, un groupe aryle ou un groupe hétérocyclique non aromatique ;
R²⁴ et R²⁵ sont indépendamment -H, -OH, un groupe aliphatique, un groupe aliphatique substitué, un groupe benzyle, un groupe aryle ou un groupe hétérocyclique non aromatique ;
t est zéro à trois ;
u est zéro à un ;
p est un à cinq;
Q est -O-, -S-, -S(O)-, -S(O)₂-, -OS(O)₂-, -C(O)-, -OC(O)-, -C(O)O-, - C(O)C(O)-O-, -O-C(O)C(O)-, -C(O)NH-, -NHC(O)-, -OC(O)NH-, -NHC(O)O-, - NH-C(O)-NH-, -S(O)₂NH-, -NHS(O)₂-, -N(R²³)-, -C(NR²³)NHNH-, - NHNHC(NR²³)-, -NR²⁴C(O)- ou -NR²⁴S(O)₂- ;
ledit groupe aminoalkyle est un groupe alkyle en C₁ à C₁₂ substitué par - NR²⁴R²⁵;
ledit groupe aliphatique est un hydrocarbure en C₁ à C₂₀ saturé ou non saturé ;
ledit groupe électro-attracteur est un alkylimino, un alkylsulfonyle, un carboxamido, un ester carboxylique d'alkyle, -CH=NH ou -NO₂ ;
ledit groupe acyle est un carbonyle aliphatique, un carbonyle aromatique, un sulfonyle aliphatique ou un sulfonyle aromatique ;
ledit groupe acyle substitué est un carbonyle aliphatique substitué, un carbonyle aromatique substitué, un sulfonyle aliphatique substitué ou un sulfonyle aromatique substitué ;
l'hétérocycle dudit groupe hétérocyclique non aromatique est un noyau non aromatique comportant cinq à huit chaînons qui contient un ou plusieurs hétéroatomes choisis dans le groupe consistant en O, N, et S ; et
ledit groupe aromatique est choisi dans le groupe consistant en un carbocycle aromatique en C₆, un hétérocycle aromatique en C₅ à C₆ comprenant un ou plusieurs hétéroatomes choisis dans le groupe consistant en O, N, et S et un carbocycle aromatique en C₆ ou un hétérocycle aromatique en C₅ à C₆ qui est fusionné à un ou plusieurs autres noyaux ;
ou un sel de celui-ci acceptable du point de vue physiologique.

21. Composé selon la revendication 20 dans lequel M est >CR¹R².

22. Composé selon la revendication 21 dans lequel R¹ est -OH et R² est un groupe aromatique substitué.

23. Composé selon la revendication 22 dans lequel R² est 4-chlorophényle.

24. Composé qui est une amine tertiaire de formule suivante : 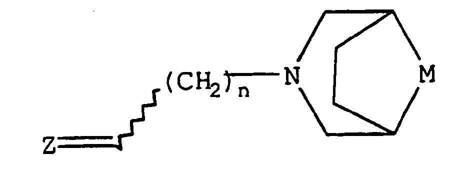 dans laquelle :
n est un à quatre ;
M est >NR², >CR¹R², -O-CR¹R²-O- ou -CH-CR¹R²-O- ;
le noyau contenant M est substitué ou non substitué ;
R¹ est -H, -OH, -N₃, un halogène, un groupe aliphatique, un groupe aliphatique substitué, un groupe aminoalkyle, -O-(groupe aliphatique), -O-(groupe aliphatique substitué), -SH, -S-(groupe aliphatique), -S-(groupe aliphatique substitué), -OC(O)-(groupe aliphatique), -O-C(O)-(groupe aliphatique substitué), - C(O)O-(groupe aliphatique), -C(O)O-(groupe aliphatique substitué), -COOH, -CN, - CO-NR³R⁴, -NR³R⁴ ou bien R¹ est une liaison covalente entre l'atome du noyau en M et un atome de carbone adjacent dans le noyau qui contient M ;
R² est -OH, un groupe acyle, un groupe acyle substitué, -NR⁵R⁶, un groupe aliphatique, un groupe aliphatique substitué, un groupe aromatique, un groupe aromatique substitué, un groupe benzyle, un groupe benzyle substitué, un groupe hétérocyclique non aromatique, un groupe hétérocyclique non aromatique substitué, - O-(groupe aromatique substitué ou non substitué) ou -O-(groupe aliphatique substitué ou non substitué) ;
R³, R⁴, R⁵ et R⁶ sont indépendamment -H, un groupe acyle, un groupe acyle substitué, un groupe aliphatique, un groupe aliphatique substitué, un groupe aromatique, un groupe aromatique substitué, un groupe benzyle, un groupe benzyle substitué, un groupe hétérocyclique non aromatique ou un groupe hétérocyclique non aromatique substitué ; ou
R¹ et R², R³ et R⁴, ou R⁵ et R⁶ pris en association avec l'atome auquel ils sont liés, forment un noyau carbocyclique ou hétérocyclique non aromatique substitué ou non substitué ;
Zest: 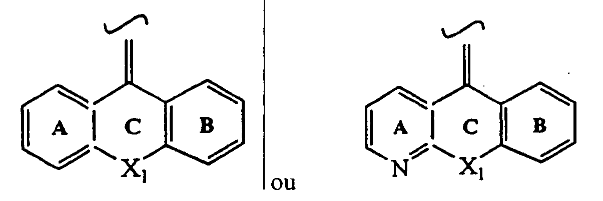
dans lequel :
X₁ est -CH₂-S-, -S-CH₂-, -O-CH₂-, -CH₂-O-, -SO-CH₂-, -CH₂-SO-, -S(O)₂-CH₂-, -CH₂-S(O)₂-, ou une liaison ;
le noyau A et le noyau B sont indépendamment substitués ou non substitués ;
dans lesquels :
ledit groupe aliphatique substitué comprend un ou plusieurs substituants choisis dans le groupe consistant en un groupe électro-attracteur, halo, azido, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), oxo, époxy, hétérocycle non aromatique, benzyle, benzyle substitué, groupe aromatique et groupe aromatique substitué ;
ledit groupe benzyle substitué et ledit groupe aromatique substitué et les noyaux A et B lorsqu'ils sont substitués comprennent un ou plusieurs substituants choisis dans le groupe consistant en un groupe électro-attracteur, halo, azido, -CN, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), groupe aliphatique et groupe aliphatique substitué ;
ledit groupe hétérocyclique non aromatique substitué comprend un ou plusieurs substituants choisis dans le groupe consistant en un groupe électro-attracteur, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, - Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), -Q-(CH₂)ₚ-(groupe hétérocyclique non aromatique), groupe aliphatique, groupe aliphatique substitué, =O, =S, =NH, =N(aliphatique), =N(aromatique) et =N(aromatique substitué) ;
R²⁰, R²¹ et R²² sont indépendamment -H, un groupe aliphatique, un groupe aliphatique substitué, un groupe aromatique, un groupe aromatique substitué, un groupe hétérocyclique non aromatique, -NHC(O)-O-(groupe aliphatique), -NHC(O)-O-(groupe aromatique) ou -NHC(O)-O-(groupe hétérocyclique non aromatique) ; ou
R²¹ et R²²; pris en association avec l'atome d'azote auquel ils sont liés, forment un noyau hétérocyclique non aromatique ;
R²³ est -H, un groupe aliphatique, un groupe benzyle, un groupe aryle ou un groupe hétérocyclique non aromatique ;
R²⁴ et R²⁵ sont indépendamment -H, -OH, un groupe aliphatique, un groupe aliphatique substitué, un groupe benzyle, un groupe aryle ou un groupe hétérocyclique non aromatique ;
t est zéro à trois ;
u est zéro à un ;
p est un à cinq ;
Q est -O-, -S-, -S(O)-, -S(O)₂-, -OS(O)₂-, -C(O)-, -OC(O)-, -C(O)O-, - C(O)C(O)-O-, -O-C(O)C(O)-, -C(O)NH-, -NHC(O)-, -OC(O)NH-, -NHC(O)O-, - NH-C(O)-NH-, -S(O)₂NH-, -NHS(O)₂-, -N(R²³)-, -C(NR²³)NHNH-, - NHNHC(NR²³)-, -NR²⁴C(O)- ou -NR²⁴S(O)₂- ;
ledit groupe aminoalkyle est un groupe alkyle en C₁ à C₁₂ substitué par - NR²⁴R²⁵;
ledit groupe aliphatique est un hydrocarbure en C₁ à C₂₀ saturé ou non saturé ;
ledit groupe électro-attracteur est un alkylimino, un alkylsulfonyle, un carboxamido, un ester carboxylique d'alkyle, -CH=NH ou -NO₂ ;
ledit groupe acyle est un carbonyle aliphatique, un carbonyle aromatique, un sulfonyle aliphatique ou un sulfonyle aromatique ;
ledit groupe acyle substitué est un carbonyle aliphatique substitué, un carbonyle aromatique substitué, un sulfonyle aliphatique substitué ou un sulfonyle aromatique substitué ;
l'hétérocycle dudit groupe hétérocyclique non aromatique est un noyau non aromatique comportant cinq à huit chaînons qui contient un ou plusieurs hétéroatomes choisis dans le groupe consistant en O, N, et S ; et
ledit groupe aromatique est choisi dans le groupe consistant en un carbocycle aromatique en C₆, un hétérocycle aromatique en C₅ à C₆ comprenant un ou plusieurs hétéroatomes choisis dans le groupe consistant en O, N, et S et un carbocycle aromatique en C₆ ou un hétérocycle aromatique en C₅ à C₆ qui est fusionné à un ou plusieurs autres noyaux ;
ou un sel de celui-ci acceptable du point de vue physiologique.

25. Composé qui est une amine tertiaire de formule suivante : 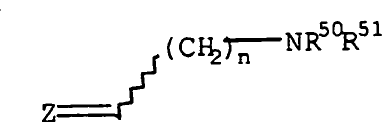 dans laquelle :
n est un à quatre ;
R⁵⁰ est R⁵¹ sont chacun, indépendamment, -H, un groupe aliphatique, un groupe aliphatique substitué, un groupe aminoalkyle, -NR³R⁴, un groupe aromatique, un groupe aromatique substitué, un groupe benzyle, un groupe benzyle substitué, un groupe hétérocyclique non aromatique, un groupe hétérocyclique non aromatique substitué, ou une liaison covalente entre l'atome d'azote et un atome de carbone adjacent ;
R³ et R⁴ sont indépendamment -H, un groupe acyle, un groupe acyle substitué, un groupe aliphatique, un groupe aliphatique substitué, un groupe aromatique, un groupe aromatique substitué, un groupe benzyle, un groupe benzyle substitué, un groupe hétérocyclique non aromatique ou un groupe hétérocyclique non aromatique substitué ;
Z est : 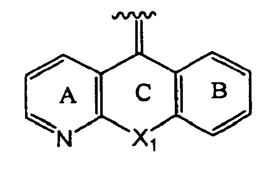
dans lequel :
X₁ est -CH₂-S-, -S-CH₂-, -O-CH₂-, -CH₂-O-, -SO-CH₂-, -CH₂-SO-, -S(O)₂-CH₂-, -CH₂-S(O)₂-, ou une liaison ;
le noyau A et le noyau B sont indépendamment substitués ou non substitués ;
dans lesquels :
ledit groupe aliphatique substitué comprend un ou plusieurs substituants choisis dans le groupe consistant en un groupe électro-attracteur, halo, azido, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), oxo, époxy, hétérocycle non aromatique, benzyle, benzyle substitué, groupe aromatique et groupe aromatique substitué ;
ledit groupe benzyle substitué et ledit groupe aromatique substitué et les noyaux A et B lorsqu'ils sont substitués comprennent un ou plusieurs substituants choisis dans le groupe consistant en un groupe électro-attracteur, halo, azido, -CN, - COOH, -OH, -CONR²⁴R²⁵, -NR²⁴R²⁵, -OS(O)₂NR²⁴R²⁵, -S(O)₂NR²⁴R²⁵, -SO₃H, - S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, -Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), - Q-(groupe hétérocyclique non aromatique), -Q-(CH₂)ₚ-(groupe hétérocyclique non aromatique), groupe aliphatique et groupe aliphatique substitué ;
ledit groupe hétérocyclique non aromatique substitué comprend un ou plusieurs substituants choisis dans le groupe consistant en un groupe électro-attracteur, halo, azido, -CN, -COOH, -OH, -CONR²⁴R²⁵, -NR²⁴R²⁵, -OS(O)₂NR²⁴R²⁵, -S(O)₂NR²⁴R²⁵, -SO₃H, -S(O)₂NH₂, guanidino, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²², -(O)ᵤ-(CH₂)ₜ-NHC(O)OR²⁰, -Q-H, - Q-(groupe aliphatique), -Q-(groupe aliphatique substitué), -Q-(aryle), -Q-(groupe aromatique), -Q-(groupe aromatique substitué), -Q-(CH₂)ₚ-(groupe aromatique substitué ou non substitué), -Q-(groupe hétérocyclique non aromatique), -Q-(CH₂)ₚ-(groupe hétérocyclique non aromatique), groupe aliphatique, groupe aliphatique substitué, =O, =S, =NH, =N(aliphatique), =N(aromatique) et =N(aromatique substitué) ;
R²⁰, R²¹ et R²² sont indépendamment -H, un groupe aliphatique, un groupe aliphatique substitué, un groupe aromatique, un groupe aromatique substitué, un groupe hétérocyclique non aromatique, -NHC(O)-O-( groupe aliphatique), -NHC(O)-O-(groupe aromatique) ou -NHC(O)-O-(groupe hétérocyclique non aromatique) ; ou
R²¹ et R²², pris en association avec l'atome d'azote auquel ils sont liés, forment un noyau hétérocyclique non aromatique ;
R²³ est -H, un groupe aliphatique, un groupe benzyle, un groupe aryle ou un groupe hétérocyclique non aromatique ;
R²⁴ et R²⁵ sont indépendamment -H, -OH, un groupe aliphatique, un groupe aliphatique substitué, un groupe benzyle, un groupe aryle ou un groupe hétérocyclique non aromatique ;
t est zéro à trois ;
u est zéro à un ;
p est un à cinq;
Q est -O-, -S-, -S(O)-, -S(O)₂-, -OS(O)₂-, -C(O)-, -OC(O)-, -C(O)O-, - C(O)C(O)-O-, -O-C(O)C(O)-, -C(O)NH-, -NHC(O)-, -OC(O)NH-, -NHC(O)O-, - NH-C(O)-NH-, -S(O)₂NH-, -NHS(O)₂-, -N(R²³)-, -C(NR²³)NHNH-,-NHNHC(NR²³)-, -NR²⁴C(O)- ou -NR²⁴S(O)₂- ;
ledit groupe aminoalkyle est un groupe alkyle en C₁ à C₁₂ substitué par - NR²⁴R²⁵;
ledit groupe aliphatique est un hydrocarbure en C₁ à C₂₀ saturé ou non saturé ;
ledit groupe électro-attracteur est un alkylimino, un alkylsulfonyle, un carboxamido, un ester carboxylique d'alkyle, -CH=NH ou -NO₂ ;
ledit groupe acyle est un carbonyle aliphatique, un carbonyle aromatique, un sulfonyle aliphatique ou un sulfonyle aromatique ;
ledit groupe acyle substitué est un carbonyle aliphatique substitué, un carbonyle aromatique substitué, un sulfonyle aliphatique substitué ou un sulfonyle aromatique substitué ;
l'hétérocycle dudit groupe hétérocyclique non aromatique est un noyau non aromatique comportant cinq à huit chaînons qui contient un ou plusieurs hétéroatomes choisis dans le groupe consistant en O, N, et S ; et
ledit groupe aromatique est choisi dans le groupe consistant en un carbocycle aromatique en C₆, un hétérocycle aromatique en C₅ à C₆ comprenant un ou plusieurs hétéroatomes choisis dans le groupe consistant en O, N, et S et un carbocycle aromatique en C₆ ou un hétérocycle aromatique en C₅ à C₆ qui est fusionné à un ou plusieurs autres noyaux ;
ou un sel de celui-ci acceptable du point de vue physiologique.

26. Composé selon la revendication 24 dans lequel Z est : 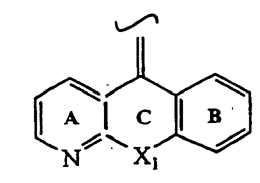 dans lequel :
X₁ est -CH₂-S-, -S-CH₂-, -O-CH₂-, -CH₂-O-, -SO-CH₂-, -CH₂-SO-, -S(O)₂-CH₂-, -CH₂-S(O)₂-, ou une liaison ; et
le noyau A et le noyau B sont indépendamment substitués ou non substitués.

27. Composé selon l'une quelconque des revendications 6 à 23, 25 et 26 dans lequel le noyau B est substitué en para de l'atome de carbone du noyau B qui est lié à X₁ dans le noyau C, de sorte que Z est : 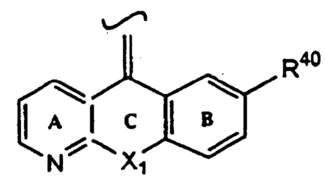 dans lequel :
R⁴⁰ est -OH, -COOH, -NO₂, halogène, un groupe aliphatique, un groupe aliphatique substitué, un groupe aromatique, un groupe aromatique substitué, - NR²⁴R²⁵, -CONR²⁴R²⁵, -Q-(groupe aliphatique), -Q-(groupe aliphatique substitué)-, - O-(groupe aliphatique), -O-(groupe aliphatique substitué)-, -O-(groupe aromatique), -O-(groupe aromatique substitué)-, un groupe électro-attracteur, -(O)ᵤ-(CH₂)ₜ-C(O)OR²⁰, -(O)ᵤ-(CH₂)ₜ-OC(O)R²⁰, -(O)ᵤ-(CH₂)ₜ-C(O)-NR²¹R²² ou -(O)ᵤ-(CH₂)ₜ-NHC(O)OR²⁰ ;
R²⁰, R²¹ ou R²² sont indépendamment -H, un groupe aliphatique, un groupe aliphatique substitué, un groupe aromatique, un groupe aromatique substitué ou un groupe hétérocyclique non aromatique ; ou
R²¹ et R²², pris en association avec l'atome d'azote auquel ils sont liés forment un groupe hétérocyclique non aromatique ;
Q est -NR²⁴C(O)-, -NR²⁴S(O)₂- ou -C(O)O- ;
R²⁴ et R²⁵ sont indépendamment -H, -OH, un groupe aliphatique ou un groupe aliphatique substitué ;
u est zéro ou un ; et
t est un nombre entier de zéro à 3.

28. Composé selon l'une quelconque des revendications 6 à 23, 25 et 26 dans lequel le noyau B est substitué en para de l'atome de carbone du noyau B qui est lié à X₁ dans le noyau C, de sorte que Z est : 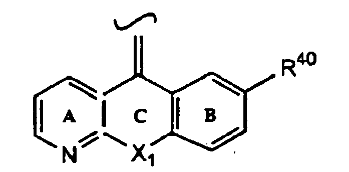 dans lequel :
R⁴⁰ est -C(=NR⁶⁰)NR²¹R²², -O-C(O)-NR²¹R²⁶, -S(O)₂-NR²¹R²² ou -NH-C(O)-NR²¹R²² ; dans lesquels
R²¹ et R²² sont indépendamment -H, un groupe aliphatique, un groupe aliphatique substitué, un groupe aromatique, un groupe aromatique substitué ou un groupe hétérocyclique non aromatique ; ou
R²¹ et R²², pris en association avec l'atome d'azote auquel ils sont liés, forment un groupe hétérocyclique non aromatique substitué ou non substitué ;
R²⁶ est -H, un groupe aliphatique, un groupe aliphatique substitué, un groupe aromatique, un groupe aromatique substitué ou un groupe hétérocyclique non aromatique, -C(O)-O-(groupe aliphatique substitué ou non substitué), -C(O)-O-(groupe aromatique substitué ou non substitué), -S(O)₂-(groupe aliphatique substitué ou non substitué), -S(O)₂-(groupe aromatique substitué ou non substitué) ; ou
R²⁶ et R²¹, pris en association avec l'atome d'azote auquel ils sont liés, forment un groupe hétérocyclique non aromatique substitué ou non substitué.

29. Composé selon l'une quelconque des revendications 1 à 28 dans lequel X₁ est -CH₂-O-.

30. Composé qui est une amine tertiaire de formule suivante : 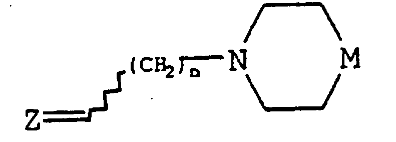 dans laquelle :
M est CR¹R² ;
R¹ est -OH ;
R² est 4-chlorophényle ;
n est deux ;
Zest: 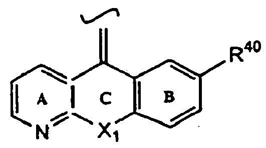
X₁ est -CH₂-O- ; et
R40 est 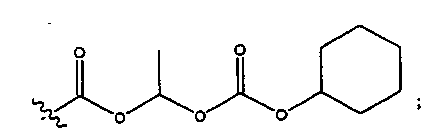
ou un sel de celui-ci acceptable du point de vue physiologique.

31. Composé selon l'une quelconque des revendications 6 à 10 dans lequel R¹ est un groupe alkyle, alcényle ou alcynyle linéaire substitué, ramifié ou cyclique en C₁ à C₂₀.

32. Composé selon l'une quelconque des revendications 1 à 31 pour utilisation dans un procédé de traitement thérapeutique du corps humain ou animal.

33. Composé selon la revendication 32 pour utilisation dans le traitement d'une maladie associée à un recrutement et/ou activation leucocytaire anormale.

34. Composé selon la revendication 32 pour utilisation dans le traitement d'une maladie inflammatoire chronique.

35. Composé selon la revendication 32 pour utilisation dans le traitement de l'arthrite rhumatoïde.

36. Composé selon la revendication 32 pour utilisation dans le traitement de la sclérose en plaques.

37. Utilisation d'un composé selon l'une quelconque des revendications 1 à 31 pour la fabrication d'un médicament pour le traitement d'une maladie associée à un recrutement et/ou activation leucocytaire anormale.

38. Utilisation d'un composé selon l'une quelconque des revendications 1 à 31 pour la fabrication d'un médicament pour le traitement d'une maladie inflammatoire chronique.

39. Utilisation d'un composé selon l'une quelconque des revendications 1 à 31 pour la fabrication d'un médicament pour le traitement de l'arthrite rhumatoïde.

40. Utilisation d'un composé selon l'une quelconque des revendications 1 à 31 pour la fabrication d'un médicament pour le traitement de la sclérose en plaques.
